# EUROPEAN PATENT SPECIFICATION

(11) **EP 3 003 346 B1**
(45) Date of publication and mention of the grant of the patent: **16.01.2019**
(21) Application number: 14728259.4
(22) Date of filing: 30.05.2014
(51) Int. Cl.: A61K 38/00, A61K 39/12, A61K 39/385, A61K 39/00, C07K 14/37, A61K 9/08, C07K 14/44

(54) **FISH VACCINE**
FISCHIMPFSTOFF
VACCIN POUR POISSON

(30) Priority: 30.05.2013 GB 201309664
(43) Date of publication of application: 13.04.2016
(73) Proprietor: The University Court of The University of Aberdeen, Aberdeen Aberdeenshire AB24 3FX (GB)
(72) Inventor: WAWRA, Stephan, Aberdeen Aberdeenshire AB24 3FX (GB); VAN WEST, Pieter, Aberdeen Aberdeenshire AB24 3FX (GB); SECOMBES, Christopher John, Aberdeen Aberdeenshire AB24 3FX (GB)
(74) Representative: Andrews, Robert
(86) International application number: PCT/GB2014/051655
(87) International publication number: WO 2014/191759

(56) References cited:
- WO-A1-2011/148135
- S. WAWRA ET AL: "Host-targeting protein 1 (SpHtp1) from the oomycete Saprolegnia parasitica translocates specifically into fish cells in a tyrosine-O-sulphate-dependent manner", PROCEEDINGS OF THE NATIONAL ACADEMY OF SCIENCES, vol. 109, no. 6, 7 February 2012 (2012-02-07), pages 2096-2101, XP055136468, ISSN: 0027-8424, DOI: 10.1073/pnas.1113775109
- STEPHEN C WHISSON ET AL: "A translocation signal for delivery of oomycete effector proteins into host plant cells", NATURE, NATURE PUBLISHING GROUP, UNITED KINGDOM, vol. 450, no. 7166, 1 November 2007 (2007-11-01), pages 115-118, XP002656435, ISSN: 0028-0836, DOI: 10.1038/NATURE06203 [retrieved on 2007-09-30]

## Description

### BACKGROUND

The farming of fish, molluscs and crustaceans plays an important and growing part in the global supply of food. Farmed fish is supplying ∼30% by weight of world total fish production in 2001 (up from ∼4% in 1970). Aquaculture is therefore an important industry, with the culture of high-value species such as salmon and salmon trout worth US$10.7 billion in 2007.

Atlantic salmon is a particularly important species, and is the most intensively farmed fish in the world. Major producers of farmed Atlantic salmon include Norway, Chile, Canada, and the UK.

Maintaining the health of farmed fish stocks is crucial for maximizing fish survival rate, growth and therefore profitability in aquaculture. Controlling the levels of fish borne diseases and parasites are key to this.

In the wild fish populations, diseases and parasites are typically present at low levels. Transmission of pathogens is reduced in the relatively low population densities seen in wild stocks, with numbers of sick individuals also kept in check by natural predation. However, in the crowded net pens commonly used in aquaculture, the farmed fish are shielded from natural predators and kept in close proximity to infected individuals. Under these conditions, diseases and parasites can rapidly enter the farmed population and reach epidemic levels.

### Disease control in aquaculture

In order to control the levels of disease, farmed fish can be vaccinated against the most common pathogens which affect farmed fish. The advent of widespread vaccination has significantly boosted the productivity of aquaculture, as well as allowing the use of antibiotics to be significantly reduced.

### Vaccination techniques

Vaccines against a broad range of pathogenic antigens are available, including those from *Vibrio anguillarum, Vibrio salmonicida, Aeromonas salmonicida, Yersinia ruckeri,*

*Renibacterium salmonis, Piscirickettsia salmonis,* IPN, IHN and ISA (see Sommerset et al. 2005, Expert Rev. Vaccines 4(1), 89-101). Fish may be vaccinated by oral administration, immersion (bath, dip or spray) or injection (intraperitoneal or intramuscular).

Much effort has been put into the development of oral vaccines, as this administration method offers the advantages of being easy to deliver (for example, in food), thereby saving labour on behalf of the farmer and minimizing stress to the fish. However, immunization by this route has been reported to be weak and of short duration, as well as requiring a large quantity of antigen to be delivered. These disadvantages mean that there has been little uptake of oral vaccines (see Sommerset et al. 2005, Expert Rev. Vaccines 4(1), 89-101).

Immersion vaccines share many of the features of oral vaccines: they are easy to deliver and minimize the stress to the fish, but also typically require large amounts of antigen and result in immunity that is weaker and of a shorter duration than that offered by injection. However, unlike oral administration, there are several pathogens for which immersion vaccinations are considered to offer reasonable protection (for example, versus infections by *Vibrio anguillarum, Vibrio salmonicida* and *Yersinia ruckeri*).

WO2011/148135 describes proteins and protein constructs and their translocation into cultured the fish cell lines RTG-2 (trout gonad), RTL (trout liver), RTGill (trout gill) and SDJ (Zebra fish).

Despite the availability of effective immersion vaccines against some pathogens, many of the economically important diseases (such as furunculosis) require that the commercially available antigen is delivered together with an adjuvant for an effective level of immunity is to be reliably stimulated. Since the available fish adjuvants are often oil-based, this means that antigens requiring an adjuvant cannot be delivered by the immersion method. Oral delivery is also often ineffective, presumably due to digestion of the antigen/adjuvant before an immune response is raised.

A consequence of the adjuvant requirement by economically important disease antigens is that vaccines for these diseases must be injected (for example, intraperitoneally or intramuscularly). Since a single injection can be used to deliver more than one type of antigen, this means that 'multi-antigen injected vaccines' are currently the vaccination method of choice in the aquaculture industry (see Sommerset et al. 2005, Expert Rev. Vaccines 4(1), 89-101).

Vaccination by injection offers the advantage that it reliably generates a strong and long-lasting immune response. However, the injection process is costly and labour-intensive and causes significant handling stress on the fish. This creates a risk of post-vaccination infection (in particular, *Saprolegnia* infections), which can decrease fish survival and growth rates.

In addition to the risk of post-handling infection, injection can also lead to so-called "local reactions" in the injected fish. These "local reactions" include, for example, local and/or diffuse peritonitis with adhesions to internal organs adhesions, melanisation, and/or multiple granulomata. It is believed that in some cases, the use of adjuvants with the injected antigen increases the severity of these reactions. As well as being a fish welfare issue, these reactions can lead to reduced growth rates, fish that are unfit for consumption and interfere with fish processing (such as gutting).

In view of the above discussion of existing vaccination methods, there is an ongoing need for alternative fish vaccination methods that avoid the disadvantages of injection/adjuvants but which still reliably deliver high levels of long-lasting immunity.

Wawra et al, Proc. Natl. Acad. Sci. USA, 2012, 109(6):2096-2101 demonstrates that "Host-targeting protein 1 (SpHtpl), from the oomycete Saprolegnia parasitica translocates specifically into fish cells" in vitro.

WO 2011/148135 relates to "proteins comprising sequence elements found in the AVR3a protein from the potato late blight pathogen Phytophthora infestans", "derivatives of these proteins and methods of modulating their function and activity" and "uses of these proteins, including the use to direct protein translocation into eukaryotic cells."

### DISCLOSURE

The present inventors have cloned and expressed a fusion protein containing an antigenic polypeptide (RFP) fused to amino acids 24 to 68 of the protein SpHtp1, a putative effector from the fish pathogenic oomycete, *Saprolegnia parasitica.* Rainbow trout (*Oncorhynchus mykiss*) were then exposed to the composition by immersing the trout in a dilute solution (3µM) of the composition for a short time (1.5 hours). Surprisingly, and as shown in the examples, the composition was able to enter the cells of the live fish and elicit an immune (antibody) response against the antigenic polypeptide. No adjuvants were required to stimulate the immune response. Injection of the composition into the fish was not required.

Thus, the findings of the present inventors allow for the effective vaccination of fish against almost any antigen without the need for injection and the resultant stress and infection risk to the fish. By coupling an antigen to the SpHtp1 translocation sequence and immersing a fish in a solution of the resulting composition, an immune response against the antigen can be stimulated.

Thus, disclosed herein is a composition comprising, consisting, or consisting essentially of: (i) a translocation sequence; and (ii) a heterologous payload coupled to the translocation sequence.

The properties of the translocation sequence enable the composition to translocate across the plasma membrane of a eukaryotic cell, for example a fish cell. In some cases the composition is able to translocate across the plasma membrane of cells in culture (for example, a RTG-2 cell line, a RTL cell line, a RTGill cell line, or a RTS11 cell line) and/or across the plasma membrane of cells in a live fish (for example, cells present in the gills or gut of the Rainbow trout (*Oncorhynchus mykiss*) and other species important for marine, brackish and fresh water aquaculture such as salmonids, catfish, carps, sea bass, flat fish, and Tilapia's. In particular: Grass carp (*Ctenopharyngodon idella*), Silver carp (*Hypophthalmichthys molitrix*), Catla (*Cyprinus catla* or *Gibelion catla*), Common Carp (*Cyprinus carpio*), Bighead carp (*Hypophthalmichthys nobilis* or *Aristichthys nobilis*), Crucian carp (*Carassius carassius*), *Oreochromis niloticus* Nile Tilapia (*Oreochromis niloticus*), Mozambique Tilapia (*Oreochromis mossambicus*) and other Tilapia's, Pangas catfishes (*Pangasius pangasius*), Roho (*Labeo rohita*)*,* Atlantic salmon (*Salmo salar*), Arctic charr (*Saivelinus alpinus*), brown trout (*Salmo trutta*), rainbow trout (*Oncorhynchus mykiss*), sea trout (*Salmo trutta*) and other trouts. In some cases the composition stimulates an immune (e.g. antibody) response in an organism (e.g. fish) that is exposed to the composition; preferably, the stimulated antibodies recognize an epitope in the payload (which may be, for example, a peptide antigen).

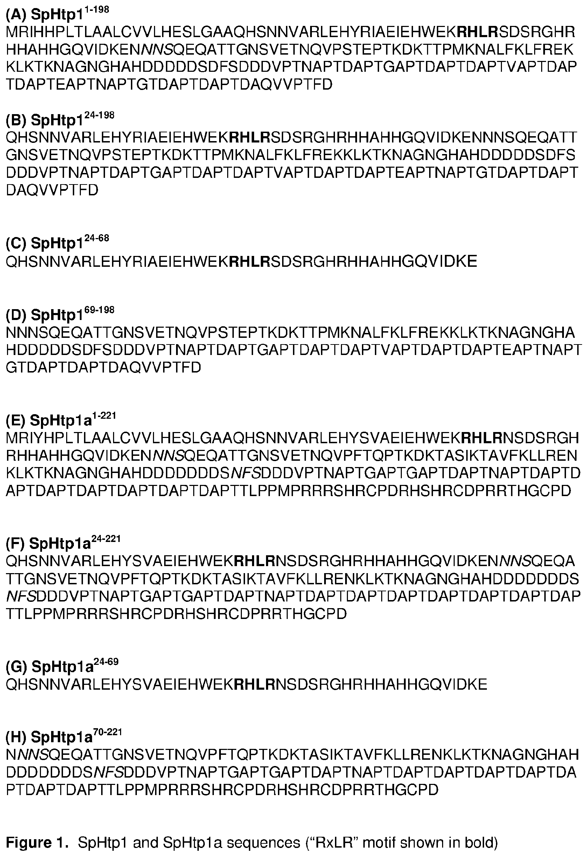

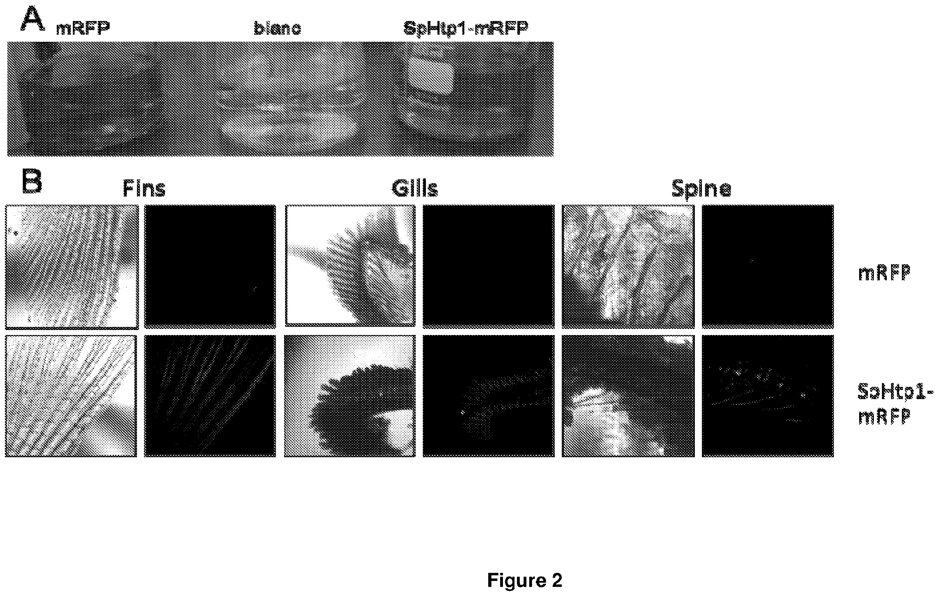

As described herein, the translocation sequence may comprise, consist or consist essentially of a polypeptide having the sequence of SpHtp1²⁴⁻⁶⁸ or SpHtp1a²⁴⁻⁶⁹ as shown in Figure 1. As shown in the examples, this polypeptide is capable of directing a heterologous payload into the cells of a live fish such that the fish mounts an immune (antibody) response against the payload.

The present disclosure further provides nucleic acids, vectors and host cells suitable for production of the composition, along with vaccines comprising the composition.

Also provided herein are processes for producing the nucleic acids, vectors, host cells, compositions and vaccines based thereon.

Some aspects and embodiments of the present disclosure will now be discussed in more detail.

### TRANSLOCATION SEQUENCE

Thus, in one aspect the compositions disclosed herein comprise a translocation sequence which comprises, consists or consists essentially of a polypeptide having the sequence of SpHtp1²⁴⁻⁶⁸ or SpHtp1a²⁴⁻⁶⁹ as shown in Figure 1.

### Origin of SpHtp1

SpHtp1 is a putative effector from the fish pathogenic oomycete, *Saprolegnia parasitica.*

The inventors have previously shown that the host targeting protein SpHtp1 bind to tyrosine-O-sulfate, and that sulfatase treatment of cells strongly decreases the observed uptake of saprolegnia host targeting protein (works also with SpHtp1a) proteins into those cells (Wawra et al., 2012). It is therefore believed that these proteins and homologs thereof recognize and bind O-sulfated cell surface molecules as part of the translocation mechanism.

The amino acid sequences of 'full-length' SpHtp1 (1-198) and a number of fragments are shown in Figure 1.

Earlier work of the inventors has shown that a SpHtp1²⁴⁻⁶⁸:RFP composition is able to enter cultured fish cells; translocation has been observed into trout epithelia cells (RTG-2 cell line) (see Wawra et al. 2012, www.pnas.org/cgi/doi/10.1073/pnas.1113775109), trout liver cells (RTL cell line), trout gill cells (RTGill cell line) (Submitted paper Lobach and Wawra et al.), the macrophage cell line RTS11 and primary macrophages and a Zebra fish cell line (SDJ.1). This process can be inhibited by e.g. the peanut agglutinin and soy bean agglutinin in an allosteric manner on all tested fish cell lines (RTG-2, RTL, RTGill).

Notwithstanding the above, the novel finding described herein that the SpHtp1^{24m68}:RFP composition can translocate into the cells of live fish immersed in a dilute solution of the composition (and, thereby, elicit an immune response) is unexpected. Cells in culture are relatively 'open', that is, the surrounding media is relatively benign and their surface membranes are relatively accessible; in comparison, tissues in live fish that are exposed to the external environment (e.g. gut, gills, skin) have defences such as a mucous layer and extracellular/surface proteases designed to prevent any unwanted exogenous molecules from entering the fish. Accordingly, the fact that the SpHtp1²⁴⁻⁶⁸:RFP composition was able to survive the 'live' environment, enter the fish, and induce an immune response was highly surprising.

The ability of the SpHtp1²⁴⁻⁶⁸ composition to cross cell membranes appears to be fish-specific, since the same composition was not able to translocate into human (HEK293) or onion cells (see Wawra et al. 2012, www.pnas.org/cgi/doi/10.1073/pnas.1113775109) or the human A549 cell line (Wawra et al. 2012, MPMI Vol 26 (5) pp528-36). However, the reported ability of some other proteins to enter non-fish cells suggest that an amino acid variant of the SpHtp1²⁴⁻⁶⁸ composition may have a different, or no, organism specificity.

### SpHtp1 as a translocation sequence

'Full-length' *SpHtp1* contains 198 amino acids (see Figure 1). However the fragment consisting of amino acids 24 to 68 (SpHtp1²⁴⁻⁶⁸) fused to mRFP retains the ability to translocate into live fish cells. The SpHtp1 homolog, SpHtp1a, also has this translocation ability. Thus, in some embodiments the translocation sequence consists of, or consists essentially of, SpHtp1²⁴⁻⁶⁸ or SpHtp1a²⁴⁻⁶⁹ shown in Figure 1.

Longer polypeptides comprising the SpHtp1²⁴⁻⁶⁸ or SpHtp1a²⁴⁻⁶⁹ fragments have the ability to translocate into the cells of live fish. Thus, in some embodiments the translocation sequence comprises SpHtp1²⁴⁻⁶⁸ or SpHtp1a²⁴⁻⁶⁹ shown in Figure 1. The translocation sequence may be of any length. However, in some embodiments it is no more than 200 amino acids, such as no more than 150, no more than 125, no more than 100 or no more than 75 amino acids. In some embodiments the translocation sequence is no more than 60 amino acids. In some embodiments the translocation sequence is no more than 50 amino acids.

Where the SpHtp1²⁴⁻⁶⁸ or SpHtp1 a²⁴⁻⁶⁹ shown in Figure 1 forms part of a longer translocation sequence, the SpHtp1²⁴⁻⁶⁸ or SpHtp1a²⁴⁻⁶⁹ fragment can be at any point of the translocation sequence: at the very N-terminal, at the very C-terminal, or at an intermediate point between the N- and C- terminals.

### Variants

Sequence variants of *SpHtp1*²⁴⁻⁶⁸ are also able to translocate into the cells of live fish. Thus, in some embodiments the translocation sequence consists of, or consists essentially of, a sequence variant of SpHtp1²⁴⁻⁶⁸ shown in Figure 1. For example, the SpHtp1 homolog SpHtp1a shown in Figure 1 is able to translocate into the cells of live fish. Thus, in some embodiments the translocation sequence consists of, or consists essentially of, a sequence variant of SpHtp1a²⁴⁻⁶⁹ shown in Figure 1.

Longer polypeptides comprising sequence variants of the SpHtp1²⁴⁻⁶⁸ or SpHtp1a²⁴⁻⁶⁹ shown in Figure 1 have the ability to translocate into the cells of live fish. Thus, in some embodiments the translocation sequence comprises a sequence variant of SpHtp1²⁴⁻⁶⁸ or SpHtp1a²⁴⁻⁶⁹ shown in Figure 1. The translocation sequence may be of any length. However, in some embodiments it is no more than 200 amino acids, such as no more than 150, no more than 125, no more than 100 or no more than 75 amino acids. In some embodiments the translocation sequence is no more than 60 amino acids. In some embodiments the translocation sequence is no more than 50 amino acids.

Where the sequence variant of SpHtp1²⁴⁻⁶⁸ or SpHtp1a²⁴⁻⁶⁹ shown in Figure 1 forms part of a longer translocation sequence, the variant SpHtp1²⁴⁻⁶⁸ or SpHtp1a²⁴⁻⁶⁹ fragment can be at point of the translocation sequence: at the very N-terminal, at the very C-terminal, or at an intermediate point between the N- and C- terminals.

As used herein, a "sequence variant of SpHtp1²⁴⁻⁶⁸ or SpHtp1a²⁴⁻⁶⁹ shown in Figure 1" is an amino acid sequence having at least 60, 70, 75, 80, 85, 90, 95, 96, 97, 98, 99 or 100% identity to the amino acid sequence SpHtp1²⁴⁻⁶⁸ or SpHtp1a²⁴⁻⁶⁹ shown in Figure 1.

In one embodiment the translocation sequence will have equal to, or no more than, 1, 2, 3, 4, 5, 6, 7, 8, 9, 10, 12, 15 or 20 substitutions, deletions, or additions in the amino acid sequence SpHtp1²⁴⁻⁶⁸ or SpHtp1a²⁴⁻⁶⁹ as shown in Figure 1.

Identity may be as defined using sequence comparisons are made using FASTA and FASTP (see Pearson & Lipman, 1988. Methods in Enzymology 183: 63-98). Parameters are preferably set, using the default matrix, as follows: Gapopen (penalty for the first residue in a gap): -12 for proteins / -16 for DNA; Gapext (penalty for additional residues in a gap): -2 for proteins / -4 for DNA; KTUP word length: 2 for proteins / 6 for DNA.

For the avoidance of doubt, the level of sequence identity is measured over the full-length of the amino acid sequence SpHtp1²⁴⁻⁶⁸ or SpHtp1a²⁴⁻⁶⁹ shown in Figure 1 (45 or 46 amino acids).

The variant translocation sequences may originate from other native proteins, or may be prepared by those skilled in the art, for instance by site directed or random mutagenesis of the SpHtp1²⁴⁻⁶⁸ or SpHtp1a²⁴⁻⁶⁹ sequence shown in Figure 1. Alternatively the variant translocation sequences maybe produced by direct synthesis.

Changes may be desirable for a number of reasons, including introducing or removing the following features: sites which are required for post translation modification; cleavage sites in the encoded polypeptide; motifs in the encoded polypeptide (e.g. epitopes). Leader or other targeting sequences (e.g. hydrophobic anchoring regions) may be added or removed from the expressed protein to determine its location following expression.

Other desirable mutations may be made by random or site directed mutagenesis of the nucleic acid encoding the polypeptide in order to alter the activity (e.g. specificity) or stability of the encoded polypeptide.

Changes may be by way of conservative variation, i.e. substitution of one hydrophobic residue such as isoleucine, valine, leucine or methionine for another, or the substitution of one polar residue for another, such as arginine for lysine, glutamic for aspartic acid, or glutamine for asparagine. As is well known to those skilled in the art, altering the primary structure of a polypeptide by a conservative substitution may not significantly alter the activity of that peptide because the side-chain of the amino acid which is inserted into the sequence may be able to form similar bonds and contacts as the side chain of the amino acid which has been substituted out. This is so even when the substitution is in a region which is critical in determining the peptides conformation.

Also included are variants having non-conservative substitutions. As is well known to those skilled in the art, substitutions to regions of a peptide which are not critical in determining its conformation may not greatly affect its ability to raise antibodies because they do not greatly alter the peptide's three dimensional structure.

In regions which are critical in determining the peptides conformation or activity such changes may confer advantageous properties on the polypeptide. Indeed, changes such as those described above may confer slightly advantageous properties on the peptide e.g. altered specificity, stability or immunogenicity.

### PAYLOAD

The payload is a "heterologous payload". As used herein, a "heterologous payload" is a payload that is heterologous to the translocation sequence to which it is coupled. In this context, "heterologous" means that the translocation sequence and payload are not derived from the same organism (for example, not from the same strain or species); that is, the translocation sequence and payload are derived from different organisms (for example, derived from different strains or species). Accordingly, in embodiments where the translocation sequence is SpHtp1²⁴⁻⁶⁸ or SpHtp1 a²⁴⁻⁶⁹ shown in Figure 1, the payload is not from *Saprolegnia parasitica* (the organism in which SpHtp1 and SpHtp1a are natively found).

The payload may be any type of molecule, for example a polypeptide, nucleic acid, lipid or small organic molecule. The terms "peptide", "polypeptide" and "protein" as used herein are used interchangeably to mean a polymer of two or more amino acids coupled through peptide bonds.

Preferably, the payload is a peptide or polypeptide. In embodiments where the payload is a peptide or polypeptide, the translocation sequence may be of any length. However, in some embodiments it is no more than 500 amino acids, such as no more than 400, no more than 300, no more than 200 or no more than 100 amino acids. In some embodiments the translocation sequence is no more than 75 amino acids. In some embodiments the translocation sequence is no more than 50 amino acids, such as no more than 30 amino acids.

In some embodiments the payload is a particle or bead. For example, the payload may be a particle with high contrast in detection methods (such as a gold particle).

In some embodiments the payload is a therapeutic agent, a marker (e.g. a fluorescent protein such as GFP or RFP) or a protective agent (e.g. an agent that protects the cell from the effects of a cytotoxin to which the cell is subsequently exposed). In some embodiments the payload is a cytotoxic molecule (i.e. a molecule, which when bound to or taken up by a target cell stimulates the death and lysis of the cell) or an RNA molecule such as a siRNA.

Cytotoxic molecules include members of the following groups or families: nitrogen - mustard types (e.g. melphalan), anthracyclines (e.g. adriamycin, doxorubicin, and daunomycin), nucleoside analogues (e.g. cytosine arabinoside) or antimetabolites (e.g. methotrexate). Also encompassed by the term "cytotoxic molecules" as used herein are enzymes intended to catalyse the conversion of a non-toxic prodrug into a cytotoxic drug (for example a HSV-Thymidine Kinase / Ganciclovir system). The prodrug may be systemically administered.

In some embodiments the payload comprises an antigen or immunogen.

### Antigens and Immunogens

In some embodiments the payload consists of a single antigen. In other embodiments, the payload comprises one, two, three, four, five, six, seven, eight, or more than eight antigens. In embodiments where the payload comprises more than one antigen, each antigens can be from the same or different organisms. In one embodiment, each antigen in the payload is from a different organism (i.e. antigens 1, 2, 3, 4 are correspondingly form organisms A, B, C, D).

As used herein, the term "antigen" is used to describe a substance that provokes the production of one or more antibodies when introduced into an organism (as detectable by techniques such as ELISA).

An antigen may be a peptide, lipid or nucleic acid. Preferably, the antigen is a peptide antigen.

The antigen may be a specific type or group of molecule. For example, the antigen may be a viral coat protein, a viral envelope protein, a viral lipid, a viral glycan, a bacterial envelope protein, a bacterial coat protein, a bacterial lipid, and/ or a bacterial glycan.

An antigen may contain only a single epitope. Alternatively, in some embodiments an antigen contains two, three, four, five or more than 5 epitopes.

The antigen may be from a fish pathogen. For example, the antigen may be a peptide or polypeptide from a fish pathogen. Example fish pathogens from which antigens may be from are (i) bacterial pathogens, for example *Vibrio anguillarum* & *Vibrio salmonicida* (vibriosis), *Aeromonas salmonicida* & *Aeromonas hydrophila* (furunculosis), *Yersinia ruckeri* (Enteric Redmouth Disease), *Renibacterium salmonis, Lactococcus sp., Streptococcus sp.,* and *Piscirickettsia salmonis*; (ii) viral pathogens, for example Infectious pancreatic necrosis virus (IPNV), Infectious Salmon Anemia virus (ISAV), Salmon Pancreas Disease virus (SPD virus), Sleeping Disease of Trout virus (SDV), Viral Nervous Necrosis virus (VNNV) and Infectious Heamatopoietic Necrosis virus (IHNV), Viral haemorrhagic septicaemia virus (VHSV); (iii) Fungal pathogens, for example *Saprolegnia sp.* (such as *S*. *parasitica, S. diclina and S. australis*) or *Aphanomyces* sp. (*A. invadans* and *A. astaci*) and *Branchiomyces sp.* (Gill rot); and/or (iv) Parasitic pathogens, for example *Ichthyophthirius multifiliis, Cryptocaryon irritans* and *Trichodina sp.* (Trichodiniasis), Amoebic gill disease (eg. *Neoparamoeba perurans*), sea lice (copepods within the order *Siphonostomatoida,* family *Caligidae* (for example *Lepeophtheirus salmonis, Caligus rogercresseyi, Caligus clemensi, Caligus elongatus*), proliferative kidney disease or PKD (*Tetracapsuloides bryosalmonae*) and other species.

The antigen may be a peptide or polypeptide from a virus, for example a peptide or polypeptide from the outer proteins or coat proteins of a virus such as IPNV (example polypeptides, or encoding nucleotides = Genbank accession numbers ACY35988.1, ACY35989.1, ACY35990.1; UniProt accession numbers D0VF01, D0VF02, D0VF03), ISAV (example polypeptides, or encoding nucleotides = Genbank accession numbers EU625675, FJ594325; UniProt accession numbers C6ETL2, C6G756), SPD (example polypeptides, or encoding nucleotides = Genbank accession number AJ012631.1; UniProt accession number Q9WJ34), SDV (example polypeptides, or encoding nucleotides = Genbank accession number AJ238578.1; UniProt accession number Q8QL52), IHNV (example polypeptides, or encoding nucleotides = Genbank accession number X89213.1; UniProt accession numbers Q08449, Q08455, Q08454, Q08453, Q82706, Q08445, Q08454, Q82707) VHSV (example polypeptides, or encoding nucleotides = Genbank accession numbers EU481506.1, ACA34520.1, ACA34521.1, ACA34522.1, ACA34523.1, ACA34524.1, ACA34525.1).

### COUPLING OF TRANSLOCATION SEQUENCE AND PAYLOAD

In some embodiments, the translocation sequence is covalently bonded to the payload, for example, via a peptide bond. In embodiments where the payload is a peptide or polypeptide the translocation sequence and the payload may be coupled via an peptide bond so that they form a contiguous polypeptide chain; that is, the composition of the disclosure may be a fusion protein comprising the translocation sequence fused to the peptide payload. The fusion protein may be of any length. However, in some embodiments it is no more than 500 amino acids, such as no more than 400, no more than 300, no more than 200 or no more than 100 amino acids. In some embodiments the translocation sequence is no more than 80 amino acids. In some embodiments the translocation sequence is no more than 60 amino acids.

The coupling of the translocation sequence and the payload may be direct i.e. without intervening elements (such as amino acids).

The payload may be an "exogenous protein" i.e. a protein that is not natively expressed in the eukaryotic cell into which it is being translocated. Exogenous proteins include, for example, fusions of native proteins, or fragments of native proteins, with non-native polypeptides or other molecules.

The translocation sequence and payload may be separated by a short linker sequence or residue in order to facilitate expression or folding e.g. one, two, three, four, five or more gly residues. However, the presence of a linker is not required. Similarly, in embodiments where the payload contains two or more antigens, the antigens may be separated by a short linker sequence or residue in order to facilitate expression or folding e.g. one, two, three, four, five or more gly residues. Again, the presence of a linker is not required.

Having described the fusion and other proteins of the present disclosure some other aspects of the disclosure will now be discussed.

### Nucleic acids, vectors and host cells

The compositions of disclosure herein are conveniently prepared from recombinant nucleic acids which can be expressed in host cells with good yield, for example from a pET vector and can be expressed in *Escherichia coli.*

Thus in one aspect there is provided a nucleic acid encoding a composition of the disclosure. Such nucleic acid is herein referred to "composition nucleic acid" for brevity. This composition nucleic acid may be put together, for example, using "overlap PCR".

Nucleic acid according to the present disclosure may include cDNA, RNA, genomic DNA and modified nucleic acids or nucleic acid analogues (e.g. peptide nucleic acid). Where a DNA sequence is specified, e.g. with reference to a figure, unless context requires otherwise the RNA equivalent, with U substituted for T where it occurs, is encompassed. Nucleic acid molecules according to the present disclosure may be provided isolated and/or purified from their natural environment, in substantially pure or homogeneous form, or free or substantially free of other nucleic acids of the species of origin, and double or single stranded. Where used herein, the term "isolated" encompasses all of these possibilities. The nucleic acid molecules may be wholly or partially synthetic. In particular they may be recombinant in that nucleic acid sequences which are not found together in nature (do not run contiguously) have been ligated or otherwise combined artificially. Nucleic acids may comprise, consist, or consist essentially of, any of the sequences discussed hereinafter.

Aspects of the disclosure further embrace isolated nucleic acid comprising a sequence which is complementary to any of those discussed hereinafter.

In one aspect of the disclosure, the composition nucleic acid described above is in the form of a recombinant and preferably replicable vector.

Composition nucleic acids can be incorporated into a specially constructed vector or can be constituted *in situ* by introducing nucleic acid encoding the various portions such that the whole sequence is created in frame in the vector. Such processes for making the recombinant vectors form one aspect of the present disclosure.

It is envisaged that compositions as defined herein, based on newly characterized translocation sequences or payloads (e.g. from newly characterized antigens from new or known antigens) may be prepared simply and easily by sequencing, synthesis of DNA, followed by PCR and ligation (e.g. using the appropriate restriction sites).

"Vector" is defined to include, inter alia, any plasmid, cosmid, or phage in double or single stranded linear or circular form which may or may not be self-transmissible or mobilizable, and which can transform a prokaryotic or eukaryotic host either by integration into the cellular genome or exist extrachromosomally (e.g. autonomous replicating plasmid with an origin of replication).

Generally speaking, those skilled in the art are well able to construct vectors and design protocols for recombinant gene expression. Suitable vectors can be chosen or constructed, containing appropriate regulatory sequences, including transcriptional and translational regulatory elements, such as transcriptional enhancer sequences, translational enhancer sequences, promoters, ribosomal entry sites, including internal ribosomal entry sites, activators, translational start and stop signals, transcription terminators, cistronic regulators, polycistronic regulators, marker genes and other sequences as appropriate. For further details see, for example, Molecular Cloning: a Laboratory Manual: 2nd edition, Sambrook et al, 1989, Cold Spring Harbor Laboratory Press or Current Protocols in Molecular Biology, Second Edition, Ausubel et al. eds., John Wiley & Sons, 1992.

Specifically included are shuttle vectors by which is meant a DNA vehicle capable, naturally or by design, of replication in two different host organisms, which may be selected from actinomycetes and related species, bacteria and eukaryotic (e.g. yeast or fungal cells).

A vector including nucleic acid according to the present disclosure need not include a promoter or other regulatory sequence, particularly if the vector is to be used to introduce the nucleic acid into cells for recombination into the genome.

However preferably the nucleic acid in the vector is under the control of, and operably linked to, an appropriate promoter or other regulatory elements for transcription in a host cell such as a microbial, e.g. bacterial cell. By "promoter" is meant a sequence of nucleotides from which transcription may be initiated of DNA operably linked downstream (i.e. in the 3' direction on the sense strand of double-stranded DNA). Example vectors include plasmids pQE, pET or other commercial or generally accessible plasmids.

A preferred vector is the pET11-a plasmid, although where expression is desired in XL1 - blue, other vectors may be preferred.

"Operably linked" means joined as part of the same nucleic acid molecule, suitably positioned and oriented for transcription to be initiated from the promoter. DNA operably linked to a promoter is "under transcriptional initiation regulation" of the promoter.

In one embodiment, the promoter is an inducible promoter. The term "inducible" as applied to a promoter is well understood by those skilled in the art. In essence, expression under the control of an inducible promoter is "switched on" or increased in response to an applied stimulus. The nature of the stimulus varies between promoters. Some inducible promoters cause little or undetectable levels of expression (or no expression) in the absence of the appropriate stimulus. Other inducible promoters cause detectable constitutive expression in the absence of the stimulus. Whatever the level of expression is in the absence of the stimulus, expression from any inducible promoter is increased in the presence of the correct stimulus. A typical inducible promoter is the *lac* promoter induced by IPTG.

The present disclosure also provides methods comprising introduction of such an expression construct into a cell e.g. a microbial cell (e.g. bacterial, yeast or fungal) cell or an insect cell and/or induction of expression of the expression construct within the cell, by application of a suitable stimulus e.g. an effective exogenous inducer.

Also disclosed herein is a host cell containing a expression construct according to the disclosure, especially a microbial cell such as *E*. *coli e.g. E. coli* XL1-blue cells

The disclosure further encompasses a host cell transformed with nucleic acid or a vector according to the present disclosure (e.g. comprising the fusion nucleic acid) especially a microbial cell such as *E. coli.*

The fusion protein can be produced by culturing a microbial cell as described above e.g. by growing the cells in liquid medium with appropriate selection reagents (e.g. LB medium with ampicillin).

Also disclosed herein is a process for producing a composition for use in a vaccine in a human or animal (such as a fish), the process comprising:
i) providing a microbial cell as described above,
ii) culturing the cell such as to product the composition of the disclosure,
iii) purifying the protein composition therefrom.

Optionally step i) is preceded by the steps described above i.e. preparation of the vector and\or introduction into a host cell.

Optionally step iii) may be followed by formulating the composition as a vaccine. Vaccines are discussed in more detail below.

It will be appreciated that while these steps produce very pure product, further preparative steps are not excluded. These may include gel filtration, PEG, ammonium sulfate or ethanol precipitation, acid extraction, phosphocellulose chromatography, hydrophobic interaction chromatography, affinity chromatography, hydroxylapatite chromatography, reverse phase chromatography, preparative electrophoresis, detergent solubilization, selective precipitation, centrifugation, ultracentrifugation, density gradient centrifugation, ultrafiltration through a size exclusion filter, and so on. General techniques are further described in, for example, R. Scopes, Peptide Purification: Principles and Practice, Springer- Verlag: N. Y. (1982).

### VACCINES AND USES

The present disclosure provides vaccines comprising the composition of the disclosure or composition nucleic acids as defined above, and uses thereof as vaccines. In particular, the present disclosure envisages the use of the compositions of the disclosure in vaccines against disease in fish or other marine animal. In principle, the payload can comprise an antigen from any fish or other marine animal pathogen, meaning, the compositions described herein have the capacity to vaccinate fish or other marine animal against any pathogen.

A significant advantage of vaccines comprising the compositions of the present disclosure is that they can be administered without handling the fish or other marine animal (e.g. via immersion or oral routes; see example). Thus, the vaccines of the present disclosure present a low cost, low-labour and handling-stress free alternative to vaccination by injection, the current method of choice in the aquaculture industry.

Whilst it may be desirable to administer vaccines of the present disclosure without handling the fish or other marine animal, it is also possible to administer vaccines of the present disclosure by injection. The injected vaccines can comprise an adjuvant and be administered in an effective amount to a human or animal (such as a fish or other marine animal) in order to elicit an immune response. In preferred embodiments, the compositions are administered without an adjuvant to a human or animal (such as a fish or other marine animal). It is believed that such administration without an adjuvant will decrease the occurance and/or severity of "local reactions".

In one embodiment, the vaccine of the disclosure comprises a composition nucleic acid as defined above. In this embodiment, the vaccine can be administered by injection. The vaccine may be administered with or without an adjuvant.

Furthermore, vaccines can be produced in which the composition's payload contains antigens from multiple different fish (or other marine animal) pathogens, in principle allowing for simultaneous vaccination against multiple diseases. Alternatively, multiple simultaneous vaccination can be achieved by, for example, preparing an immersion solution containing several different (for example, 2, 3, 4, 5, 6, 7, 8, 9 or 10) different compositions or composition nucleic acids of the disclosure immersion of a fish (or other marine animal) in this solution will result in vaccination against the antigens ion each of the payloads.

Disclosed herein is a vaccine which comprises:
(i) a fragment of SpHtp1¹⁻¹⁹⁸ as shown in Figure 1, which fragment comprises SpHtp1²⁴⁻⁸⁸;
(ii) a fragment of SpHtp1a¹⁻²²¹ as shown in Figure 1, which fragment comprises SpHtp1 a²⁴⁻⁶⁹; or
(iii) a polypeptide having at least 60, 70, 75, 80, 85, 90, 95, 96, 97, 98, 99 or 100% identity to (i) or (ii) which is able to translocate across cell membranes;

In combination with one or more of a pharmaceutically acceptable excipient, carrier, buffer, stabiliser (e.g. protease inhibitor) or adjuvant. In preferred embodiments, the vaccine does not include an adjuvant. In some embodiments the fragment is no more than 150 amino acids long, such as no more than 130, no more than 125, no more than 100 or no more than 75 amino acids. In some embodiments the fragment is no more than 60 amino acids. In some embodiments the fragment is no more than 50 amino acids.

Vaccines and their uses will now be described in more detail.

Vaccines will comprise a composition of the disclosure or a composition nucleic acid. A vaccine may comprise only one type of composition or composition nucleic acid, or may comprise more than one composition or composition nucleic acid, such as 2, 3, 4, 5, 6, 7, 8, 9, 10 or more than 10 different types of composition or composition nucleic acid. Vaccines may comprise, in addition to the above one or more compositions or composition nucleic acids, a pharmaceutically acceptable excipient, carrier, buffer, stabiliser (e.g. protease inhibitor) or other materials well known to those skilled in the art. Such materials should be non-toxic and should not interfere with the efficacy of the active ingredient (i.e. the composition of the disclosure). The precise nature of the carrier or other material may depend on the route of administration, e.g. oral or immersion routes.

In some embodiments the vaccines can comprise components that increase the efficacy of uptake of the composition or composition nucleic acid of the disclosure by the organism to be immunized, for example cofactors of a payload.

Also described herein is the use of a vaccine of the disclosure in the manufacture of a medicament for inducing an immune response in a human or animal. In one aspect the disclosure provides a vaccine for inducing an immune response in a human or animal. In some embodiments the immune response is the generation of antibodies against one or more antigens comprised in the payload. In some embodiments the animal is a fish or other marine animal, such as salmonids, catfish, carps, sea bass, flat fish, and Tilapia's. In particular: Grass carp (*Ctenopharyngodon idella*), Silver carp (*Hypophthalmichthys molitrix*), Catla (*Cyprinus catla* or *Gibelion catla*), Common Carp (*Cyprinus carpio*), Bighead carp (*Hypophtha*/*michthys nobilis* or *Aristichthys nobilis*), Crucian carp (*Carassius carassius*), *Oreochromis niloticus* Nile Tilapia (*Oreochromis niloticus*), Mozambique Tilapia (*Oreochromis mossambicus*) and other Tilapia's, Pangas catfishes (*Pangasius pangasius*), Roho (*Labeo rohita*), Atlantic salmon (*Salmo salar*), Arctic charr (*Salvelinus alpinus*), brown trout (*Salmo trutta*), rainbow trout (*Oncorhynchus mykiss*), sea trout (*Salmo trutta*) and other trouts.

Described herein are methods of reducing the likelihood of contracting a condition associated with infection by a pathogen in a human or animal (such as a fish or other marine animal), comprising administering an immunologically effective dose of a vaccine of the disclosure. For example, the method may reduce the likelihood of contracting a condition associated with infection by, i) bacterial pathogens, for example *Vibrio anguillarum* & *Vibrio salmonicida* (vibriosis), *Aeromonas salmonicida* & *Aeromonas hydrophila* (furunculosis), *Yersinia ruckeri* (Enteric Redmouth Disease), *Renibacterium salmonis, Lactococcus sp., Streptococcus sp.,* and *Piscirickettsia salmonis*; (ii) viral pathogens, for example Infectious pancreatic necrosis virus (IPNV), Infectious Salmon Anemia virus (ISAV), Salmon Pancreas Disease virus (SPD virus), Sleeping Disease of Trout virus (SDV), Viral Nervous Necrosis virus (VNNV) and Infectious Heamatopoietic Necrosis virus (IHNV), Viral haemorrhagic septicaemia virus (VHSV); (iii) Fungal pathogens, for example *Saprolegnia sp.* (such as *S*. *parasitica, S. diclina and S. australis*) or *Aphanomyces* sp. (*A. invadans* and *A. astaci*) and *Branchiomyces sp.* (Gill rot); and/or (iv) Parasitic pathogens, for example *Ichthyophthirius multifiliis, Cryptocaryon irritans* and *Trichodina sp.* (Trichodiniasis), Amoebic gill disease (eg. *Neoparamoeba perurans*), sea lice (copepods within the order *Siphonostomatoida,* family *Caligidae* (for example *Lepeophtheirus salmonis, Caligus rogercresseyi, Caligus clemensi, Caligus elongatus*), proliferative kidney disease or PKD (*Tetracapsuloides bryosalmonae*) and other species)

Thus, the vaccines can be utilized in a vaccine strategy to induce an immune response in a human or animal. The vaccines can comprise an adjuvant and be administered in an effective amount to a human or animal (such as a fish or other marine animal) in order to elicit an immune response. In some cases, the compositions are administered without an adjuvant to a human or animal (such as a fish or other marine animal).

The vaccines of the present disclosure can be administered using any technique currently utilized in the art. Suitable dosing regimens are preferably determined taking into account factors well known in the art including age, weight, sex and medical condition of the subject; the route of administration; the desired effect; and the particular composition employed. The vaccine can be used in multi-dose vaccination formats.

A dose may consist of the range from about 1 µg to about 1.0 mg of the composition per subject kg. In another embodiment of the present disclosure the range is from about 0.01 mg to 1.0 mg of the composition per subject kg. However, one may prefer to adjust dosage based on the amount of protein delivered. In either embodiment, these ranges are guidelines. More precise dosages can be determined by assessing the immunogenicity of the composition so that an immunologically effective dose is delivered. An immunologically effective dose is one that stimulates the immune system of the patient to establish an immunological response. Preferably, the level of immune system stimulation will be sufficient to develop an immunological memory sufficient to provide long term protection against disease caused by the pathogenic organism from which the payload antigen(s) originates.

In some embodiments, the animal to be vaccinated is a fish and the composition of the disclosure is delivered by immersing the fish in an immersion solution comprising the composition of the invention.

Thus, described herein is a method for vaccinating a fish or other marine animal, the method comprising: (i) providing an immersion solution comprising a composition of the disclosure; (ii) immersing a fish or other marine animal to be vaccinated in the immersion solution; (iii) incubating the fish or other marine animal to be vaccinated in the immersion solution for a treatment period.

In some preferred embodiments of the above immersion vaccination methods, the composition of is administered without an adjuvant.

In some embodiments, the animal to be vaccinated is a fish and the composition (or composition nucleic acid) is delivered by injection. In preferred embodiments, the compositions are administered without an adjuvant.

In the above immersion or injection vaccination methods, the composition of the disclosure will have a payload comprising an antigen from a fish or other marine animal pathogen. For example, an antigen from one or more of *Vibrio anguillarum, Vibrio salmonicida, Aeromonas salmonicida, Aeromonas hydrophila, Yersinia ruckeri, Renibacterium salmonis, Lactococcus sp., Streptococcus sp., Piscirickettsia salmonis,* Infectious pancreatic necrosis virus (IPNV), Infectious Salmon Anemia virus (ISAV), Salmon Pancreas Disease virus (SPDV), Sleeping Disease of Trout virus (SDV), Viral Nervous Necrosis virus(VNNV), Infectious Heamatopoietic Necrosis virus (IHNV), *Saprolegnia sp.* (such as *S*. *parasitica, S. diclina and S*. *australis*) or), *Aphanomyces* sp. (*A. invadans* and *A. astaci*)), *Branchiomyces sp., Ichthyophthirius multifiliis, Cryptocaryon irritans* and *Trichodina sp.,* Amoebic gill disease (eg. *Neoparamoeba perurans*), sea lice (copepods within the order *Siphonostomatoida,* family *Caligidae* (for example *Lepeophtheirus salmonis, Caligus rogercresseyi, Caligus clemensi, Caligus elongatus*), proliferative kidney disease or PKD (*Tetracapsuioides bryosalmonae*).

In the above immersion vaccination methods, the concentration of the composition of the disclosure in the immersion solution will be sufficient to allow for vaccination of the immersed fish in a reasonable period of time. For example, the concentration of composition in the immersion solution can be from 0.1 to 10µM, such as 1 to 5µM, 2 to 4µM or around 3µM. The treatment period can be, for example, 15 min to 3 hours, such as 30 mins to 2 hours or 1 to 1.5 hours. Other useful treatment period are, for example, 3 hours to 6 hours, such as 4 hours to 5 hours or at least 6 hours.

Accordingly, described herein is a method for vaccinating a fish or other marine animal, the method comprising: (i) providing an immersion solution comprising a composition of the disclosure at a concentration between 0.1 and 10 µM, wherein the composition has a payload comprising an antigen from a fish or other marine animal pathogen; (ii) immersing a fish or other marine animal to be vaccinated in the immersion solution; (iii) incubating the fish or other marine animal to be vaccinated in the immersion solution for a treatment period from 15 minutes to 3 hours. Preferably, the method is performed without the use of an adjuvant.

Preferably, an immune (e.g. antibody) response will be elicited in a fish or other marine animal subjected to the above immersion vaccination method. For example, at a timepoint 8 weeks after vaccination the fish or other marine animal may have a blood titer of at least 1:8, for example at least 1:16 or at least 1:32, such as at least 1:64, at least 1:128, at least 1:256, at least 1:512, or at least 1:1024. In some embodiments the fish or other marine animal has have a blood titer of at least 1:32 at a timepoint 8 weeks after vaccination. In some embodiments a titer of "at least 1:x" means the antibody is antibody is detectable against the antigen at that dilution via ELISA.

Thus, described herein is the invention a method for manufacturing antibodies the method comprising the steps of: (i) vaccinating a fish (or other marine animal) with a composition or composition nucleic acid of the disclosure so as to elicit an antibody production; (ii) harvesting the antibodies from the fish (or other marine animal).

The present disclosure provides a composition for use in the above immersion or injection vaccination methods. In an embodiment, the disclosure provides a composition or composition nucleic acid for use in immersion or injection vaccination of a fish or other marine animal without the use of an adjuvant. The present disclosure also provides an immersion solution for use in the above immersion vaccination methods.

Also described herein is a composition of the disclosure for use in immersion vaccination of a fish or other marine animal without the use of an adjuvant, the composition having a payload comprising an antigen from a fish pathogen; wherein immersion of the fish or other marine animal for a treatment period from 15 minutes to 3 hours in an immersion solution containing the composition of the disclosure at a concentration between 0.1 and 10 µM) elicits an immune (e.g antibody) response in the fish or other marine animal.

The present disclosure provides a composition of the disclosure for use in the immersion or injection vaccination of fish or other marine animal.

In some embodiments the composition is delivered by oral administration in food i.e. the composition is combined with food prior to administration. Thus, the present disclosure provides human or animal feed comprising: (i) a composition of the disclosure, and (ii) human or animal foodstuff. For example, in the vaccination of fish or other marine animal the animal feed would comprise the composition together with typical fish or other marine animal food stuffs such as fish meal, fish oil, starch (wheat, maize gluten, sunflower meal). The present disclosure further comprises a method for vaccinating a human or animal (such as a fish or other marine animal) by feeding the human or animal the feed.

The timing of doses depends upon factors well known in the art. After the initial administration one or more booster doses may subsequently be administered to maintain antibody titers. An example of a dosing regime would be a dose on day 1, a second dose at 1 or 2 months, a third dose at either 3, 4, 5, 6, 7, 8, 9, 10, 11, or 12 months or greater than 12 months, and additional booster doses at distant times as needed.

Decisions on dosage etc, is within the responsibility of medical practitioners, and typically takes account of the disorder to be treated or prevented, the condition of the individual patient, the site of delivery, the method of administration and other factors known to practitioners. Examples of the techniques and protocols mentioned above can be found in Remington's Pharmaceutical Sciences, 16th edition, Osol, A. (ed), 1980.

The immune response so generated can be completely or partially protective against disease and symptoms caused by infection with the pathogenic organism from which the payload antigen(s) originates.

### FIGURES

Figure 1. Amino acid sequences of SpHtp1 fragments
Figure 2.
   A) Beakers with X-ray Tetra's swimming in a suspension of tank water and 3µM SpHtp1-mRFP, or 3µM mRFP, or no protein added (blanc). Fish remained in the suspension for one hour.
   B) Finn, gill and bone tissue were crudely dissected and placed under the Confocal Microscope after one hour of immersion. The 'no protein' control is not shown, but there was no red-fluorescence observed in the fishes.

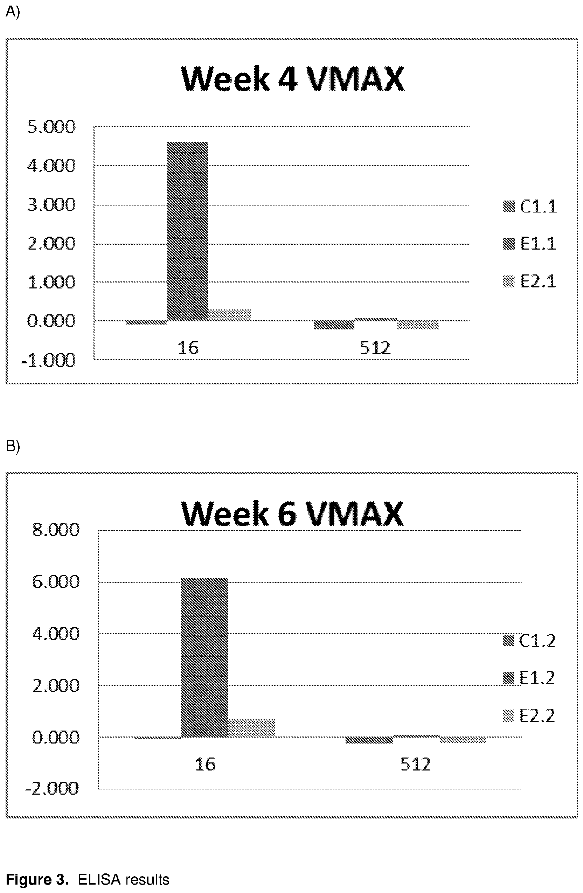

Figure 3. ELISA results after immersion treatment of rainbow trout
   C1.1 = Salmon treated with mRFP(His)₆ only
   E1.1 = Salmon treated with SpHtp1²⁴⁻⁶⁸mRFP(His)₆
   E2.1 = Salmon treated with SpHtp1²⁴⁻⁶⁸mRFP(His)₆

Graphs show the Vₘₐₓ rates of the detection at two different serum dilutions tested ("16" and "512").

### EXAMPLES

### Example 1: ELISA results - Testing for rainbow trout IgG capable to bind mRFP(His)₆ after immersion vaccination (see also Figure 3)

Three salmon were exposed to protein constructs by immersion; two experimental animals (E1.1 & E2.1) were exposed to were exposed to SpHtp1²⁴⁻⁶⁸:mRFP(His)₆, whilst one control animal (C1.1) was exposed to mRFP(His)₆ only. Exposure was by 1.5 hour immersion in tank water comprising 3 µM of the relevant composition.

After 4 and 6 week blood samples were taken and tested by ELISA for binding to mRFP(His)₆: the results are shown in Figure 3. As can be seen, both experimental fish test positive for antibodies against mRFP. Whilst lower than E1.1, the antibody levels observed in E2.1 are sufficient for antibody immunity. It is also noted that variation in immune response between individuals is not unexpected.

Note that it is likely a higher antibody titer would have been recorded at 8 weeks. However, the data at 8 weeks was not obtained as the experiment was prematurely terminated.

## Claims

1. A composition for use in the immersion vaccination of fish or other marine animal comprising: (i) a translocation sequence comprising a polypeptide having at least 90% sequence identity to the SpHtp1²⁴⁻⁶⁸ or SpHtp1a²⁴⁻⁶⁹ shown in Figure 1; and (ii) a heterologous payload coupled to the translocation sequence;
wherein the heterologous payload comprises an antigen from a fish or other marine animal pathogen, and wherein the heterologous payload is:
(a) a polypeptide from an organism or virus selected from *Vibrio anguillarum, Vibrio salmonicida, Aeromonas salmonicida, Aeromonas hydrophila, Yersinia ruckeri, Renibacterium salmonis, Lactococcus sp., Streptococcus sp., Piscirickettsia salmonis,* Infectious pancreatic necrosis virus (IPNV), Infectious Salmon Anemia virus (ISAV), Salmon Pancreas Disease virus (SPD virus), Sleeping Disease of Trout virus (SDV), Viral Nervous Necrosis virus (VNNV) and Infectious Heamatopoietic Necrosis virus (IHNV), Viral haemorrhagic septicaemia virus (VHSV), S. *diclina, S. australis, A. invadans, A. astaci, Branchiomyces sp., Ichthyophthirius multifiliis, Cryptocaryon irritans, Trichodina sp., Neoparamoeba perurans, Lepeophtheirus salmonis, Caligus rogercresseyi, Caligus clemensi, Caligus elongates or Tetracapsuloides bryosalmonae*; or
(b) a polypeptide, or polypeptide encoded by a sequence selected from the following, or a fragment thereof:
Genbank accession numbers ACY35988.1, ACY35989.1, ACY35990.1, EU625675.1, FJ594325.1, AJ012631.1, AJ238578.1, X89213.1, EU481506.1, ACA34520.1, ACA34521.1, ACA34522.1, ACA34523.1, ACA34524.1, or ACA34525.1.

2. The composition for use of claim 1 wherein the composition comprising: (i) a translocation sequence consisting of a polypeptide having the sequence of SpHtp1²⁴⁻⁶⁸ or SpHtp1a²⁴⁻⁶⁹ shown in Figure 1; and (ii) a heterologous payload coupled to the translocation sequence, wherein the heterologous payload comprises an antigen from a fish or other marine animal pathogen.

3. The composition for use of either one of claims 1 or 2 wherein the composition is a fusion protein.

4. A nucleic acid encoding the composition of any one of claims 1 to 3.

5. An expression vector comprising the nucleic acid of claim 4.

6. A host cell comprising the expression vector of claim 5.

7. A vaccine for use in the immersion vaccination of fish or other marine animal, the vaccine comprising a composition according to any one of claim 1 to 3, optionally in combination with a pharmaceutically acceptable excipient, carrier, buffer or stabilizer.

8. The vaccine for use of claim 7, wherein the immersion vaccination induces an immune response in a fish or other marine animal, optionally wherein the immune response is the generation of antibodies against one or more payload antigens.

9. The vaccine for use of claim 7, wherein the immersion vaccination reduces the likelihood of a fish or other marine animal contracting a condition associated with infection by a pathogen, the method comprising administering an immunologically effective dose of the vaccine of claim 7.

10. The vaccine for use according to claim 7, the immersion vaccination method comprising:
(i) providing an immersion solution comprising a composition according to any one of claims 1 to 3;
(ii) immersing the fish or other marine animal in the immersion solution;
(iii) incubating the fish or other marine animal in the immersion solution for a treatment period.

11. The vaccine for use according to claim 10, wherein the immersion solution contains the composition at a concentration of between 0.1 and 10 µM and the treatment period is between 15 minutes and 3 hours.

12. The vaccine for use according to either one of claims 10 or 11 wherein the method is performed without the use of an adjuvant.

13. A composition for use in a method for vaccinating a fish or other marine animal, the method comprising:
(i) providing an immersion solution comprising the composition;
(ii) immersing the fish or other marine animal in the immersion solution;
(iii) incubating the fish or other marine animal in the immersion solution for a treatment period;
wherein the composition comprises: (i) a translocation sequence comprising a polypeptide having at least 90% sequence identity to the SpHtp1²⁴⁻⁶⁸ or SpHtp1a²⁴⁻⁶⁹ shown in Figure 1; and (ii) a heterologous payload coupled to the translocation sequence, wherein the heterologous payload comprises an antigen from a fish or other marine animal pathogen.

14. The composition for use according to claim 13, wherein the immersion solution contains the composition at a concentration of between 0.1 and 10 µM and the treatment period is between 15 minutes and 3 hours.

15. The composition for use according to either one of claims 13 to 14, wherein the method is performed without the use of an adjuvant.

## Patentansprüche

1. Zusammensetzung zur Verwendung in der Immersionsimpfung eines Fisches oder anderen Meerestieres, die Folgendes umfasst: (i) eine Translokationssequenz, umfassend ein Polypeptid mit zumindest 90 % Sequenzidentität mit dem in Figur 1 gezeigten SpHtp1²⁴⁻⁶⁸ oder SpHtp1a²⁴⁻⁶⁹; und (ii) eine heterologe Nutzlast, die mit der Translokationssequenz verbunden ist;
wobei die heterologe Nutzlast ein Antigen von einem Fisch- oder anderen Meerestierpathogen umfasst und wobei die heterologe Nutzlast Folgendes ist:
(a) ein Polypeptid von einem Organismus oder Virus, der/das aus Vibrio anguillarum, Vibrio salmonicida, Aeromonas salmonicida, Aeromonas hydrophila, Yersinia ruckeri, Renibacterium salmonis, Lactococcus sp., Streptococcus sp., Piscirickettsia salmonis, infektiösem Pankreasnekrosevirus (IPNV), infektiösem Lachsanämievirus (ISAV), Lachspankreas-Erkrankungsvirus (SPD-Virus), Forellen-Narkolepsie-Virus (SDV), Virus, das virale Nervennekrose hervorruft (VNNV), und Virus, das infektiöse hämatopoetische Nekrose hervorruft (IHNV), Virus, das virale hämorrhagische Septikämie hervorruft (VHSV), S. diclina, S. australis, A. invadans, A. astaci, Branchiomyces sp., Ichthyophthirius multifiliis, Cryptocaryon irritans, Trichodina sp., Neoparamoeba perurans, Lepeophtheirus salmonis, Caligus rogercresseyi, Caligus clemensi, Caligus elongates oder Tetracapsuloides bryosalmonae ausgewählt ist; oder
(b) ein Polypeptid oder Polypeptid, für das eine Sequenz kodiert, die aus den folgenden ausgewählt ist, oder ein Fragment davon:
Genbank-Zugriffsnummern ACY35988.1, ACY35989.1, ACY35990.1, EU625675.1, FJ594325.1, AJ012631.1, AJ238578.1, X89213.1, EU481506.1, ACA34520.1, ACA34521.1, ACA34522.1, ACA34523.1, ACA34524.1 oder ACA34525.1.

2. Zusammensetzung zur Verwendung nach Anspruch 1, wobei die Zusammensetzung Folgendes umfasst: (i) eine Translokationssequenz, bestehend aus einem Polypeptid mit der Sequenz des in Figur 1 gezeigten SpHtp1²⁴⁻⁶⁸ oder SpHtp1a²⁴⁻⁶⁹; und (ii) eine heterologe Nutzlast, die mit der Translokationssequenz verbunden ist, wobei die heterologe Nutzlast ein Antigen von einem Fisch- oder anderen Meerestierpathogen umfasst.

3. Zusammensetzung zur Verwendung nach einem der Ansprüche 1 oder 2, wobei die Zusammensetzung ein Fusionsprotein ist.

4. Nucleinsäure, die für eine Zusammensetzung nach einem der Ansprüche 1 bis 3 kodiert.

5. Expressionsvektor, der eine Nucleinsäure nach Anspruch 4 umfasst.

6. Wirtszelle, die den Expressionsvektor nach Anspruch 5 umfasst.

7. Vakzine zur Verwendung in der Immersionsimpfung eines Fisches oder anderen Meerestieres, wobei die Vakzine eine Zusammensetzung nach einem der Ansprüche 1 bis 3 umfasst, gegebenenfalls in Kombination mit einem pharmazeutisch annehmbaren Exzipienten, Träger, Puffer oder Stabilisator.

8. Vakzine zur Verwendung nach Anspruch 7, wobei die Immersionsimpfung eine Immunantwort bei einem Fisch oder anderen Meerestier hervorruft, wobei die Immunantwort gegebenenfalls die Produktion von Antikörpern gegen ein oder mehrere Nutzlastantigene ist.

9. Vakzine zur Verwendung nach Anspruch 7, wobei die Immersionsimpfung die Wahrscheinlichkeit verringert, dass ein Fisch oder anderes Meerestier an einem Leiden erkrankt, das mit einer Infektion durch ein Pathogen assoziiert wird, wobei das Verfahren die Verabreichung einer immunologisch wirksamen Dosis der Vakzine nach Anspruch 7 umfasst.

10. Vakzine zur Verwendung nach Anspruch 7, wobei das Immersionsvakzine-Verfahren Folgendes umfasst:
(i) Bereitstellen einer Immersionslösung, umfassend eine Zusammensetzung nach einem der Ansprüche 1 bis 3;
(ii) Eintauchen des Fisches oder anderen Meerestieres in die Immersionslösung;
(iii) Inkubieren des Fisches oder anderen Meerestieres einen Behandlungszeitraum lang in der Immersionslösung.

11. Vakzine zur Verwendung nach Anspruch 10, wobei die Immersionslösung die Zusammensetzung in einer Konzentration zwischen 0,1 und 10 µM enthält und der Behandlungszeitraum zwischen 15 min und 3 h beträgt.

12. Vakzine zur Verwendung nach einem der Ansprüche 10 oder 11, wobei das Verfahren ohne Verwendung eines Adjuvans durchgeführt wird.

13. Zusammensetzung zur Verwendung in einem Verfahren zur Impfung eines Fisches oder anderen Meerestieres, wobei das Verfahren Folgendes umfasst:
(i) Bereitstellen einer Immersionslösung, die die Zusammensetzung umfasst;
(ii) Eintauchen des Fisches oder anderen Meerestieres in die Immersionslösung;
(iii) Inkubieren des Fisches oder anderen Meerestieres einen Behandlungszeitraum lang in der Immersionslösung;
wobei die Zusammensetzung Folgendes umfasst: (i) eine Translokationssequenz, umfassend ein Polypeptid mit zumindest 90 % Sequenzidentität mit dem in Figur 1 gezeigten SpHtp1²⁴⁻⁶⁸ oder SpHtp1a²⁴⁻⁶⁹; und (ii) eine heterologe Nutzlast, die mit der Translokationssequenz verbunden ist, wobei die heterologe Nutzlast ein Antigen von einem Fisch- oder anderen Meerestierpathogen umfasst.

14. Zusammensetzung zur Verwendung nach Anspruch 13, wobei die Immersionslösung die Zusammensetzung in einer Konzentration zwischen 0,1 und 10 µM enthält und der Behandlungszeitraum zwischen 15 min und 3 h beträgt.

15. Zusammensetzung zur Verwendung nach einem der Ansprüche 13 oder 14, wobei das Verfahren ohne die Verwendung eines Adjuvans erfolgt.

## Revendications

1. Composition destinée à être utilisée dans la vaccination par immersion d'un poisson ou d'un autre animal marin comprenant : (i) une séquence de translocation comprenant un polypeptide présentant au moins 90 % d'identité de séquence avec la SpHtp1²⁴⁻⁶⁸ ou la SpHtp1a²⁴⁻⁶⁹ montrée sur la Figure 1 ; et (ii) une charge hétérologue couplée à la séquence de translocation ;
dans laquelle la charge hétérologue comprend un antigène d'un agent pathogène de poisson ou d'un autre animal marin, et dans laquelle la charge hétérologue est :
(a) un polypeptide d'un organisme ou d'un virus sélectionné parmi *Vibrio anguillarum, Vibrio salmonicida, Aeromonas salmonicida, Aeromonas hydrophila, Yersinia ruckeri, Renibacterium salmonis, Lactococcus sp.*, *Streptococcus sp., Piscirickettsia salmonis,* le virus de la nécrose pancréatique infectieuse (IPNV), le virus de l'anémie infectieuse du saumon (ISAV), le virus de la maladie du pancréas du saumon (virus SPD), le virus de la maladie du sommeil de la truite (SDV), le virus de la nécrose nerveuse virale (VNNV) et le virus de la nécrose hématopoïétique infectieuse (IHNV), le virus de la septicémie hémorragique virale (VHSV), *S. diclina, S. australis, A. invadans, A. astaci, Branchiomyces sp., Ichthyophthirius multifiliis, Cryptocaryon irritans, Trichodina sp., Neoparamoeba perurans, Lepeophtheirus salmonis, Caligus rogercresseyi, Caligus clemensi, Caligus elongates* ou *Tetracapsuloides bryosalmonae* ; ou
(b) un polypeptide, ou un polypeptide codé par une séquence sélectionnée parmi les suivantes, ou un fragment de celles-ci :
les numéros d'accès Genbank ACY35988.1, ACY35989.1, ACY35990.1, EU625675.1, FJ594325.1, AJ012631.1, AJ238578.1, X89213.1, EU481506.1, ACA34520.1, ACA34521.1, ACA34522.1, ACA34523.1, ACA34524.1, ou ACA34525.1.

2. Composition destinée à être utilisée selon la revendication 1, la composition comprenant : (i) une séquence de translocation consistant un polypeptide possédant la séquence de la SpHtp1²⁴⁻⁶⁸ ou la SpHtp1a²⁴⁻⁶⁹ montrée sur la Figure 1 ; et (ii) une charge hétérologue couplée à la séquence de translocation, dans laquelle la charge hétérologue comprend un antigène d'un agent pathogène de poisson ou d'un autre animal marin.

3. Composition destinée à être utilisée selon l'une quelconque des revendications 1 ou 2, où la composition est une protéine de fusion.

4. Acide nucléique codant pour la composition selon l'une quelconque des revendications 1 à 3.

5. Vecteur d'expression comprenant l'acide nucléique selon la revendication 4.

6. Cellule hôte comprenant le vecteur d'expression selon la revendication 5.

7. Vaccin destiné à être utilisé dans la vaccination par immersion d'un poisson ou d'un autre animal marin, le vaccin comprenant une composition selon l'une quelconque des revendications 1 à 3, facultativement en combinaison avec un excipient, véhicule, tampon ou stabilisateur pharmaceutiquement acceptable.

8. Vaccin destiné à être utilisé selon la revendication 7, dans lequel la vaccination par immersion induit une réponse immunitaire chez un poisson ou un autre animal marin, facultativement où la réponse immunitaire est la génération d'anticorps dirigés contre un ou plusieurs antigènes de la charge.

9. Vaccin destiné à être utilisé selon la revendication 7, dans lequel la vaccination par immersion réduit la probabilité qu'un poisson ou un autre animal marin contracte une affection associée à une infection par un agent pathogène, le procédé comprenant l'administration d'une dose immunologiquement efficace du vaccin selon la revendication 7.

10. Vaccin destiné à être utilisé selon la revendication 7, le procédé de vaccination par immersion comprenant :
(i) la mise à disposition d'une solution d'immersion comprenant une composition selon l'une quelconque des revendications 1 à 3 ;
(ii) l'immersion du poisson ou d'un autre animal marin dans la solution d'immersion ;
(iii) l'incubation du poisson ou d'un autre animal marin dans la solution d'immersion pendant une période de traitement.

11. Vaccin destiné à être utilisé selon la revendication 10, où la solution d'immersion contient la composition à une concentration comprise entre 0,1 et 10 µM et la période de traitement est comprise entre 15 minutes et 3 heures.

12. Vaccin destiné à être utilisé selon l'une quelconque des revendications 10 ou 11, où le procédé est réalisé sans utiliser d'adjuvant.

13. Composition destinée à être utilisée dans un procédé de vaccination d'un poisson ou d'un autre animal marin, le procédé comprenant :
(i) la mise à disposition d'une solution d'immersion comprenant la composition ;
(ii) l'immersion du poisson ou d'un autre animal marin dans la solution d'immersion ;
(iii) l'incubation du poisson ou d'un autre animal marin dans la solution d'immersion pendant une période de traitement ;
où la composition comprend : (i) une séquence de translocation comprenant un polypeptide présentant au moins 90 % d'identité de séquence avec la SpHtp1²⁴⁻⁶⁸ ou la SpHtp1a²⁴⁻⁶⁹ montrée sur la Figure 1 ; et (ii) une charge hétérologue couplée à la séquence de translocation, dans laquelle la charge hétérologue comprend un antigène d'un agent pathogène de poisson ou d'un autre animal marin.

14. Composition destinée à être utilisée selon la revendication 13, où la solution d'immersion contient la composition à une concentration comprise entre 0,1 et 10 µM et la période de traitement est comprise entre 15 minutes et 3 heures.

15. Composition destinée à être utilisée selon l'une quelconque des revendications 13 à 14, où le procédé est réalisé sans utiliser d'adjuvant.


### SEQUENCE LISTING

<110> The University Court of the University of Aberdeen
<120> Fish Vaccine
<130> RKA/BP6985352
<150> GB 1309664.9
   <151> 2013-05-30
<160> 33
<170> PatentIn version 3.3
<210> 1
   <211> 198
   <212> PRT
   <213> Saprolegnia parasitica
<400> 1
   Met Arg Ile His His Pro Leu Thr Leu Ala Ala Leu Cys Val Val Leu
   1               5                   10                  15
   His Glu Ser Leu Gly Ala Ala Gln His Ser Asn Asn Val Ala Arg Leu
               20                  25                  30
   Glu His Tyr Arg Ile Ala Glu Ile Glu His Trp Glu Lys Arg His Leu
           35                  40                  45
   Arg Ser Asp Ser Arg Gly His Arg His His Ala His His Gly Gln Val
       50                  55                  60
   Ile Asp Lys Glu Asn Asn Asn Ser Gln Glu Gln Ala Thr Thr Gly Asn
   65                  70                  75                  80
   Ser Val Glu Thr Asn Gln Val Pro Ser Thr Glu Pro Thr Lys Asp Lys
                   85                  90                  95
   Thr Thr Pro Met Lys Asn Ala Leu Phe Lys Leu Phe Arg Glu Lys Lys
               100                 105                 110
   Leu Lys Thr Lys Asn Ala Gly Asn Gly His Ala His Asp Asp Asp Asp
           115                 120                 125
   Asp Ser Asp Phe Ser Asp Asp Asp Val Pro Thr Asn Ala Pro Thr Asp
       130                 135                 140
   Ala Pro Thr Gly Ala Pro Thr Asp Ala Pro Thr Asp Ala Pro Thr Val
   145                 150                 155                 160
   Ala Pro Thr Asp Ala Pro Thr Asp Ala Pro Thr Glu Ala Pro Thr Asn
                   165                 170                 175
   Ala Pro Thr Gly Thr Asp Ala Pro Thr Asp Ala Pro Thr Asp Ala Gln
               180                 185                 190
   Val Val Pro Thr Phe Asp
           195
<210> 2
   <211> 175
   <212> PRT
   <213> Saprolegnia parasitica
<400> 2
   Gln His Ser Asn Asn Val Ala Arg Leu Glu His Tyr Arg Ile Ala Glu
   1               5                   10                  15
   Ile Glu His Trp Glu Lys Arg His Leu Arg Ser Asp Ser Arg Gly His
               20                  25                  30
   Arg His His Ala His His Gly Gln Val Ile Asp Lys Glu Asn Asn Asn
           35                  40                  45
   Ser Gln Glu Gln Ala Thr Thr Gly Asn Ser Val Glu Thr Asn Gln Val
       50                  55                  60
   Pro Ser Thr Glu Pro Thr Lys Asp Lys Thr Thr Pro Met Lys Asn Ala
   65                  70                  75                  80
   Leu Phe Lys Leu Phe Arg Glu Lys Lys Leu Lys Thr Lys Asn Ala Gly
                   85                  90                  95
   Asn Gly His Ala His Asp Asp Asp Asp Asp Ser Asp Phe Ser Asp Asp
               100                 105                 110
   Asp Val Pro Thr Asn Ala Pro Thr Asp Ala Pro Thr Gly Ala Pro Thr
           115                 120                 125
   Asp Ala Pro Thr Asp Ala Pro Thr Val Ala Pro Thr Asp Ala Pro Thr
       130                 135                 140
   Asp Ala Pro Thr Glu Ala Pro Thr Asn Ala Pro Thr Gly Thr Asp Ala
   145                 150                 155                 160
   Pro Thr Asp Ala Pro Thr Asp Ala Gln Val Val Pro Thr Phe Asp
                   165                 170                 175
<210> 3
   <211> 45
   <212> PRT
   <213> Saprolegnia parasitica
<400> 3
   Gln His Ser Asn Asn Val Ala Arg Leu Glu His Tyr Arg Ile Ala Glu
   1               5                   10                  15
   Ile Glu His Trp Glu Lys Arg His Leu Arg Ser Asp Ser Arg Gly His
               20                  25                  30
   Arg His His Ala His His Gly Gln Val Ile Asp Lys Glu
           35                  40                  45
<210> 4
   <211> 130
   <212> PRT
   <213> Saprolegnia parasitica
<400> 4
   Asn Asn Asn Ser Gln Glu Gln Ala Thr Thr Gly Asn Ser Val Glu Thr
   1               5                   10                  15
   Asn Gln Val Pro Ser Thr Glu Pro Thr Lys Asp Lys Thr Thr Pro Met
               20                  25                  30
   Lys Asn Ala Leu Phe Lys Leu Phe Arg Glu Lys Lys Leu Lys Thr Lys
           35                  40                  45
   Asn Ala Gly Asn Gly His Ala His Asp Asp Asp Asp Asp Ser Asp Phe
       50                  55                  60
   Ser Asp Asp Asp Val Pro Thr Asn Ala Pro Thr Asp Ala Pro Thr Gly
   65                  70                  75                  80
   Ala Pro Thr Asp Ala Pro Thr Asp Ala Pro Thr Val Ala Pro Thr Asp
                   85                  90                  95
   Ala Pro Thr Asp Ala Pro Thr Glu Ala Pro Thr Asn Ala Pro Thr Gly
               100                 105                 110
   Thr Asp Ala Pro Thr Asp Ala Pro Thr Asp Ala Gln Val Val Pro Thr
           115                 120                 125
   Phe Asp
       130
<210> 5
   <211> 221
   <212> PRT
   <213> Saprolegnia parasitica
<400> 5
   Met Arg Ile Tyr His Pro Leu Thr Leu Ala Ala Leu Cys Val Val Leu
   1               5                   10                  15
   His Glu Ser Leu Gly Ala Ala Gln His Ser Asn Asn Val Ala Arg Leu
               20                  25                  30
   Glu His Tyr Ser Val Ala Glu Ile Glu His Trp Glu Lys Arg His Leu
           35                  40                  45
   Arg Asn Ser Asp Ser Arg Gly His Arg His His Ala His His Gly Gln
       50                  55                  60
   Val Ile Asp Lys Glu Asn Asn Asn Ser Gln Glu Gln Ala Thr Thr Gly
   65                  70                  75                  80
   Asn Ser Val Glu Thr Asn Gln Val Pro Phe Thr Gln Pro Thr Lys Asp
                   85                  90                  95
   Lys Thr Ala Ser Ile Lys Thr Ala Val Phe Lys Leu Leu Arg Glu Asn
               100                 105                 110
   Lys Leu Lys Thr Lys Asn Ala Gly Asn Gly His Ala His Asp Asp Asp
           115                 120                 125
   Asp Asp Asp Asp Ser Asn Phe Ser Asp Asp Asp Val Pro Thr Asn Ala
       130                 135                 140
   Pro Thr Gly Ala Pro Thr Gly Ala Pro Thr Asp Ala Pro Thr Asn Ala
   145                 150                 155                 160
   Pro Thr Asp Ala Pro Thr Asp Ala Pro Thr Asp Ala Pro Thr Asp Ala
                   165                 170                 175
   Pro Thr Asp Ala Pro Thr Asp Ala Pro Thr Asp Ala Pro Thr Thr Leu
               180                 185                 190
   Pro Pro Met Pro Arg Arg Arg Ser His Arg Cys Pro Asp Arg His Ser
           195                 200                 205
   His Arg Cys Asp Pro Arg Arg Thr His Gly Cys Pro Asp
       210                 215                 220
<210> 6
   <211> 198
   <212> PRT
   <213> Saprolegnia parasitica
<400> 6
   Gln His Ser Asn Asn Val Ala Arg Leu Glu His Tyr Ser Val Ala Glu
   1               5                   10                  15
   Ile Glu His Trp Glu Lys Arg His Leu Arg Asn Ser Asp Ser Arg Gly
               20                  25                  30
   His Arg His His Ala His His Gly Gln Val Ile Asp Lys Glu Asn Asn
           35                  40                  45
   Asn Ser Gln Glu Gln Ala Thr Thr Gly Asn Ser Val Glu Thr Asn Gln
       50                  55                  60
   Val Pro Phe Thr Gln Pro Thr Lys Asp Lys Thr Ala Ser Ile Lys Thr
   65                  70                  75                  80
   Ala Val Phe Lys Leu Leu Arg Glu Asn Lys Leu Lys Thr Lys Asn Ala
                   85                  90                  95
   Gly Asn Gly His Ala His Asp Asp Asp Asp Asp Asp Asp Ser Asn Phe
               100                 105                 110
   Ser Asp Asp Asp Val Pro Thr Asn Ala Pro Thr Gly Ala Pro Thr Gly
           115                 120                 125
   Ala Pro Thr Asp Ala Pro Thr Asn Ala Pro Thr Asp Ala Pro Thr Asp
       130                 135                 140
   Ala Pro Thr Asp Ala Pro Thr Asp Ala Pro Thr Asp Ala Pro Thr Asp
   145                 150                 155                 160
   Ala Pro Thr Asp Ala Pro Thr Thr Leu Pro Pro Met Pro Arg Arg Arg
                   165                 170                 175
   Ser His Arg Cys Pro Asp Arg His Ser His Arg Cys Asp Pro Arg Arg
               180                 185                 190
   Thr His Gly Cys Pro Asp
           195
<210> 7
   <211> 46
   <212> PRT
   <213> Saprolegnia parasitica
<400> 7
   Gln His Ser Asn Asn Val Ala Arg Leu Glu His Tyr Ser Val Ala Glu
   1 5 10 15
   Ile Glu His Trp Glu Lys Arg His Leu Arg Asn Ser Asp Ser Arg Gly
   20 25 30
   His Arg His His Ala His His Gly Gln Val Ile Asp Lys Glu
   35 40 45
<210> 8
   <211> 152
   <212> PRT
   <213> Saprolegnia parasitica
<400> 8
   Asn Asn Asn Ser Gln Glu Gln Ala Thr Thr Gly Asn Ser Val Glu Thr
   1 5 10 15
   Asn Gln Val Pro Phe Thr Gln Pro Thr Lys Asp Lys Thr Ala Ser Ile
   20 25 30
   Lys Thr Ala Val Phe Lys Leu Leu Arg Glu Asn Lys Leu Lys Thr Lys
   35 40 45
   Asn Ala Gly Asn Gly His Ala His Asp Asp Asp Asp Asp Asp Asp Ser
   50 55 60
   Asn Phe Ser Asp Asp Asp Val Pro Thr Asn Ala Pro Thr Gly Ala Pro
   65 70 75 80
   Thr Gly Ala Pro Thr Asp Ala Pro Thr Asn Ala Pro Thr Asp Ala Pro
   85 90 95
   Thr Asp Ala Pro Thr Asp Ala Pro Thr Asp Ala Pro Thr Asp Ala Pro
   100 105 110
   Thr Asp Ala Pro Thr Asp Ala Pro Thr Thr Leu Pro Pro Met Pro Arg
   115 120 125
   Arg Arg Ser His Arg Cys Pro Asp Arg His Ser His Arg Cys Asp Pro
   130 135 140
   Arg Arg Thr His Gly Cys Pro Asp
   145 150
<210> 9
   <211> 260
   <212> PRT
   <213> Infectious pancreatic necrosis virus
<400> 9
   Met Leu Pro Glu Asn Gly Pro Ala Ser Ile Pro Asp Asp Ile Thr Glu
   1               5                   10                  15
   Arg His Ile Leu Lys Gln Glu Thr Ser Ser Tyr Asn Leu Glu Val Ser
               20                  25                  30
   Asp Ser Gly Ser Gly Leu Leu Val Cys Phe Pro Gly Ala Pro Gly Ser
           35                  40                  45
   Arg Val Gly Ala His Tyr Arg Trp Asn Leu Asn Gln Thr Glu Leu Glu
       50                  55                  60
   Phe Asp Arg Trp Leu Glu Thr Ser Gln Asp Leu Lys Lys Ala Phe Asn
   65                  70                  75                  80
   Tyr Gly Arg Leu Ile Ser Arg Lys Tyr Asp Ile Gln Ser Ser Thr Leu
                   85                  90                  95
   Pro Ala Gly Leu Tyr Ala Leu Asn Gly Thr Leu Asn Ala Ala Thr Phe
               100                 105                 110
   Glu Gly Ser Leu Ser Glu Val Glu Ser Leu Thr Tyr Asn Ser Leu Met
           115                 120                 125
   Ser Leu Thr Thr Asn Pro Gln Asp Lys Val Asn Asn Gln Leu Val Thr
       130                 135                 140
   Lys Gly Ile Thr Val Leu Asn Leu Pro Thr Gly Phe Asp Lys Pro Tyr
   145                 150                 155                 160
   Val Arg Leu Glu Asp Glu Thr Pro Gln Gly Ser Gln Ser Met Asn Gly
                   165                 170                 175
   Ala Arg Met Arg Cys Thr Ala Ala Ile Ala Pro Arg Arg Tyr Glu Ile
               180                 185                 190
   Asp Leu Pro Ser Glu Arg Leu Pro Thr Val Ala Ala Thr Gly Thr Pro
           195                 200                 205
   Thr Thr Ile Tyr Glu Gly Asn Ala Asp Ile Val Asn Ser Thr Thr Val
       210                 215                 220
   Thr Gly Asp Val Thr Phe Gln Leu Ala Ala Glu Pro Ala Asn Glu Thr
   225                 230                 235                 240
   Arg Phe Asp Phe Ile Leu Gln Phe Leu Gly Leu Asp Asn Asp Val Pro
   245 250 255
   Val Val Ser Val
   260
<210> 10
   <211> 260
   <212> PRT
   <213> Infectious pancreatic necrosis virus
<400> 10
   Met Leu Pro Glu Thr Gly Pro Ala Ser Ile Pro Asp Asp Ile Thr Glu
   1 5 10 15
   Arg His Ile Leu Lys Gln Glu Thr Ser Ser Tyr Asn Leu Glu Val Ser
   20 25 30
   Glu Ser Gly Ser Gly Leu Leu Val Cys Phe Pro Gly Ala Pro Gly Ser
   35 40 45
   Arg Ile Gly Ala His Tyr Arg Trp Asn Ala Asn Gln Thr Gly Leu Glu
   50 55 60
   Phe Asp Gln Trp Leu Glu Thr Ser Gln Asp Leu Lys Lys Ala Phe Asn
   65 70 75 80
   Tyr Gly Arg Leu Ile Ser Arg Lys Tyr Asp Ile Gln Ser Ser Thr Leu
   85 90 95
   Pro Ala Gly Leu Tyr Ala Leu Asn Gly Thr Leu Asn Ala Ala Thr Phe
   100 105 110
   Glu Gly Ser Leu Ser Glu Val Glu Ser Leu Thr Tyr Asn Ser Leu Met
   115 120 125
   Ser Leu Thr Thr Asn Pro Gln Asp Lys Val Asn Asn Gln Leu Val Thr
   130 135 140
   Lys Gly Val Thr Val Leu Asn Leu Pro Thr Gly Phe Asp Lys Pro Tyr
   145 150 155 160
   Val Arg Leu Glu Asp Glu Thr Pro Gln Gly Leu Gln Ser Met Asn Gly
   165 170 175
   Ala Lys Met Arg Cys Thr Ala Ala Thr Ala Pro Arg Arg Tyr Glu Ile
   180 185 190
   Asp Leu Pro Ser Gln Arg Leu Pro Pro Val Pro Ala Thr Gly Thr Leu
   195 200 205
   Thr Thr Leu Tyr Glu Gly Asn Ala Asp Ile Val Asn Ser Thr Thr Val
   210 215 220
   Thr Gly Asp Ile Asn Phe Ser Leu Ala Glu Gln Pro Ala Val Glu Thr
   225 230 235 240
   Lys Phe Asp Phe Gln Leu Asp Phe Met Gly Leu Asp Asn His Val Pro
   245 250 255
   Val Val Thr Val
   260
<210> 11
   <211> 260
   <212> PRT
   <213> Infectious pancreatic necrosis virus
<400> 11
   Met Leu Pro Glu Thr Gly Pro Ala Ser Ile Pro Asp Asp Ile Thr Glu
   1 5 10 15
   Arg His Ile Leu Lys Gln Glu Thr Ser Ser Tyr Asn Leu Glu Val Ser
   20 25 30
   Glu Ser Gly Ser Gly Ile Leu Val Cys Phe Pro Gly Ala Pro Gly Ser
   35 40 45
   Arg Ile Gly Ala His Tyr Arg Trp Asn Ala Asn Gln Thr Gly Leu Glu
   50 55 60
   Phe Asp Gln Trp Leu Glu Thr Ser Gln Asp Leu Lys Lys Ala Phe Asn
   65 70 75 80
   Tyr Gly Arg Leu Ile Ser Arg Lys Tyr Asp Ile Gln Ser Ser Thr Leu
   85 90 95
   Pro Ala Gly Leu Tyr Ala Leu Asn Gly Thr Leu Asn Ala Ala Thr Phe
   100 105 110
   Glu Gly Ser Leu Ser Glu Val Glu Ser Leu Thr Tyr Asn Ser Leu Met
   115 120 125
   Ser Leu Thr Thr Asn Pro Gln Asp Lys Val Asn Asn Gln Leu Val Thr
   130 135 140
   Lys Gly Val Thr Val Leu Asn Leu Pro Thr Gly Phe Asp Lys Pro Tyr
   145                 150                 155                 160
   Val Arg Leu Glu Asp Glu Thr Pro Gln Gly Leu Gln Ser Met Asn Gly
                   165                 170                 175
   Ala Lys Met Arg Cys Thr Ala Ala Ile Ala Pro Arg Arg Tyr Glu Ile
               180                 185                 190
   Asp Leu Pro Ser Gln Arg Leu Pro Pro Val Pro Ala Thr Gly Ala Leu
           195                 200                 205
   Thr Thr Leu Tyr Glu Gly Asn Ala Asp Ile Val Asn Ser Thr Thr Val
       210                 215                 220
   Thr Gly Asp Ile Asn Phe Ser Leu Ala Glu Gln Pro Ala Val Glu Thr
   225                 230                 235                 240
   Lys Phe Asp Phe Gln Leu Asp Phe Met Gly Leu Asp Asn Asp Val Pro
                   245                 250                 255
   Val Val Thr Val
               260
<210> 12
   <211> 1122
   <212> DNA
   <213> Infectious salmon anemia virus
<400> 12
   atggcacgat tcataatttt attcctactg ttggcgcctg tttacagtcg tctatgtctt     60
   agaaactacc ctgacaccac ctggttaggt gactctcgaa gcgatcagtc cagagtgaat    120
   ccacagtctt tggatctggt gactgagttc aagggggtgc tgcaggccaa aaacggaaat    180
   ggacttttga agcagatgag tggaaggttt ccaagtgact ggtacacacc tactacaaag    240
   taccggatcc tatacttggg aaccaatgac tgcactgacg gacctactga catgatcatc    300
   ccaacttcga tgacactgga caacgcggca agggagctgt acctgggagc atgcagggga    360
   gacgtgagag tgacgcctac atttgtggga gcagcaattg ttggacttgt tggacgaaca    420
   gacgcaatta ccggtttttc ggtgaaggtg ttgactttca acagccctac aattgtagtg    480
   gtgggattga atggaatgtc cggaatctac aaggtctgca ttgcagcaac atctgggaat    540
   gtgggaggag tgacacttat caacggatgc ggatatttca acacaccttt gaggtttgac    600
   aatttccaag gacaaatcta cgtgtcagac acctttgaag tgaggggaac caaaaacaag    660
   tgtgttctgc taagatcttc tagtgatacg cctttgtgtt cacacatcat gaggaacgtt    720
   gagttagatg agtatgtaga cacaccaaat acagggggtg tttatccttc tgatggtttt    780
   gattcactac atggttcagc ttccgttaga acgttcctca ctgatgcatt gacatgccca    840
   gacattgact ggagtagaat tgatgctgct tcgtgtgaat atgacagctg ccctaagatg    900
   gttaaagatt ttgaccagac aagcttaggt aacacagaca cacttatcat gagggaggta    960
   gcattgcaca aggagatgat cagtaaactt cagaggaaca tcacagatgt aaaaacatct   1020
   gtgttgagca acatcttcat ttctatgggt gtagcaggtt ttgggattgc tctgttccta   1080
   gcaggttgga aggcatgtat ttggattgca gcattcatgt ac                      1122
<210> 13
   <211> 1186
   <212> DNA
   <213> Infectious salmon anemia virus
<400> 13
   atggcacgat tcataatttt attcctactg ttggcgcctg tttacagtcg tctatgtctt      60
   agaaactacc ctgacaccac ctggttaggt gactctcgaa gcgatcagtc cagagtgaat     120
   ccacagtctt tggatctggt gactgagttc aagggggtgc tgcaggccaa aaacggaaat    180
   ggacttttga agcagatgag tggaaggttt ccaagtgact ggtacacacc tactacaaag     240
   taccggatcc tatacttggg aaccaatgac tgcactgacg gacctactga catgatcatc     300
   ccaactccga tgacactgga caacgcggca agggagctgt acctgggagc atgcagggga     360
   gacgtgagag tgacgcctac atttgtggga gcagcaattg ttggacttgt tggacgaaca     420
   gacgcaatta ccggtttttc ggtgaaggtg ttgactttca acagctctac aattgtagtg     480
   gtgggattga atggaatgtc cggaatctac aaggtctgca ttgcagcaac atctgggaat     540
   gtgggaggag tgacacttat caacggatgc ggatatttca acacaccttt gaggtttgac     600
   aatttccaag gacaaatcta cgtgtcagac acctttgaag tgaggggaac caaaaacaag     660
   tgtgttctgc taagatcttc tagtgatacg cctttgtgtt cacacatcat gaggaacgtt     720
   gagttagatg agtatgtaga cacaccaaat acagggggtg tttatccttc tgatggtttt    780
   gattcactac atggttcagc ttccgttaga acgttcctca ctgatgcatt gacatgccca     840
   gacattgact ggagtagaat tgatgctgct tcgtgtgaat atgacagctg ccctaagatg     900
   gttaaagatt ttgaccagac aagcttaggt aacacagaca cacttatcat gagggaggta     960
   gcattgcaca aggagatgat cagtaaactt cagaggaaca tcacagatgt aaaaacatct    1020
   gtgttgagca acatcttcat ttctatgggt gtagcaggtt ttgggattgc tctgttccta    1080
   gcaggttgga aggcatgtat ttggattgca gcattcatgt acaagtctag aggtagaatt    1140
   ccaccatcga acctgtttgt tgcttgatta tgaaagaaag acaacc                   1186
<210> 14
   <211> 374
   <212> PRT
   <213> Infectious salmon anemia virus
<400> 14
   Met Ala Arg Phe Ile Ile Leu Phe Leu Leu Leu Ala Pro Val Tyr Ser
   1               5                   10                  15
   Arg Leu Cys Leu Arg Asn Tyr Pro Asp Thr Thr Trp Leu Gly Asp Ser
               20                  25                  30
   Arg Ser Asp Gln Ser Arg Val Asn Pro Gln Ser Leu Asp Leu Val Thr
           35                  40                  45
   Glu Phe Lys Gly Val Leu Gln Ala Lys Asn Gly Asn Gly Leu Leu Lys
       50                  55                  60
   Gln Met Ser Gly Arg Phe Pro Ser Asp Trp Tyr Thr Pro Thr Thr Lys
   65                  70                  75                  80
   Tyr Arg Ile Leu Tyr Leu Gly Thr Asn Asp Cys Thr Asp Gly Pro Thr
                   85                  90                  95
   Asp Met Ile Ile Pro Thr Ser Met Thr Leu Asp Asn Ala Ala Arg Glu
               100                 105                 110
   Leu Tyr Leu Gly Ala Cys Arg Gly Asp Val Arg Val Thr Pro Thr Phe
           115                 120                 125
   Val Gly Ala Ala Ile Val Gly Leu Val Gly Arg Thr Asp Ala Ile Thr
       130                 135                 140
   Gly Phe Ser Val Lys Val Leu Thr Phe Asn Ser Pro Thr Ile Val Val
   145                 150                 155                 160
   Val Gly Leu Asn Gly Met Ser Gly Ile Tyr Lys Val Cys Ile Ala Ala
                   165                 170                 175
   Thr Ser Gly Asn Val Gly Gly Val Thr Leu Ile Asn Gly Cys Gly Tyr
               180                 185                 190
   Phe Asn Thr Pro Leu Arg Phe Asp Asn Phe Gln Gly Gln Ile Tyr Val
           195                 200                 205
   Ser Asp Thr Phe Glu Val Arg Gly Thr Lys Asn Lys Cys Val Leu Leu
       210                 215                 220
   Arg Ser Ser Ser Asp Thr Pro Leu Cys Ser His Ile Met Arg Asn Val
   225                 230                 235                 240
   Glu Leu Asp Glu Tyr Val Asp Thr Pro Asn Thr Gly Gly Val Tyr Pro
                   245                 250                 255
   Ser Asp Gly Phe Asp Ser Leu His Gly Ser Ala Ser Val Arg Thr Phe
               260                 265                 270
   Leu Thr Asp Ala Leu Thr Cys Pro Asp Ile Asp Trp Ser Arg Ile Asp
           275                 280                 285
   Ala Ala Ser Cys Glu Tyr Asp Ser Cys Pro Lys Met Val Lys Asp Phe
       290                 295                 300
   Asp Gln Thr Ser Leu Gly Asn Thr Asp Thr Leu Ile Met Arg Glu Val
   305                 310                 315                 320
   Ala Leu His Lys Glu Met Ile Ser Lys Leu Gln Arg Asn Ile Thr Asp
                   325                 330                 335
   Val Lys Thr Ser Val Leu Ser Asn Ile Phe Ile Ser Met Gly Val Ala
               340                 345                 350
   Gly Phe Gly Ile Ala Leu Phe Leu Ala Gly Trp Lys Ala Cys Ile Trp
           355                 360                 365
   Ile Ala Ala Phe Met Tyr
       370
<210> 15
   <211> 388
   <212> PRT
   <213> Infectious salmon anemia virus
<400> 15 Leu Phe Val Ala
385
<210> 16
   <211> 5178
   <212> DNA
   <213> Salmon Pancreas Disease virus
<400> 16
   actatggact cagcggcaat gaacgtggag gcttttaaaa gtttcgcctg taaggacacc     60
   gacctgtgga ctgagttcgc ggaaaaacca gtaaggttgt cgcccggcca aatcgaagag    120
   tatgtctttc atctacaagg ggccaaggcc aatgtgatgc acagcagagt cgaagccgta    180
   tgccctgacc tctcggaggt ggctatggac aggttcacac tagacatgaa acgcgacgtc    240
   aaagtgacgc caggcacgaa gcacgtagag gagagaccta aagtccaaga gattcaagcg     300
   gccgacccca tggccaccgc gtacttgtgc gccatccata gagagctagt ccgaaggctg     360
   aaggccgtcc tgaaaccgtc tatacacgtg ttgttcgata tgagctccga ggattttgat    420
   gctatcgtgg gccatgggat gaagttgggt gacaaggtgc tggaaacgga catctcctca    480
   ttcgacaaga gccaggacca agccatggcg gttacagcgc tgatgctgct gagggacttg    540
   ggagtagaag aagacctcct gaccctaatt gaggcgtctt tcggcgacat cacttctgcc    600
   cacctgccca caggcaccag atttcagttt ggatcgatga tgaagtctgg actttttctg    660
   acgctgttcg tgaacacgct gcttaacatc accatagctg cccgagtttt acgggagcag    720
   ctggctgata ccaggtgtgc cgcgtttatc ggtgacgaca acgtaatcac cggagtagtg    780
   tctgacgaca tgatggtggc caggtgcgca tcctggctga acatggaggt gaagatcatg    840
   gacatggaaa ttggcaacat gagtccttat ttttgtggcg gcttcctgtt actcgacacg    900
   gtaacaggca ctgtaagccg agtgtcggac cctgtaaaac gcctgatgaa gatgggaaaa    960
   ccggccctga acgatccaga aacggacgtg gacagatgcc gcgcactgcg cgaagaagtg   1020
   gaaagctggt acagagtggg gattcagtgg ccactgcagg tggctgccgc cacacgctat   1080
   ggcgtgaacc acctgccgct ggccacaatg gcgatggcca cgctcgccca ggacttgaga   1140
   tcgtacctgg gcgcgcgagg ggagtacgta tccctctacg tctaacctta atattttctg 1200
   catcatactt ccaaacaatc atgtttccca tgcaattcac caactcagcc tatcgccaga 1260
   tggagcccat gtttgcaccg ggttcccgag gacaagtaca gccgtaccgg ccgcgcacta 1320
   agcgccgcca ggagccgcaa gtcggcaacg ccgccattac tgccctcgcg aaccagatga 1380
   gtgcgctcca gttgcaggta gctggacttg ccggccaggc aagggtggac cgccgtgggc 1440
   caagacgtgt tcagaagaac aagcagaaga agaagaactc ttccaacgga gaaaaaccca 1500
   aagagaagaa gaagaagcaa aaacaacagg agaagaaggg aagcggtggc gaaaaagtca 1560
   agaagactag gaaccgaccc gggaaggagg taaggatctc cgtaaagtgt gcccgacaga 1620
   gcaccttccc cgtgtaccac gaaggtgcta tatccggcta cgctgtgctg attggatctc 1680
   gcgtattcaa gccggcacac gtgaagggta agatcgacca ccctgaactg gcagacatca 1740
   agttccaggt cgccgaggac atggacctcg aagcagctgc gtacccgaag agcatgcgag 1800
   accaagcggc tgaaccagcg accatgatgg acagagtgta caactgggag tatggcacta 1860
   tcagagtgga ggataatgtc ataatcgacg caagcggtag gggcaagccg ggtgacagtg 1920
   gcagggccat caccgacaac tcgggaaagg ttgttggtat tgtcctcgga ggaggacccg 1980
   atggcaggcg cacacgcctc tccgtgatag gtttcgacaa gaagatgaag gctagggaga 2040
   tcgcctacag tgatgccata ccttggacac gcgctccggc cctcctgctg ctgcctatgg 2100
   ttattgtctg cacctacaat tccaacacct tcgattgctc caaaccgtcc tgccaggact 2160
   gctgcattac tgctgaacca gagaaggcca tgaccatgct gaaggacaat ctgaacgacc 2220
   cgaactactg ggacctactc attgctgtca ccacctgtgg ctccgcccgg agaaagaggg 2280
   ctgtgtctac gtcgcctgcc gccttttacg acacacagat cctcgccgcc cacgcagctg 2340
   cctccccata cagggcgtac tgccccgatt gtgacggaac agcgtgtatc tcgccgatag 2400
   ccatcgacga ggtggtgagc agtggcagcg accacgtcct ccgcatgcgg gttggttctc 2460
   aatcgggagt gaccgctaag ggtggtgcgg cgggtgagac ctctctgcga tacctgggaa 2520
   gggacgggaa ggttcacgcc gcagacaaca cgcgactcgt ggtgcgcacg actgcaaagt 2580
   gcgacgtgct gcaggccact ggccactaca tcctggccaa ctgcccagtg gggcagagcc 2640
   taaccgttgc ggccacactg gatggcaccc ggcatcaatg caccacggtt ttcgaacacc 2700
   aagtaacgga gaagttcacc agagaacgca gcaagggcca ccatctgtcc gacatgacca 2760
   agaaatgcac cagattttcc actacaccaa aaaagtccgc cctctacctc gttgatgtgt 2820
   atgacgctct gccgatttct gtagagatta gcaccgtcgt aacatgcagc gacagccagt 2880
   gcacagtgag ggtgccacct ggtaccacag tgaaattcga caagaaatgc aagagcgctg 2940
   actcggcaac cgtcactttc accagcgact cccagacgtt tacgtgtgag gagccagtcc 3000
   taacggctgc cagtatcacc cagggcaagc cacacctcag atcggcaatg ttgcctagcg 3060
   gaggcaagga agtgaaagca aggatcccgt tcccgttccc gccggaaacc gcaacttgca 3120
   gagtgagtgt agccccactg ccgtcgatca cctacgagga aagcgatgtc ctgctagccg 3180
   gtaccgcaaa ataccctgtg ctgctaacca cacggaacct tggtttccat agcaacgcca 3240
   catccgaatg gatccagggc aagtacctgc gccgcatccc ggtcacgcct caagggatcg 3300
   agctaacatg gggaaacaac gcgccgatgc acttttggtc atccgtcagg tacgcatccg 3360
   gggacgctga tgcgtacccc tgggaacttc tggtgtacca caccaagcac catccagagt 3420
   acgcgtgggc gtttgtagga gttgcatgcg gcctgctggc tatcgcagcg tgcatgtttg 3480
   cgtgcgcatg cagcagggtg cggtactctc tggtcgccaa cacgttcaac tcgaacccac 3540
   caccattgac cgcactgact gcagcactgt gttgcatacc aggggctcgc gcggaccaac 3600
   cctacttgga catcattgcc tactttttag gggtaagagg gtggtcagcc ctgctggtca 3660
   tccttgcgta tgtacagagc tgcaagagct acgaacacac cgtggtggtc ccaatggatc 3720
   caagagcccc gtcgtacgaa gcagtgataa accggaatgg gtatgatcca ttgaagctga 3780
   ccatctcagt gaatttcacc gtcatctcac caactacggc tctggaatat tggacctgcg 3840
   caggagtccc catcgtcgag ccgccccatg tgggctgctg cacgtcggtg tcctgcccct 3900
   ctgacctctc tacgctgcat gcgtttactg gcaaagctgt ctccgacgtg cactgcgatg 3960
   tgcacacaaa cgtgtacccc ttgttgtggg gcgcggctca ctgcttctgt tccaccgaga 4020
   atacacaggt cagcgctgtg gcagccaccg tttctgagtt ctgtgcccag gactcagagc 4080
   gtgccgaagc gttcagcgta cacagcagct cagtcaccgc tgaggtcctg gtgacgcttg 4140
   gtgaagtggt gacggcagtc cacgtttacg tggacggggt aacatcagcc aggggcactg 4200
   acctcaagat cgtggctgga ccaataacaa ccgactactc cccattcgat cgcaaagtag 4260
   tccgcatcgg cgaagaggtc tataactatg actggcctcc ttacggggct ggccgaccag 4320
   gcacattcgg agacattcaa gctaggtcaa ccaactatgt caaacccaac gatctgtatg 4380
   gggacatcgg aattgaagta ctgcagccga ctaacgacca cgtacatgtg gcttacacgt 4440
   atacgacctc tgggttactg cgttggctgc aggacgctcc gaaaccactc agtgtcacag 4500
   caccgcacgg ttgtaagatc agtgccaatc cgctcctggc cctcgattgt ggggttggtg 4560
   ccgtccccat gtccatcaac attccggacg cgaagtttac ccgcaaatta aaggatccga 4620
   aaccatcggc cctgaaatgc gtggtggaca gctgcgagta cggggtggac tacgggggcg 4680
   ccgccacgat cacctacgag ggccacgagg ccgggaagtg cgggattcat tccctgacac 4740
   caggagtccc cctgagaaca tcggtggttg aagtggttgc tggcgccaat accgtcaaaa 4800
   cgaccttctc ctcacccacg cccgaggttg cactcgaggt agagatctgt tcggcaatag 4860
   tgaagtgcgc tggtgagtgc actccaccga aggaacatgt ggtcgcaacc aggcctcgcc 4920
   atggcagcga ccctggaggc tacatctccg ggcccgcaat gcgctgggcc ggagggattg 4980
   tagggaccct agtggtcctg ttccttatcc ttgccgtcat ctactgcgtg gtgaagaagt 5040
   gccgctccaa aagaatccgg atagtcaaga gctaaattcc ggtatacaaa ttgctcacta 5100
   ggagcccatc cgatcccaca gggagtagga tgagtcatct attggtttta aaattttcaa 5160
   tacaaaaaaa aaaaaaaa 5178
<210> 17
   <211> 394
   <212> PRT
   <213> Salmon pancreas disease virus
<400> 17
   Thr Met Asp Ser Ala Ala Met Asn Val Glu Ala Phe Lys Ser Phe Ala
   1               5                   10                  15
   Cys Lys Asp Thr Asp Leu Trp Thr Glu Phe Ala Glu Lys Pro Val Arg
               20                  25                  30
   Leu Ser Pro Gly Gln Ile Glu Glu Tyr Val Phe His Leu Gln Gly Ala
           35                  40                  45
   Lys Ala Asn Val Met His Ser Arg Val Glu Ala Val Cys Pro Asp Leu
       50                  55                  60
   Ser Glu Val Ala Met Asp Arg Phe Thr Leu Asp Met Lys Arg Asp Val
   65                  70                  75                  80
   Lys Val Thr Pro Gly Thr Lys His Val Glu Glu Arg Pro Lys Val Gln
                   85                  90                  95
   Glu Ile Gln Ala Ala Asp Pro Met Ala Thr Ala Tyr Leu Cys Ala Ile
               100                 105                 110
   His Arg Glu Leu Val Arg Arg Leu Lys Ala Val Leu Lys Pro Ser Ile
           115                 120                 125
   His Val Leu Phe Asp Met Ser Ser Glu Asp Phe Asp Ala Ile Val Gly
       130                 135                 140
   His Gly Met Lys Leu Gly Asp Lys Val Leu Glu Thr Asp Ile Ser Ser
   145                 150                 155                 160
   Phe Asp Lys Ser Gln Asp Gln Ala Met Ala Val Thr Ala Leu Met Leu
                   165                 170                 175
   Leu Arg Asp Leu Gly Val Glu Glu Asp Leu Leu Thr Leu Ile Glu Ala
               180                 185                 190
   Ser Phe Gly Asp Ile Thr Ser Ala His Leu Pro Thr Gly Thr Arg Phe
           195                 200                 205
   Gln Phe Gly Ser Met Met Lys Ser Gly Leu Phe Leu Thr Leu Phe Val
       210                 215                 220
   Asn Thr Leu Leu Asn Ile Thr Ile Ala Ala Arg Val Leu Arg Glu Gln
   225                 230                 235                 240
   Leu Ala Asp Thr Arg Cys Ala Ala Phe Ile Gly Asp Asp Asn Val Ile
                   245                 250                 255
   Thr Gly Val Val Ser Asp Asp Met Met Val Ala Arg Cys Ala Ser Trp
               260                 265                 270
   Leu Asn Met Glu Val Lys Ile Met Asp Met Glu Ile Gly Asn Met Ser
           275                 280                 285
   Pro Tyr Phe Cys Gly Gly Phe Leu Leu Leu Asp Thr Val Thr Gly Thr
       290                 295                 300
   Val Ser Arg Val Ser Asp Pro Val Lys Arg Leu Met Lys Met Gly Lys
   305                 310                 315                 320
   Pro Ala Leu Asn Asp Pro Glu Thr Asp Val Asp Arg Cys Arg Ala Leu
                   325                 330                 335
   Arg Glu Glu Val Glu Ser Trp Tyr Arg Val Gly Ile Gln Trp Pro Leu
               340                 345                 350
   Gln Val Ala Ala Ala Thr Arg Tyr Gly Val Asn His Leu Pro Leu Ala
           355                 360                 365
   Thr Met Ala Met Ala Thr Leu Ala Gln Asp Leu Arg Ser Tyr Leu Gly
       370                 375                 380
   Ala Arg Gly Glu Tyr Val Ser Leu Tyr Val
   385                 390
<210> 18
   <211> 4179
   <212> DNA
   <213> Sleeping disease virus
<400> 18
   cacaatggcg atggccacgc tcgcccagga tctgagatcg tacttgggcg cgcgagggga 60
   gtacgtatcc ctctacgcct aaccttaata ttttctgcat catactctca accaaccatg 120
   tttcccatgc aattcaccaa ctcagcctat cgccagatgg agcccatgtt cgcaccggct 180
   tctcgaggac aagtacagcc gtatcggccg cgcacaaagc gccgccaaga gccgcaagtc 240
   ggcaacgctg ctattgctgc cctcgcgaac cagatgagcg cgctccagct gcaggtggct 300
   ggacttgccg gccaggcaag gttggaccgt cctggaccga gactttttcc agaaaaaaca 360
   agcagaagaa gaagaactct tccaacggag aaaaaccccc ggagaagaag aaagaagcca 420
   aaaccacctg gagaagaaag ggaacggcgg ggaaaaagcc aagaaacccc cggaaccggc 480
   cccggaaaga agttaggatc tccgttaagc gtgcccgaca gaacaccttc cccgttgtac 540
   catgacggtg ccatatccgg ctatgcggtg ctgattggct cccgcgtgtt taagccagcg 600
   cacgtgaagg gtaagttcga ccaccccgaa ctggcggaca tcaagttcca ggtcgccgag     660
   gtcatggacc tcgaagcagc cgcataccct aagtgcatgc gagaccaggc ggctgaacca     720
   gcaaccatga tggatggagt gtacaatggg gagtacggca atattcagga gtggaggaca 780
   attttgtatt cgatgcgagc ggcagaggca agccggggtg acagtggcag gccattcacc 840
   gacaactcag gaaaggttgt cggtatcgtc ctcggaggag gacccgatgg taggcgcaca 900
   cgtctctccg tgataggttt cgacaagaag ctgaaggcca gagagatcgc ctacagcgag 960
   gccatccctt ggacacgcgc accagctctc ctgctgctgc ctatggtcat cgcctgcacc 1020
   tacaactcca atacctttga ttgctccaaa ccgtcctgcc aggattgttg cattactgct 1080
   gaaccaaaga aggccatgac tatgctgaag gacaacctga atgacccgaa ctactgggac 1140
   ctgctcattg ccgtcaccac ctgcagttcc gcccgaaaaa agagggctgt gtctacgtcg 1200
   cctgtcgccg tttacgacac acaaattctc gccgcccacg cagctgcctc cccgtatagg 1260
   gcgtactgcc ccgattgtga cggaactgcc tgcatctcgc cgatagctat cgacgaggtg 1320
   gtaagtagcg gtagtgacca cgtccttcgc atccgggtcg gttctcaatc gggagtgacc 1380
   gctaaaggcg gtgcggcggg tgaaacctct ctgcgatacc tgggaaggga cggtaaggtt 1440
   tacgccgcgg acaacacgcg gctcgtggtg cgcaccactg caaagtgtga cgtgctgcag 1500
   gccactggcc actacattct ggccaactgc ccagtggggc agagtctcac tgttgcggcc 1560
   acactggacg gtacccggca tcaatgcacc acggttttcg aacatcaagt aacggagaag 1620
   ttcacaagag aacgcagcaa gggccaccac ctgtccgatc tgaccaagaa atgcaccagg 1680
   ttctccacca ccccgaagaa gtccgcgctc tatctcgttg atgtgtatga tgctctgccg 1740
   acttctgtag agatcagcac cgtggtgaca tgcaacgaaa gacagtgcac agtgagggtg 1800
   ccacccggta ccacagtgaa attcgataag aggtgcaaga acgctgccaa agagaccgtc 1860
   accttcacca gcgactccca gacgtttacg tgcgaggagc cggtcctaac ggccgccagc 1920
   atcacccagg gcaagccgca cctcagatcg tcaatgttgc ccagcggagg caaagaggtg 1980
   aaagcgagga ttccattccc gttcccgcca gagactgcga cttgcagagt gagcatcgcc 2040
   ccactgccat cgattaccta tgaggaaagc gatgttctgc tggccggcac tgcgaaatac 2100
   cccgtgctgc taactacacg gaaccttggt ttccatagca acgccacatc tgaatggatc 2160
   cagggtaagt acctgcgccg catcccggtc acgccccaag ggattgaact aatgttggga 2220
   aacaacgcac cgctgcactt ctggtcatct gtcaggtacg catctggaga cgccgacgcg 2280
   tacccctggg aacttctggt gcaccacatc aagcaccatc cggagtacgc gtgggcgttt 2340
   gtaggagttg catgtggcct gctggccgtt gcagcatgca tgttcgcgtg cgcatgcaac 2400
   agggtgcggt actctctgct cgccaacacg ttcaacccga acccaccacc attgaccgca 2460
   ctgactgcag cattgtgctg catacctggg gctcgcgcgg atcaacccta cctggacatc 2520
   attgcctact tgtggaccaa cagcaaagtg gccttcgggc tgcaatgcgc ggcgcccgtg 2580
   gcttgcatgc tcatcgttac atacgccctt agacattgca gattgtgctg caattctttt 2640
   ttaggggtaa gagggtggtc ggctctgctg gtcatccttg cgtatgtaca gagctgcaag 2700
   gcgtacgaac acaccgtggt ggtcccaatg gatccaagag ccccgtcgta cgaggcggtg 2760
   ataaaccgga atgggtatga ccccctgaag cttaccatcg cagtgaactt taccgtcatc 2820
   tcaccaacta cggctctgga atactggacc tgtgcaggag tccctgtcgt cgagccgccc 2880
   catgtgggct gctgcacgtc agtgtcctgc cctccgacct tctccacgct gcacgcgttc 2940
   accggcaaag ccgtctccga cgtgcactgc gatgtgcaca cgaacgtgta ccccttgttg 3000
   tggggtgcgg ctcactgctt ctgttccact gaaaacacgc aggtcagcgc tgtggccgcc 3060
   accgtttctg agttctgtgc tcaggactca gagcgcgccg aggcgttcag cgttcacagc 3120
   agctcagtca ctgcagagat tctggtgacg cttggtgaag tggtgacggc ggtccacgtt 3180
   tacgtggacg gggtaacatc agccaggggt accgacctca agatcgtggc tggcccaata 3240
   acaactgact actccccgtt tgaccgcaaa gtagtccgta tcggcgaaga ggtctataat 3300
   tacgactggc ctccttacgg ggctggtcga ccaggcacat tcggagacat tcaagctagg 3360
   tcaaccaact atgtcaaacc caatgatctg tacggggaca tcggaattga agtactgcag 3420
   ccgactaatg accacgtgca cgtggcttac acgtatacga cctctgggtt gctgcgttgg 3480
   ttgcaggacg ctccgaaacc actcagtgtc acagcaccgc acggttgtaa gatcagtgct 3540
   aacccgctcc tggccctcga ttgtggggtt ggtgccgtcc ccatgtccat caacattccg 3600
   gacgcgaagt tcacccgcaa actaaaggac ccgaaacctt cggccctgaa atgcgtggtg 3660
   gacagttgcg agtacggggt ggactacggg ggcgccgcca cgatcaccta cgagggccac 3720
   gaggctggga agtgcgggat ccattccctg acaccaggag tccctctgag aacatcagtg 3780
   gttgaagtag ttgccggcgc taataccgtc aaaacgacct tctcctcacc cacgcccgag 3840
   gttacactcg aggtagagat ctgttcggca atagtgaagt gcgccagtga gtgcactcca   3900
   ccgaaggaac acgtagtcgc agccaggcct cgccatggca gcgacactgg aggctacatc   3960
   tccgggcccg caatgcgctg ggccggaagg attgtaggga accctagtgg tcctgtttcc   4020
   tcatccttgg ccgtcaccta ctgcgtggtg aagaagtgcc gctctaaaag aatccggata   4080
   gtcaagagct aaattccggt atacaaatcg ctcactagga gcccatccga acccacaggg   4140
   agtaggatga gtcatctatt ggtttttaaa attttcaat                           4179
<210> 19
   <211> 1322
   <212> PRT
   <213> Sleeping disease virus
<400> 19
   Met Phe Pro Met Gln Phe Thr Asn Ser Ala Tyr Arg Gln Met Glu Pro
   1               5                   10                  15
   Met Phe Ala Pro Ala Ser Arg Gly Gln Val Gln Pro Tyr Arg Pro Arg
               20                  25                  30
   Thr Lys Arg Arg Gln Glu Pro Gln Val Gly Asn Ala Ala Ile Ala Ala
           35                  40                  45
   Leu Ala Asn Gln Met Ser Ala Leu Gln Leu Gln Val Ala Gly Leu Ala
       50                  55                  60
   Gly Gln Ala Arg Val Asp Arg Arg Gly Pro Arg Arg Val Gln Lys Asn
   65                  70                  75                  80
   Lys Gln Lys Lys Lys Asn Ser Ser Asn Gly Glu Lys Pro Lys Glu Lys
                   85                  90                  95
   Lys Lys Lys Gln Lys Gln Gln Glu Lys Lys Gly Ser Gly Gly Glu Lys
               100                 105                 110
   Ala Lys Lys Pro Arg Asn Arg Pro Gly Lys Glu Val Arg Ile Ser Val
           115                 120                 125
   Lys Arg Ala Arg Gln Ser Thr Phe Pro Val Tyr His Asp Gly Ala Ile
       130                 135                 140
   Ser Gly Tyr Ala Val Leu Ile Gly Ser Arg Val Phe Lys Pro Ala His
   145                 150                 155                 160
   Val Lys Gly Lys Phe Asp His Pro Glu Leu Ala Asp Ile Lys Phe Gln
                   165                 170                 175
   Val Ala Glu Val Met Asp Leu Glu Ala Ala Ala Tyr Pro Lys Cys Met
               180                 185                 190
   Arg Asp Gln Ala Ala Glu Pro Ala Thr Met Met Asp Gly Val Tyr Asn
           195                 200                 205
   Gly Glu Tyr Gly Asn Ile Gln Glu Trp Arg Thr Ile Leu Tyr Ser Met
       210                 215                 220
   Arg Ala Ala Glu Ala Ser Arg Gly Asp Ser Gly Arg Pro Phe Thr Asp
   225                 230                 235                 240
   Asn Ser Gly Lys Val Val Gly Ile Val Leu Gly Gly Gly Pro Asp Gly
                   245                 250                 255
   Arg Arg Thr Arg Leu Ser Val Ile Gly Phe Asp Lys Lys Leu Lys Ala
               260                 265                 270
   Arg Glu Ile Ala Tyr Ser Glu Ala Ile Pro Trp Thr Arg Ala Pro Ala
           275                 280                 285
   Leu Leu Leu Leu Pro Met Val Ile Ala Cys Thr Tyr Asn Ser Asn Thr
       290                 295                 300
   Phe Asp Cys Ser Lys Pro Ser Cys Gln Asp Cys Cys Ile Thr Ala Glu
   305                 310                 315                 320
   Pro Lys Lys Ala Met Thr Met Leu Lys Asp Asn Leu Asn Asp Pro Asn
                   325                 330                 335
   Tyr Trp Asp Leu Leu Ile Ala Val Thr Thr Cys Ser Ser Ala Arg Lys
               340                 345                 350
   Lys Arg Ala Val Ser Thr Ser Pro Val Ala Val Tyr Asp Thr Gln Ile
           355                 360                 365
   Leu Ala Ala His Ala Ala Ala Ser Pro Tyr Arg Ala Tyr Cys Pro Asp
       370                 375                 380
   Cys Asp Gly Thr Ala Cys Ile Ser Pro Ile Ala Ile Asp Glu Val Val
   385                 390                 395                 400
   Ser Ser Gly Ser Asp His Val Leu Arg Ile Arg Val Gly Ser Gln Ser
                   405                 410                 415
   Gly Val Thr Ala Lys Gly Gly Ala Ala Gly Glu Thr Ser Leu Arg Tyr
               420                 425                 430
   Leu Gly Arg Asp Gly Lys Val Tyr Ala Ala Asp Asn Thr Arg Leu Val
           435                 440                 445
   Val Arg Thr Thr Ala Lys Cys Asp Val Leu Gln Ala Thr Gly His Tyr
       450                 455                 460
   Ile Leu Ala Asn Cys Pro Val Gly Gln Ser Leu Thr Val Ala Ala Thr
   465                 470                 475                 480
   Leu Asp Gly Thr Arg His Gln Cys Thr Thr Val Phe Glu His Gln Val
                   485                 490                 495
   Thr Glu Lys Phe Thr Arg Glu Arg Ser Lys Gly His His Leu Ser Asp
               500                 505                 510
   Leu Thr Lys Lys Cys Thr Arg Phe Ser Thr Thr Pro Lys Lys Ser Ala
           515                 520                 525
   Leu Tyr Leu Val Asp Val Tyr Asp Ala Leu Pro Thr Ser Val Glu Ile
       530                 535                 540
   Ser Thr Val Val Thr Cys Asn Glu Arg Gln Cys Thr Val Arg Val Pro
   545                 550                 555                 560
   Pro Gly Thr Thr Val Lys Phe Asp Lys Arg Cys Lys Asn Ala Ala Lys
                   565                 570                 575
   Glu Thr Val Thr Phe Thr Ser Asp Ser Gln Thr Phe Thr Cys Glu Glu
               580                 585                 590
   Pro Val Leu Thr Ala Ala Ser Ile Thr Gln Gly Lys Pro His Leu Arg
           595                 600                 605
   Ser Ser Met Leu Pro Ser Gly Gly Lys Glu Val Lys Ala Arg Ile Pro
       610                 615                 620
   Phe Pro Phe Pro Pro Glu Thr Ala Thr Cys Arg Val Ser Ile Ala Pro
   625                 630                 635                 640
   Leu Pro Ser Ile Thr Tyr Glu Glu Ser Asp Val Leu Leu Ala Gly Thr
                   645                 650                 655
   Ala Lys Tyr Pro Val Leu Leu Thr Thr Arg Asn Leu Gly Phe His Ser
               660                 665                 670
   Asn Ala Thr Ser Glu Trp Ile Gln Gly Lys Tyr Leu Arg Arg Ile Pro
           675                 680                 685
   Val Thr Pro Gln Gly Ile Glu Leu Met Leu Gly Asn Asn Ala Pro Leu
       690                 695                 700
   His Phe Trp Ser Ser Val Arg Tyr Ala Ser Gly Asp Ala Asp Ala Tyr
   705                 710                 715                 720
   Pro Trp Glu Leu Leu Val His His Ile Lys His His Pro Glu Tyr Ala
                   725                 730                 735
   Trp Ala Phe Val Gly Val Ala Cys Gly Leu Leu Ala Val Ala Ala Cys
               740                 745                 750
   Met Phe Ala Cys Ala Cys Asn Arg Val Arg Tyr Ser Leu Leu Ala Asn
           755                 760                 765
   Thr Phe Asn Pro Asn Pro Pro Pro Leu Thr Ala Leu Thr Ala Ala Leu
       770                 775                 780
   Cys Cys Ile Pro Gly Ala Arg Ala Asp Gln Pro Tyr Leu Asp Ile Ile
   785                 790                 795                 800
   Ala Tyr Leu Trp Thr Asn Ser Lys Val Ala Phe Gly Leu Gln Cys Ala
                   805                 810                 815
   Ala Pro Val Ala Cys Met Leu Ile Val Thr Tyr Ala Leu Arg His Cys
               820                 825                 830
   Arg Leu Cys Cys Asn Ser Phe Leu Gly Val Arg Gly Trp Ser Ala Leu
           835                 840                 845
   Leu Val Ile Leu Ala Tyr Val Gln Ser Cys Lys Ala Tyr Glu His Thr
       850                 855                 860
   Val Val Val Pro Met Asp Pro Arg Ala Pro Ser Tyr Glu Ala Val Ile
   865                 870                 875                 880
   Asn Arg Asn Gly Tyr Asp Pro Leu Lys Leu Thr Ile Ala Val Asn Phe
                   885                 890                 895
   Thr Val Ile Ser Pro Thr Thr Ala Leu Glu Tyr Trp Thr Cys Ala Gly
               900                 905                 910
   Val Pro Val Val Glu Pro Pro His Val Gly Cys Cys Thr Ser Val Ser
           915                 920                 925
   Cys Pro Ser Asp Leu Ser Thr Leu His Ala Phe Thr Gly Lys Ala Val
       930                 935                 940
   Ser Asp Val His Cys Asp Val His Thr Asn Val Tyr Pro Leu Leu Trp
   945                 950                 955                 960
   Gly Ala Ala His Cys Phe Cys Ser Thr Glu Asn Thr Gln Val Ser Ala
                   965                 970                 975
   Val Ala Ala Thr Val Ser Glu Phe Cys Ala Gln Asp Ser Glu Arg Ala
               980                 985                 990
   Glu Ala Phe Ser Val His Ser Ser  Ser Val Thr Ala Glu  Ile Leu Val
           995                 1000                 1005
   Thr Leu  Gly Glu Val Val Thr  Ala Val His Val Tyr  Val Asp Gly
       1010                 1015                 1020
   Val Thr  Ser Ala Arg Gly Thr  Asp Leu Lys Ile Val  Ala Gly Pro
       1025                 1030                 1035
   Ile Thr  Thr Asp Tyr Ser Pro  Phe Asp Arg Lys Val  Val Arg Ile
       1040                 1045                 1050
   Gly Glu  Glu Val Tyr Asn Tyr  Asp Trp Pro Pro Tyr  Gly Ala Gly
       1055                 1060                 1065
   Arg Pro  Gly Thr Phe Gly Asp  Ile Gln Ala Arg Ser  Thr Asn Tyr
       1070                 1075                 1080
   Val Lys  Pro Asn Asp Leu Tyr  Gly Asp Ile Gly Ile  Glu Val Leu
       1085                 1090                 1095
   Gln Pro  Thr Asn Asp His Val  His Val Ala Tyr Thr  Tyr Thr Thr
       1100                 1105                 1110
   Ser Gly  Leu Leu Arg Trp Leu  Gln Asp Ala Pro Lys  Pro Leu Ser
       1115                 1120                 1125
   Val Thr  Ala Pro His Gly Cys  Lys Ile Ser Ala Asn  Pro Leu Leu
       1130                 1135                 1140
   Ala Leu  Asp Cys Gly Val Gly  Ala Val Pro Met Ser  Ile Asn Ile
       1145                 1150                 1155
   Pro Asp  Ala Lys Phe Thr Arg  Lys Leu Lys Asp Pro  Lys Pro Ser
       1160                 1165                 1170
   Ala Leu Lys Cys Val Val Asp Ser Cys Glu Tyr Gly Val Asp Tyr
       1175                1180                1185
   Gly Gly Ala Ala Thr Ile Thr Tyr Glu Gly His Glu Ala Gly Lys
       1190                1195                1200
   Cys Gly Ile His Ser Leu Thr Pro Gly Val Pro Leu Arg Thr Ser
       1205                1210                1215
   Val Val Glu Val Val Ala Gly Ala Asn Thr Val Lys Thr Thr Phe
       1220                1225                1230
   Ser Ser Pro Thr Pro Glu Val Thr Leu Glu Val Glu Ile Cys Ser
       1235                1240                1245
   Ala Ile Val Lys Cys Ala Ser Glu Cys Thr Pro Pro Lys Glu His
       1250                1255                1260
   Val Val Ala Ala Arg Pro Arg His Gly Ser Asp Thr Gly Gly Tyr
       1265                1270                1275
   Ile Ser Gly Pro Ala Met Arg Trp Ala Gly Arg Ile Val Gly Asn
       1280                1285                1290
   Pro Ser Gly Pro Val Ser Ser Ser Leu Ala Val Thr Tyr Cys Val
       1295                1300                1305
   Val Lys Lys Cys Arg Ser Lys Arg Ile Arg Ile Val Lys Ser
       1310                1315                1320
<210> 20
   <211> 11137
   <212> DNA
   <213> Infectious haematopoietic necrosis virus
<400> 20
   gtataaaaaa agtaacttga ctaagctcag aaggacacaa ggacagaaaa aagaaaaagg      60
   tggcacttca gttgtaacct gataacgtgc ttcagacact ataaaccgag acagaacaag     120
   cagaactatt ttcactgaaa acaacacctg agagacagaa acggatcacg aacgatgaca     180
   agcgcactca gagagacgtt cactggactc agagacatca aggggggagt cctcgaagat     240
   gcagagacgg agtatcgtcc cggtacgata accctccctc tatttttctc caagacagac     300
   tttgacctag agatgatcaa gcgggcggtg agtcaagtcg gaggagaggg aacgagaagg     360
   gcattgggcc tcctgtgcgc gttcgtcatt gcagagacgg tcccatcgga ggcaggtacg     420
   gtcgccgaac ttctggaagc cctgggcttt gtgctggagt ctttggacac tggggcacca     480
   ccagacgcta ccttcgcaga ccccaacaac aagcttgcag aaacgatcgt aaaggaaaat 540
   gtccttgagg ttgtgaccgg cctcctcttc acctgcgccc tactgacaaa gtatgatgtg 600
   gacaagatgg ccacatactg ccaaaacaag ctcgagcgtc ttgcaaccag ccaagggatt 660
   ggcgagttgg ttaacttcaa cgccaacagg ggagtcctgg ccaggatcgg gtcggtgctt 720
   agacccggac agaagctcac caaggctatc tatgggatca ttctcatcaa cctgtccgac 780
   ccagccatcg ctgccagagc caaggcactg tgcgccatga gactgagcgg gacaggaatg 840
   acaatggtgg gactgttcaa ccaagccgca aagaacctgg gcgcccttcc agccgacctt 900
   ctagaggatc tgtgcatgaa gtcagtggtg gagtccgcca gacgcattgt cagactgatg 960
   aggatcgtag cagaggcccc aggggtagca gcaaagtacg gtatcatgat gagcaggatg 1020
   ctcggggagg ggtacttcaa ggcctacggg atcaacgaga acgccaggat cacctgcatt 1080
   ctcatgaaca tcaacgatag gtatgacgat gggacctcga gaggactgac agggataaag 1140
   gtatccgacc ctttcaggaa gctggcgaga gagatcgctc gtctccttgt cctcaagtac 1200
   gatggcgatg gctcaaccgg agagggggcg tccgagctga tccgccgggc ggagatggca 1260
   tctcggggac cagacatggg tgaggaagag gaggaggacg aggaggacga ctactccagt 1320
   gagccaggag actccgattc attccgctga acacaaacag cccccccctc ctttttcctc 1380
   tcccgcccct cgacccatcc agatgtttac tccaccctga ctcttagata gaaaaaaatg 1440
   gcactatagt gcttttcaac tcaaaccaca acaatgtcag atggagaagg agaacagttc 1500
   ttcaacctag aaggcgaaga catactgagg ctagaatccc gcctgaaaaa cccacggaat 1560
   gacgggcaaa tcggcaagaa ccccaggcaa cggaaggagg accaggcgcc tcaagaggaa 1620
   ccaaagaaaa ccaccaggag acccgacaag aacaagggtc tatctcaact ggagcaactc 1680
   atcctaaagt acgttgagga ggagagctgt caggatgccc tgaaggactt cggaggtcta 1740
   attgccaaca tcagacaggc ccaccaggcc gaactgacat ctcacctaga aaaggttgct 1800
   acggaacacc gagccaatct tcaggctctt acaaagtccc agcaagagca cgagaaagtc 1860
   tcgaaggaga ttttgtctgc ggtaattgct attcggtcca acctcaacga gaaccacagt 1920
   cccaggccca agccactcga cccggatcag gtgaaggccg ctcgtgccct tggattcgga 1980
   attgggtatc gaacggccct taatgtcttc gaccgaataa agggagtcac cccagacaac 2040
   gcaggatccc aagaggtgaa gaacatggcc attcgggcgg cggaggagga tgaatacgag 2100
   ggaagtccca ctttcttcag gaaggtgata gacgccgtaa agaagcgcat gaagcaaggt 2160
   caatagacat cctcctccag gatcccggct accaagacag aaaaaaatgg cacgcaagtg 2220
   tatcgttcca aacgaaatcc aagagtcagt tcaaacgaga gcatgtctat tttcaagaga 2280
   gcaaagagaa cagttctgat ccctcctcct cacctcctca gcggagacga ggagagggtt 2340
   acaatactca gtgcagaggg ggagatcaag gtgactggaa gaagaccaac cacacttgag 2400
   gagaagatct attactctat gaatctggcg gctgccatcg tagggggaga tctacacccc 2460
   tcattcaaat ccatgaccta tctgttccag aaagagatgg agttcggaag cacccaagaa 2520
   aaggtcaact tcggctctcg gaaaccagca cctcagacca cctaccaggt aatgaaggcg 2580
   agggaaatct accttcagac ccagcctctc gagaagaaga tcccaatgca gacctactca 2640
   gtcagcacag agggggctac catcaccttc acaggacggt tcctgttttc atccagccat 2700
   gtaggttgcg acgacaacag gaccaaactg gcggggcttg acgggttcac aacctccaac 2760
   agctaccaga gggtcaaaga ctactatgcc caggagacag ccctggccct gaccttcgcg 2820
   gctcccgaaa agcgggggaa ggaaaaatag gagtcgacca tgccgtctct cactcaccca 2880
   tccatcggcc gacccaaccc tcctccatcc ccagagtccc tccactcctc actccgtcca 2940
   agacagaaaa aaatggcact tttgtgcttt gagacagaac gcaactcgca gagacccacc 3000
   aaaacaatgg acaccacgat caccactccg ctcattctca ttctgatcac ctgcggagca 3060
   aacagccaaa ccgtcaaacc cgacaccgca agcgaatcag accaacccac ctggtcaaac 3120
   ccgctcttca cctatcccga gggatgcact ctggacaagc tctccaaggt caatgcctct 3180
   cagctgagat gcccaaggat cttcgacgat gagaacaggg ggctaattgc ctatcccaca 3240
   tccatccggt ccctgtcagt cggaaacgac ctcggggata ttcacaccca agggaaccac 3300
   atccacaaag tcctgtaccg caccatctgc tcaacagggt tcttcggggg tcagacgata 3360
   gagaaggcgc ttgtagaaat gaaactctct acgaaagaag caggggcgta tgacaccaca 3420
   accgcagccg ctctgtactt cccagctccc cgatgccaat ggtacaccga caacgtacaa 3480
   aacgatctca tcttctacta cacaacccaa aagagtgttc tgagagatcc ctacaccaga 3540
   gactttctgg actcagattt tattggagga aaatgcacca aatcaccctg ccagactcat 3600
   tggtccaacg tcgtttggat gggtgatgca gggataccag cctgtgattc cagccaagag 3660
   ataaaaggtc acctctttgt tgataaaatc tccaatcgag tcgtgaaggc aacgagctac 3720
   ggacaccacc cctggggact gcatcaggcc tgtatgattg aattctgtgg gaaacagtgg 3780
   atacggacag atctcggtga cctaatatct gtcgaataca attctggagc agaaatcctc 3840
   tcgttcccga agtgtgagga caagactgtg gggatgaggg gaaacttgga tgactttgcc 3900
   tatctagacg atcttgtgaa ggcctctgag agcagagagg aatgtcttga ggcgcacgcc 3960
   gagataatat caacaaacag tgtgactcca tacctcctat ccaagttccg atctccacat 4020
   cccggaataa atgacgtcta cgctatgcac aaaggctcca tctatcacgg gatgtgcatg 4080
   acggtcgctg tggacgaggt atccaaggac aggacaactt acagggccca tcgcgctacc 4140
   agtttcacga aatgggaacg accctttggg gatgagtggg agggctttca cggattgcac 4200
   ggaaacaaca ccaccattat tccagacctg gagaaatacg tcgcccagta caagacgagc 4260
   atgatggaac cgatgagcat caaatcagta ccccatccaa gcatcctggc cctctacaat 4320
   gagacagacg tatcagggat ctccatcagg aaattggact cattcgacct tcaatcactc 4380
   cactggagtt tctggcccac aatctccgca ctgggtggga ttccctttgc tctcctcctt 4440
   gctgttgccg cgtgctgctg ctggtcaggg agacctccca ctccctccgc gtcgcagagt 4500
   atccccatgt atcacctggc aaaccggtcc taaaggacct caatcctcac ttcctcccca 4560
   ccagacagaa aaaaacggca catctgtgct gcaaaaagag acaatggacc accgcgacat 4620
   aaacacgaac atggaggcac tcagagaagt tctgcgatac aagaacaagg tggccggaca 4680
   cggcttcctc tttgacgacg gagacctggt atggcgtgaa gaggacgacg caacgtggag 4740
   gcggctttgc gatgtcgtca acgcactgat ctcctccaag aggatgcagc gagtattgta 4800
   catggacctc agcatcacca agggcgaggg gcatctactt tttgtggatc tccaggggac 4860
   caagaaccgc ctgcacaaag aaccccgatt caggagacat ctgatcctga ttgaagactt 4920
   tcttgcttat cccagataga aaaaaatggc acttttgtgc aaaaaaactc aagcgcgact 4980
   cacagagaac acaaccagca acgacagcaa cccatcacag aaaaatggac ttcttcgatc 5040
   ttgacataga aataaaacaa gagagacttc cagcggaatg ctccctcaac tcacccctaa 5100
   attactcact gtctgcacaa ctgacagatc gcatgacccc tagaactgag aacgtcagga 5160
   ggcaaagaga aaggctcagg tcccacatgc gagaacactt ccgggtcaag gatctatcca 5220
   ccctcgacaa tgactccacc aggctccatg cacgactcac agaggatctc atcaccattc 5280
   aaagccccga gatcgattcc tcagtccttg aaaattggac cccacttaag tcctactatg 5340
   catctctaga ctacacactc ccggaaaaga cagccttcaa gtgggaacac gcagccccct 5400
   actggaatct atttgcacaa ctgagggcaa ttctcctcca gagccaaaag atcaagaaac 5460
   aagagacggg cacgagggaa ctctactctt gtggccctct tcagatagag ttcgtggagg 5520
   gggtagtctt gtacttcaca gatcgaggat cgaaagagga attcaccaag tcaggagagc 5580
   tcccaagtgt aacaccgtat gcggacttcc ttgcctggat caagatcatc tcccaacggg 5640
   cccaggccgt gttgatggca gtcatcctca gagtgaccga caagggacta tctccactac 5700
   cagaatcact cctggccatc tatcaaaacg tggacgacat cctgaaacga gcgggccagc 5760
   ctgcgataga cctcctgaaa ctctgggagc cattggtgat tacaaaattg ggagaacttc 5820
   tcggagacag attcggacta gaagaagatt tccgaaacac aatacgaggt gaggcaacaa 5880
   aattggcaaa gaacctattc atttcacgcg ggctcaatcg cctcatggac atcctggatc 5940
   agcaaactga cgctcaaccg ctctttcaat ttttcggact attcaaacat tttgcgtacc 6000
   cacgtgtctt ctcaagagac accatccagg caatccagga ggtaagtgac aggcctagct 6060
   caatctcagc tgtcgacttt ctccatgatc agtgcgagat tcgcaaggaa ttctacgtca 6120
   gatacataaa cgcataccac agggcacctg gccttgacct ctccgccctg agtccctcct 6180
   catttctaag agacagtctc gagcgtggaa aaatcccaaa cgaaagaagc cctctctatt 6240
   caaacaaaga gtggtacttc gtcaaattca caaaaagcat cgagtggcca gttagcgaca 6300
   ctctgtcgac attcctatcg gacaaagcga tcactcgtga ccgacccgcc tggattgagg 6360
   acgggcactc gggtagagac atgtccgaga agcggctgct actcaaattt atcaaggaga 6420
   acttctcgag tgttgcagac attgtcggtg cggcggaagc gatttacaac aaccaagagg 6480
   accgactgat cgcactgaaa gtgaaagaga tggaactaaa gatcaagggc aggggattcg 6540
   ggctgatgac cttcatgcct cgtctcctcc aagtcctccg tgagagcatc gccaaaaaaa 6600
   cacaaaaact attccctgag atcaccatga cctcctcaga tctggacatg aaaaaaagaa 6660
   agttcatgct ctcaaaaaag agcgacgaca ggcgaggctt catacacgtg aataaaagtc 6720
   tggacatcaa caaattttgc accagccaga gacaattcaa ctccaatgcg gttttctcaa 6780
   gtctagatga actcatgggg acctttcctt tgttttcccg agttcacgaa atttttgaaa 6840
   aaacctggat cgtagacggg tcatcctcgg atccccccaa cttggcacac ttcactagaa 6900
   ttctcgacga atgcacggca cttggtctag acacccctca catctgggcg gacggggtct 6960
   tctcggggtt gaagggaggg atcgaagggc tctgtcaata cgtttggact atctgtcttt 7020
   tacttagagt agaacgcgtc atgcaaaaaa cagcgctcac ccactacatt ctggcacagg 7080
   gcgacaatgt gattatcaca attatcgtac cagtagaaat acatcgggat ggcaccattc 7140
   cagaacaaga gtcccgtcgt atcttggcct taagtcgaga catcgatctc tcactggaga 7200
   gtgaactaga aaaaagtggt ctcaccctaa agatcgaaga gactctaaca agtgagaaca 7260
   tctccatcta cgggaaggat cttcactgtc cgcaacacct taccctggca atcaagaagg 7320
   ccgcctccgc cgcgatcata tcaagtgagc agtaccaaga tgtcccaaca tttctatctg 7380
   gtctcggaac gagtctggag gccctctcag agtgtgtcaa caacaaagtc ggggtgcatc 7440
   tctttggagt gatcatgggc gttgcgggat ggagggacct ggcgactcat cagacttggc 7500
   gaggatggag atacccctat cacaagaagg cgataactgg gcggattcgg gcatccgaga 7560
   tgaagctcag caaaggcgaa cccaccgagc tgtccatctc tgtcctctcc aagcgacgta 7620
   gagagaccga aacactcatt gaactactat ccaacagcct gctcgggtca gccctaggaa 7680
   tgcttgcgtt ccctactcct ctagacctgg aaaaacgggg tgtgggagac tacatcaccc 7740
   atcggctcac catagcacgc aaggcccttc tgagtggaca ccttgatccc cgtatcggga 7800
   gaaaagttga atcagcatgc aacatccccc tctcgtccag aaccgaccta agcaaactgt 7860
   ttgactctcc attttcattg aacatcgcaa ccgaagagga cgcgacggca gtcatcaaac 7920
   gacaggcaac aaaaatcctc cgactgcaag agataaaaaa cgagaagctg aaggcccaga 7980
   ttgacaacat ggacaagggc atagcgaccc ttgatgcagc acttgcagga gccaccaaca 8040
   tcaaccccag actgaattac atgatcagga gcatcacgga cgaaaaggag tccgagatgt 8100
   ttgtcactaa attcgcatca gcaagaacca tgaggaccct ggcaatgaat cactcctcag 8160
   aacttcccat tgtcatcctc ctggagatga aaagccaaca aaaagagaca tacacaatct 8220
   ggagaaccaa gagaccacct gtgaccatgt ggaaatgctc cacagtgttg gcgaaggagc 8280
   tccgagacac ctcctggggc aaaaacataa tcggaggcac atccccttct ccaatcgagg 8340
   caatggagac aatccaaatt gaccccaccg aatgggaaga ccgacggtcc caggacgcca 8400
   tgtccatcaa ctactatctg tcacgggctg gcatggacga acagacagca aagctcactc 8460
   gagggttcct agtcccttac tacgggaccc aaacaaaacc ccttgtggcc aaggcctacc 8520
   tagaactcaa gggcaacccc cgaaccaaca aggctctcct tctcctgagt gttcgagagt 8580
   ctctcgtaaa aacaggcagc aatctcgaca agttgatcat aaagctctgc tcccatgcac 8640
   tagacatcga tgtagcctca ctccccgccc ttcgagcaca ggaagaggcg gcagctgggg 8700
   agggtctccg aggaggaatc aaagaatcca tgtctccagt aggtcccgat aacttctaca 8760
   caaacatcac gcacaaggtc ttcaacagga agtgggcaac accataccac gtcaacattg 8820
   cagacttcat catccaaggc ctgatagaga ccagaaggca cctccttgta aatgagagga 8880
   tgaatgggct actcccggtc agctcagtca aatgcacctc ctgcttcagg aagaaagaga 8940
   gggagttctt tgatattccc gaggaattca cctggaaaaa tgagtcaaaa acctcagatc 9000
   ccgcctacac ctacttcacc acctggtgtg acctccccag ggtctccaac ctgcccgaga 9060
   tggatcaacg gtcagccact cgtctcttgg gaagagggct cgcattaaac agatcctcct 9120
   ccggtgaaat catcaccaaa ttctactcaa tgcccatgga atcacaacgg ctccttcacc 9180
   ccgtggaact gctcctggga tatggtgaag ggttatattt tggatatttg aggagtcagc 9240
   acataaatca cggagccctc tttcacattg gcgacgagtc actggcaaag aaactcagac 9300
   gttatgtcct agacacaaag acacaacatg ccaagcaaat tggctacctc ttccaggatg 9360
   aggactcatt acatgagctc ctgggccaag gactttgtcc atatataccc aggtcaatcc 9420
   ctcttaccat cactgaacta acaaacgcct gcgcgatcac aaccatcagg gcaacggagg 9480
   taatcctaag cacaaaagcc aggatccacc atatgcccgt ccaggcaatc gacgaatcag 9540
   atgtcgacac cagcagacta gctgcgaaca ccatgcagac catactcgga gaccccagac 9600
   ccatgaacct cgtgcatctt gactgcgatc tcacccacaa catggtcgca tgggagtcag 9660
   aagtggaact cgacatcttg aaatcagaaa acttccacat tgacggactc ctggtagaac 9720
   tcactgcccg cgagctcccc attggagaca ccccctggaa gcaaagagac tggacctgtt 9780
   ccaacgatcc caagatcatc gccaagggca tcaagacaaa atccctgttc attcatcagg 9840
   gcgtgactgg ggcaatcaac ttgattcccg atcttctggt cgtgatcggg ggaggtctcg 9900
   gagggtgcgc cgttccctac cttcaggaat ggccagacac accaatcatc tttgcaaccc   9960
   tattcgacga acgggagagg atctccgagg atggagactt gattgttccc ccagagctcc  10020
   tggtgagggg catggcacct aggatgatcg agagggagat cttagaagca gagctgtgtg  10080
   acatcaccaa tgaggggaat cgtcgcctgc taattcgact ggtcacgaaa aacaagggag  10140
   gaggcaaggt ggtgctcata gacgagatcg agaatcgagg ggccccagaa tcccttctac  10200
   agagctcgct ccaagaccta ttcgagaaac tggacaaagt ctgcaaactc acctctgtgc  10260
   acacagtcag agagtcaaca gtggaacaat tctcccagag ggtaaattct atcaaacgca  10320
   gcaggaaggc agtaacactg cattggaacc gctacaacag aagagaccaa tttgaggccc  10380
   tggtcatcgt gaagggcgaa gaggccaagt ccgactaccg gatctccacg atcacctcgg  10440
   caaaggcatt tcgcaagatc gacgaacagc tcgagataga cgggagactc tcgtcgacac  10500
   agtggtccct ccccgcgctt ccttctcgag aaaaaaacat tctttttggg tacgtgtcat  10560
   ccgtcttcct taagatgaac cttgcactct cagccaacga catggaccga gagcgactga  10620
   tcgagaccat tgagggaacc gccccagggt tgatctcctg gaaggaaaaa ctcgaacaca  10680
   gggaccatgc acatcgctcc gacattgaag agaaaggaat aactcaggac aagatcttca  10740
   acctcatctg cctctcctgg gtcctcaagg gtctgagata cggagtatgg gacaccgacg  10800
   ctcaatccat tgtagctcaa acggtgtaca tcactcgagg gccaaagctc tgtccattgg  10860
   gggagaagcc caagcgcatc ttcgcatcat tcaaactaca aagcggaaag cgagttgaag  10920
   atgccaaggg attcctgagc gcactgcttc accttgaggg cttctttcca ctaggcgaac  10980
   aataggtaca ctccagcagc acccagtgcc agggaccaaa ctcccagata caaaaaaatg  11040
   gctcccgact cccatggtaa gggaaccagt cattgttttc tgttattaaa aacctttttt  11100
   aactgccttt tgagaaccct tctgttactt tttttat                            11137
<210> 21
   <211> 391
   <212> PRT
   <213> Infectious hematopoietic necrosis virus
<400> 21
   Met Thr Ser Ala Leu Arg Glu Thr Phe Thr Gly Leu Arg Asp Ile Lys
   1               5                   10                  15
   Gly Gly Val Leu Glu Asp Ala Glu Thr Glu Tyr Arg Pro Gly Thr Ile
               20                  25                  30
   Thr Leu Pro Leu Phe Phe Ser Lys Thr Asp Phe Asp Leu Glu Met Ile
           35                  40                  45
   Lys Arg Ala Val Ser Gln Val Gly Gly Glu Gly Thr Arg Arg Ala Leu
   50 55 60
   Gly Leu Leu Cys Ala Phe Val Ile Ala Glu Thr Val Pro Ser Glu Ala
   65 70 75 80
   Gly Thr Val Ala Glu Leu Leu Glu Ala Leu Gly Phe Val Leu Glu Ser
   85 90 95
   Leu Asp Thr Gly Ala Pro Pro Asp Ala Thr Phe Ala Asp Pro Asn Asn
   100 105 110
   Lys Leu Ala Glu Thr Ile Val Lys Glu Asn Val Leu Glu Val Val Thr
   115 120 125
   Gly Leu Leu Phe Thr Cys Ala Leu Leu Thr Lys Tyr Asp Val Asp Lys
   130 135 140
   Met Ala Thr Tyr Cys Gln Asn Lys Leu Glu Arg Leu Ala Thr Ser Gln
   145 150 155 160
   Gly Ile Gly Glu Leu Val Asn Phe Asn Ala Asn Arg Gly Val Leu Ala
   165 170 175
   Arg Ile Gly Ser Val Leu Arg Pro Gly Gln Lys Leu Thr Lys Ala Ile
   180 185 190
   Tyr Gly Ile Ile Leu Ile Asn Leu Ser Asp Pro Ala Ile Ala Ala Arg
   195 200 205
   Ala Lys Ala Leu Cys Ala Met Arg Leu Ser Gly Thr Gly Met Thr Met
   210 215 220
   Val Gly Leu Phe Asn Gln Ala Ala Lys Asn Leu Gly Ala Leu Pro Ala
   225 230 235 240
   Asp Leu Leu Glu Asp Leu Cys Met Lys Ser Val Val Glu Ser Ala Arg
   245 250 255
   Arg Ile Val Arg Leu Met Arg Ile Val Ala Glu Ala Pro Gly Val Ala
   260 265 270
   Ala Lys Tyr Gly Ile Met Met Ser Arg Met Leu Gly Glu Gly Tyr Phe
   275 280 285
   Lys Ala Tyr Gly Ile Asn Glu Asn Ala Arg Ile Thr Cys Ile Leu Met
   290 295 300
   Asn Ile Asn Asp Arg Tyr Asp Asp Gly Thr Ser Arg Gly Leu Thr Gly
   305                 310                 315                 320
   Ile Lys Val Ser Asp Pro Phe Arg Lys Leu Ala Arg Glu Ile Ala Arg
                   325                 330                 335
   Leu Leu Val Leu Lys Tyr Asp Gly Asp Gly Ser Thr Gly Glu Gly Ala
               340                 345                 350
   Ser Glu Leu Ile Arg Arg Ala Glu Met Ala Ser Arg Gly Pro Asp Met
           355                 360                 365
   Gly Glu Glu Glu Glu Glu Asp Glu Glu Asp Asp Tyr Ser Ser Glu Pro
       370                 375                 380
   Gly Asp Ser Asp Ser Phe Arg
   385                 390
<210> 22
   <211> 111
   <212> PRT
   <213> Infectious hematopoietic necrosis virus
<400> 22
   Met Asp His Arg Asp Ile Asn Thr Asn Met Glu Ala Leu Arg Glu Val
   1               5                   10                  15
   Leu Arg Tyr Lys Asn Lys Val Ala Gly His Gly Phe Leu Phe Asp Asp
               20                  25                  30
   Gly Asp Leu Val Trp Arg Glu Glu Asp Asp Ala Thr Trp Arg Arg Leu
           35                  40                  45
   Cys Asp Val Val Asn Ala Leu Ile Ser Ser Lys Arg Met Gln Arg Val
       50                  55                  60
   Leu Tyr Met Asp Leu Ser Ile Thr Lys Gly Glu Gly His Leu Leu Phe
   65                  70                  75                  80
   Val Asp Leu Gln Gly Thr Lys Asn Arg Leu His Lys Glu Pro Arg Phe
                   85                  90                  95
   Arg Arg His Leu Ile Leu Ile Glu Asp Phe Leu Ala Tyr Pro Arg
               100                 105                 110
<210> 23
   <211> 230
   <212> PRT
   <213> Infectious hematopoietic necrosis virus
<400> 23
   Met Ser Asp Gly Glu Gly Glu Gln Phe Phe Asn Leu Glu Gly Glu Asp
   1               5                   10                  15
   Ile Leu Arg Leu Glu Ser Arg Leu Lys Asn Pro Arg Asn Asp Gly Gln
               20                  25                  30
   Ile Gly Lys Asn Pro Arg Gln Arg Lys Glu Asp Gln Ala Pro Gln Glu
           35                  40                  45
   Glu Pro Lys Lys Thr Thr Arg Arg Pro Asp Lys Asn Lys Gly Leu Ser
       50                  55                  60
   Gln Leu Glu Gln Leu Ile Leu Lys Tyr Val Glu Glu Glu Ser Cys Gln
   65                  70                  75                  80
   Asp Ala Leu Lys Asp Phe Gly Gly Leu Ile Ala Asn Ile Arg Gln Ala
                   85                  90                  95
   His Gln Ala Glu Leu Thr Ser His Leu Glu Lys Val Ala Thr Glu His
               100                 105                 110
   Arg Ala Asn Leu Gln Ala Leu Thr Lys Ser Gln Gln Glu His Glu Lys
           115                 120                 125
   Val Ser Lys Glu Ile Leu Ser Ala Val Ile Ala Ile Arg Ser Asn Leu
       130                 135                 140
   Asn Glu Asn His Ser Pro Arg Pro Lys Pro Leu Asp Pro Asp Gln Val
   145                 150                 155                 160
   Lys Ala Ala Arg Ala Leu Gly Phe Gly Ile Gly Tyr Arg Thr Ala Leu
                   165                 170                 175
   Asn Val Phe Asp Arg Ile Lys Gly Val Thr Pro Asp Asn Ala Gly Ser
               180                 185                 190
   Gln Glu Val Lys Asn Met Ala Ile Arg Ala Ala Glu Glu Asp Glu Tyr
           195                 200                 205
   Glu Gly Ser Pro Thr Phe Phe Arg Lys Val Ile Asp Ala Val Lys Lys
       210                 215                 220
   Arg Met Lys Gln Gly Gln
   225                 230
<210> 24
   <211> 195
   <212> PRT
   <213> Infectious hematopoietic necrosis virus
<400> 24
   Met Ser Ile Phe Lys Arg Ala Lys Arg Thr Val Leu Ile Pro Pro Pro
   1               5                   10                  15
   His Leu Leu Ser Gly Asp Glu Glu Arg Val Thr Ile Leu Ser Ala Glu
               20                  25                  30
   Gly Glu Ile Lys Val Thr Gly Arg Arg Pro Thr Thr Leu Glu Glu Lys
           35                  40                  45
   Ile Tyr Tyr Ser Met Asn Leu Ala Ala Ala Ile Val Gly Gly Asp Leu
       50                  55                  60
   His Pro Ser Phe Lys Ser Met Thr Tyr Leu Phe Gln Lys Glu Met Glu
   65                  70                  75                  80
   Phe Gly Ser Thr Gln Glu Lys Val Asn Phe Gly Ser Arg Lys Pro Ala
                   85                  90                  95
   Pro Gln Thr Thr Tyr Gln Val Met Lys Ala Arg Glu Ile Tyr Leu Gln
               100                 105                 110
   Thr Gln Pro Leu Glu Lys Lys Ile Pro Met Gln Thr Tyr Ser Val Ser
           115                 120                 125
   Thr Glu Gly Ala Thr Ile Thr Phe Thr Gly Arg Phe Leu Phe Ser Ser
       130                 135                 140
   Ser His Val Gly Cys Asp Asp Asn Arg Thr Lys Leu Ala Gly Leu Asp
   145                 150                 155                 160
   Gly Phe Thr Thr Ser Asn Ser Tyr Gln Arg Val Lys Asp Tyr Tyr Ala
                   165                 170                 175
   Gln Glu Thr Ala Leu Ala Leu Thr Phe Ala Ala Pro Glu Lys Arg Gly
               180                 185                 190
   Lys Glu Lys
           195
<210> 25
   <211> 508
   <212> PRT
   <213> Infectious hematopoietic necrosis virus
<400> 25
   Met Asp Thr Thr Ile Thr Thr Pro Leu Ile Leu Ile Leu Ile Thr Cys
   1               5                   10                  15
   Gly Ala Asn Ser Gln Thr Val Lys Pro Asp Thr Ala Ser Glu Ser Asp
               20                  25                  30
   Gln Pro Thr Trp Ser Asn Pro Leu Phe Thr Tyr Pro Glu Gly Cys Thr
           35                  40                  45
   Leu Asp Lys Leu Ser Lys Val Asn Ala Ser Gln Leu Arg Cys Pro Arg
       50                  55                  60
   Ile Phe Asp Asp Glu Asn Arg Gly Leu Ile Ala Tyr Pro Thr Ser Ile
   65                  70                  75                  80
   Arg Ser Leu Ser Val Gly Asn Asp Leu Gly Asp Ile His Thr Gln Gly
                   85                  90                  95
   Asn His Ile His Lys Val Leu Tyr Arg Thr Ile Cys Ser Thr Gly Phe
               100                 105                 110
   Phe Gly Gly Gln Thr Ile Glu Lys Ala Leu Val Glu Met Lys Leu Ser
           115                 120                 125
   Thr Lys Glu Ala Gly Ala Tyr Asp Thr Thr Thr Ala Ala Ala Leu Tyr
       130                 135                 140
   Phe Pro Ala Pro Arg Cys Gln Trp Tyr Thr Asp Asn Val Gln Asn Asp
   145                 150                 155                 160
   Leu Ile Phe Tyr Tyr Thr Thr Gln Lys Ser Val Leu Arg Asp Pro Tyr
                   165                 170                 175
   Thr Arg Asp Phe Leu Asp Ser Asp Phe Ile Gly Gly Lys Cys Thr Lys
               180                 185                 190
   Ser Pro Cys Gln Thr His Trp Ser Asn Val Val Trp Met Gly Asp Ala
           195                 200                 205
   Gly Ile Pro Ala Cys Asp Ser Ser Gln Glu Ile Lys Gly His Leu Phe
       210                 215                 220
   Val Asp Lys Ile Ser Asn Arg Val Val Lys Ala Thr Ser Tyr Gly His
   225                 230                 235                 240
   His Pro Trp Gly Leu His Gln Ala Cys Met Ile Glu Phe Cys Gly Lys
                   245                 250                 255
   Gln Trp Ile Arg Thr Asp Leu Gly Asp Leu Ile Ser Val Glu Tyr Asn
               260                 265                 270
   Ser Gly Ala Glu Ile Leu Ser Phe Pro Lys Cys Glu Asp Lys Thr Val
           275                 280                 285
   Gly Met Arg Gly Asn Leu Asp Asp Phe Ala Tyr Leu Asp Asp Leu Val
       290                 295                 300
   Lys Ala Ser Glu Ser Arg Glu Glu Cys Leu Glu Ala His Ala Glu Ile
   305                 310                 315                 320
   Ile Ser Thr Asn Ser Val Thr Pro Tyr Leu Leu Ser Lys Phe Arg Ser
                   325                 330                 335
   Pro His Pro Gly Ile Asn Asp Val Tyr Ala Met His Lys Gly Ser Ile
               340                 345                 350
   Tyr His Gly Met Cys Met Thr Val Ala Val Asp Glu Val Ser Lys Asp
           355                 360                 365
   Arg Thr Thr Tyr Arg Ala His Arg Ala Thr Ser Phe Thr Lys Trp Glu
       370                 375                 380
   Arg Pro Phe Gly Asp Glu Trp Glu Gly Phe His Gly Leu His Gly Asn
   385                 390                 395                 400
   Asn Thr Thr Ile Ile Pro Asp Leu Glu Lys Tyr Val Ala Gln Tyr Lys
                   405                 410                 415
   Thr Ser Met Met Glu Pro Met Ser Ile Lys Ser Val Pro His Pro Ser
               420                 425                 430
   Ile Leu Ala Leu Tyr Asn Glu Thr Asp Val Ser Gly Ile Ser Ile Arg
           435                 440                 445
   Lys Leu Asp Ser Phe Asp Leu Gln Ser Leu His Trp Ser Phe Trp Pro
       450                 455                 460
   Thr Ile Ser Ala Leu Gly Gly Ile Pro Phe Ala Leu Leu Leu Ala Val
   465                 470                 475                 480
   Ala Ala Cys Cys Cys Trp Ser Gly Arg Pro Pro Thr Pro Ser Ala Ser
                   485                 490                 495
   Gln Ser Ile Pro Met Tyr His Leu Ala Asn Arg Ser
   500 505
<210> 26
   <211> 1986
   <212> PRT
   <213> Infectious hematopoietic necrosis virus
<400> 26
   Met Asp Phe Phe Asp Leu Asp Ile Glu Ile Lys Gln Glu Arg Leu Pro
   1 5 10 15
   Ala Glu Cys Ser Leu Asn Ser Pro Leu Asn Tyr Ser Leu Ser Ala Gln
   20 25 30
   Leu Thr Asp Arg Met Thr Pro Arg Thr Glu Asn Val Arg Arg Gln Arg
   35 40 45
   Glu Arg Leu Arg Ser His Met Arg Glu His Phe Arg Val Lys Asp Leu
   50 55 60
   Ser Thr Leu Asp Asn Asp Ser Thr Arg Leu His Ala Arg Leu Thr Glu
   65 70 75 80
   Asp Leu Ile Thr Ile Gln Ser Pro Glu Ile Asp Ser Ser Val Leu Glu
   85 90 95
   Asn Trp Thr Pro Leu Lys Ser Tyr Tyr Ala Ser Leu Asp Tyr Thr Leu
   100 105 110
   Pro Glu Lys Thr Ala Phe Lys Trp Glu His Ala Ala Pro Tyr Trp Asn
   115 120 125
   Leu Phe Ala Gln Leu Arg Ala Ile Leu Leu Gln Ser Gln Lys Ile Lys
   130 135 140
   Lys Gln Glu Thr Gly Thr Arg Glu Leu Tyr Ser Cys Gly Pro Leu Gln
   145 150 155 160
   Ile Glu Phe Val Glu Gly Val Val Leu Tyr Phe Thr Asp Arg Gly Ser
   165 170 175
   Lys Glu Glu Phe Thr Lys Ser Gly Glu Leu Pro Ser Val Thr Pro Tyr
   180 185 190
   Ala Asp Phe Leu Ala Trp Ile Lys Ile Ile Ser Gln Arg Ala Gln Ala
   195 200 205
   Val Leu Met Ala Val Ile Leu Arg Val Thr Asp Lys Gly Leu Ser Pro
       210                 215                 220
   Leu Pro Glu Ser Leu Leu Ala Ile Tyr Gln Asn Val Asp Asp Ile Leu
   225                 230                 235                 240
   Lys Arg Ala Gly Gln Pro Ala Ile Asp Leu Leu Lys Leu Trp Glu Pro
                   245                 250                 255
   Leu Val Ile Thr Lys Leu Gly Glu Leu Leu Gly Asp Arg Phe Gly Leu
               260                 265                 270
   Glu Glu Asp Phe Arg Asn Thr Ile Arg Gly Glu Ala Thr Lys Leu Ala
           275                 280                 285
   Lys Asn Leu Phe Ile Ser Arg Gly Leu Asn Arg Leu Met Asp Ile Leu
       290                 295                 300
   Asp Gln Gln Thr Asp Ala Gln Pro Leu Phe Gln Phe Phe Gly Leu Phe
   305                 310                 315                 320
   Lys His Phe Ala Tyr Pro Arg Val Phe Ser Arg Asp Thr Ile Gln Ala
                   325                 330                 335
   Ile Gln Glu Val Ser Asp Arg Pro Ser Ser Ile Ser Ala Val Asp Phe
               340                 345                 350
   Leu His Asp Gln Cys Glu Ile Arg Lys Glu Phe Tyr Val Arg Tyr Ile
           355                 360                 365
   Asn Ala Tyr His Arg Ala Pro Gly Leu Asp Leu Ser Ala Leu Ser Pro
       370                 375                 380
   Ser Ser Phe Leu Arg Asp Ser Leu Glu Arg Gly Lys Ile Pro Asn Glu
   385                 390                 395                 400
   Arg Ser Pro Leu Tyr Ser Asn Lys Glu Trp Tyr Phe Val Lys Phe Thr
                   405                 410                 415
   Lys Ser Ile Glu Trp Pro Val Ser Asp Thr Leu Ser Thr Phe Leu Ser
               420                 425                 430
   Asp Lys Ala Ile Thr Arg Asp Arg Pro Ala Trp Ile Glu Asp Gly His
           435                 440                 445
   Ser Gly Arg Asp Met Ser Glu Lys Arg Leu Leu Leu Lys Phe Ile Lys
       450                 455                 460
   Glu Asn Phe Ser Ser Val Ala Asp Ile Val Gly Ala Ala Glu Ala Ile
   465                 470                 475                 480
   Tyr Asn Asn Gln Glu Asp Arg Leu Ile Ala Leu Lys Val Lys Glu Met
                   485                 490                 495
   Glu Leu Lys Ile Lys Gly Arg Gly Phe Gly Leu Met Thr Phe Met Pro
               500                 505                 510
   Arg Leu Leu Gln Val Leu Arg Glu Ser Ile Ala Lys Lys Thr Gln Lys
           515                 520                 525
   Leu Phe Pro Glu Ile Thr Met Thr Ser Ser Asp Leu Asp Met Lys Lys
       530                 535                 540
   Arg Lys Phe Met Leu Ser Lys Lys Ser Asp Asp Arg Arg Gly Phe Ile
   545                 550                 555                 560
   His Val Asn Lys Ser Leu Asp Ile Asn Lys Phe Cys Thr Ser Gln Arg
                   565                 570                 575
   Gln Phe Asn Ser Asn Ala Val Phe Ser Ser Leu Asp Glu Leu Met Gly
               580                 585                 590
   Thr Phe Pro Leu Phe Ser Arg Val His Glu Ile Phe Glu Lys Thr Trp
           595                 600                 605
   Ile Val Asp Gly Ser Ser Ser Asp Pro Pro Asn Leu Ala His Phe Thr
       610                 615                 620
   Arg Ile Leu Asp Glu Cys Thr Ala Leu Gly Leu Asp Thr Pro His Ile
   625                 630                 635                 640
   Trp Ala Asp Gly Val Phe Ser Gly Leu Lys Gly Gly Ile Glu Gly Leu
                   645                 650                 655
   Cys Gln Tyr Val Trp Thr Ile Cys Leu Leu Leu Arg Val Glu Arg Val
               660                 665                 670
   Met Gln Lys Thr Ala Leu Thr His Tyr Ile Leu Ala Gln Gly Asp Asn
           675                 680                 685
   Val Ile Ile Thr Ile Ile Val Pro Val Glu Ile His Arg Asp Gly Thr
       690                 695                 700
   Ile Pro Glu Gln Glu Ser Arg Arg Ile Leu Ala Leu Ser Arg Asp Ile
   705 710 715 720
   Asp Leu Ser Leu Glu Ser Glu Leu Glu Lys Ser Gly Leu Thr Leu Lys
   725 730 735
   Ile Glu Glu Thr Leu Thr Ser Glu Asn Ile Ser Ile Tyr Gly Lys Asp
   740 745 750
   Leu His Cys Pro Gln His Leu Thr Leu Ala Ile Lys Lys Ala Ala Ser
   755 760 765
   Ala Ala Ile Ile Ser Ser Glu Gln Tyr Gln Asp Val Pro Thr Phe Leu
   770 775 780
   Ser Gly Leu Gly Thr Ser Leu Glu Ala Leu Ser Glu Cys Val Asn Asn
   785 790 795 800
   Lys Val Gly Val His Leu Phe Gly Val Ile Met Gly Val Ala Gly Trp
   805 810 815
   Arg Asp Leu Ala Thr His Gln Thr Trp Arg Gly Trp Arg Tyr Pro Tyr
   820 825 830
   His Lys Lys Ala Ile Thr Gly Arg Ile Arg Ala Ser Glu Met Lys Leu
   835 840 845
   Ser Lys Gly Glu Pro Thr Glu Leu Ser Ile Ser Val Leu Ser Lys Arg
   850 855 860
   Arg Arg Glu Thr Glu Thr Leu Ile Glu Leu Leu Ser Asn Ser Leu Leu
   865 870 875 880
   Gly Ser Ala Leu Gly Met Leu Ala Phe Pro Thr Pro Leu Asp Leu Glu
   885 890 895
   Lys Arg Gly Val Gly Asp Tyr Ile Thr His Arg Leu Thr Ile Ala Arg
   900 905 910
   Lys Ala Leu Leu Ser Gly His Leu Asp Pro Arg Ile Gly Arg Lys Val
   915 920 925
   Glu Ser Ala Cys Asn Ile Pro Leu Ser Ser Arg Thr Asp Leu Ser Lys
   930 935 940
   Leu Phe Asp Ser Pro Phe Ser Leu Asn Ile Ala Thr Glu Glu Asp Ala
   945 950 955 960
   Thr Ala Val Ile Lys Arg Gln Ala Thr Lys Ile Leu Arg Leu Gln Glu
                   965                 970                 975
   Ile Lys Asn Glu Lys Leu Lys Ala Gln Ile Asp Asn Met Asp Lys Gly
               980                 985                 990
   Ile Ala Thr Leu Asp Ala Ala Leu  Ala Gly Ala Thr Asn  Ile Asn Pro
           995                 1000                1005
   Arg Leu  Asn Tyr Met Ile Arg  Ser Ile Thr Asp Glu  Lys Glu Ser
       1010                1015                1020
   Glu Met  Phe Val Thr Lys Phe  Ala Ser Ala Arg Thr  Met Arg Thr
       1025                1030                1035
   Leu Ala  Met Asn His Ser Ser  Glu Leu Pro Ile Val  Ile Leu Leu
       1040                1045                1050
   Glu Met  Lys Ser Gln Gln Lys  Glu Thr Tyr Thr Ile  Trp Arg Thr
       1055                1060                1065
   Lys Arg  Pro Pro Val Thr Met  Trp Lys Cys Ser Thr  Val Leu Ala
       1070                1075                1080
   Lys Glu  Leu Arg Asp Thr Ser  Trp Gly Lys Asn Ile  Ile Gly Gly
       1085                1090                1095
   Thr Ser  Pro Ser Pro Ile Glu  Ala Met Glu Thr Ile  Gln Ile Asp
       1100                1105                1110
   Pro Thr  Glu Trp Glu Asp Arg  Arg Ser Gln Asp Ala  Met Ser Ile
       1115                1120                1125
   Asn Tyr  Tyr Leu Ser Arg Ala  Gly Met Asp Glu Gln  Thr Ala Lys
       1130                1135                1140
   Leu Thr  Arg Gly Phe Leu Val  Pro Tyr Tyr Gly Thr  Gln Thr Lys
       1145                1150                1155
   Pro Leu  Val Ala Lys Ala Tyr  Leu Glu Leu Lys Gly  Asn Pro Arg
       1160                1165                1170
   Thr Asn  Lys Ala Leu Leu Leu  Leu Ser Val Arg Glu  Ser Leu Val
       1175                1180                1185
   Lys Thr  Gly Ser Asn Leu Asp  Lys Leu Ile Ile Lys  Leu Cys Ser
       1190                1195                1200
   His Ala  Leu Asp Ile Asp Val  Ala Ser Leu Pro Ala  Leu Arg Ala
       1205                 1210                 1215
   Gln Glu  Glu Ala Ala Ala Gly  Glu Gly Leu Arg Gly  Gly Ile Lys
       1220                 1225                 1230
   Glu Ser  Met Ser Pro Val Gly  Pro Asp Asn Phe Tyr  Thr Asn Ile
       1235                 1240                 1245
   Thr His  Lys Val Phe Asn Arg  Lys Trp Ala Thr Pro  Tyr His Val
       1250                 1255                 1260
   Asn Ile  Ala Asp Phe Ile Ile  Gln Gly Leu Ile Glu  Thr Arg Arg
       1265                 1270                 1275
   His Leu  Leu Val Asn Glu Arg  Met Asn Gly Leu Leu  Pro Val Ser
       1280                 1285                 1290
   Ser Val  Lys Cys Thr Ser Cys  Phe Arg Lys Lys Glu  Arg Glu Phe
       1295                 1300                 1305
   Phe Asp  Ile Pro Glu Glu Phe  Thr Trp Lys Asn Glu  Ser Lys Thr
       1310                 1315                 1320
   Ser Asp  Pro Ala Tyr Thr Tyr  Phe Thr Thr Trp Cys  Asp Leu Pro
       1325                 1330                 1335
   Arg Val  Ser Asn Leu Pro Glu  Met Asp Gln Arg Ser  Ala Thr Arg
       1340                 1345                 1350
   Leu Leu  Gly Arg Gly Leu Ala  Leu Asn Arg Ser Ser  Ser Gly Glu
       1355                 1360                 1365
   Ile Ile  Thr Lys Phe Tyr Ser  Met Pro Met Glu Ser  Gln Arg Leu
       1370                 1375                 1380
   Leu His  Pro Val Glu Leu Leu  Leu Gly Tyr Gly Glu  Gly Val Ile
       1385                 1390                 1395
   Phe Gly  Tyr Leu Arg Ser Gln  His Ile Asn His Gly  Ala Leu Phe
       1400                 1405                 1410
   His Ile  Gly Asp Glu Ser Leu  Ala Lys Lys Leu Arg  Arg Tyr Val
       1415                 1420                 1425
   Leu Asp  Thr Lys Thr Gln His  Ala Lys Gln Ile Gly  Tyr Leu Phe
       1430                 1435                 1440
   Gln Asp Glu Asp Ser Leu His Glu Leu Leu Gly Gln Gly Leu Cys
   1445 1450 1455
   Pro Tyr Ile Pro Arg Ser Ile Pro Leu Thr Ile Thr Glu Leu Thr
   1460 1465 1470
   Asn Ala Cys Ala Ile Thr Thr Ile Arg Ala Thr Glu Val Ile Leu
   1475 1480 1485
   Ser Thr Lys Ala Arg Ile His His Met Pro Val Gln Ala Ile Asp
   1490 1495 1500
   Glu Ser Asp Val Asp Thr Ser Arg Leu Ala Ala Asn Thr Met Gln
   1505 1510 1515
   Thr Ile Leu Gly Asp Pro Arg Pro Met Asn Leu Val His Leu Asp
   1520 1525 1530
   Cys Asp Leu Thr His Asn Met Val Ala Trp Glu Ser Glu Val Glu
   1535 1540 1545
   Leu Asp Ile Leu Lys Ser Glu Asn Phe His Ile Asp Gly Leu Leu
   1550 1555 1560
   Val Glu Leu Thr Ala Arg Glu Leu Pro Ile Gly Asp Thr Pro Trp
   1565 1570 1575
   Lys Gln Arg Asp Trp Thr Cys Ser Asn Asp Pro Lys Ile Ile Ala
   1580 1585 1590
   Lys Gly Ile Lys Thr Lys Ser Leu Phe Ile His Gln Gly Val Thr
   1595 1600 1605
   Gly Ala Ile Asn Leu Ile Pro Asp Leu Leu Val Val Ile Gly Gly
   1610 1615 1620
   Gly Leu Gly Gly Cys Ala Val Pro Tyr Leu Gln Glu Trp Pro Asp
   1625 1630 1635
   Thr Pro Ile Ile Phe Ala Thr Leu Phe Asp Glu Arg Glu Arg Ile
   1640 1645 1650
   Ser Glu Asp Gly Asp Leu Ile Val Pro Pro Glu Leu Leu Val Arg
   1655 1660 1665
   Gly Met Ala Pro Arg Met Ile Glu Arg Glu Ile Leu Glu Ala Glu
   1670 1675 1680
   Leu Cys Asp Ile Thr Asn Glu Gly Asn Arg Arg Leu Leu Ile Arg
   1685 1690 1695
   Leu Val Thr Lys Asn Lys Gly Gly Gly Lys Val Val Leu Ile Asp
   1700 1705 1710
   Glu Ile Glu Asn Arg Gly Ala Pro Glu Ser Leu Leu Gln Ser Ser
   1715 1720 1725
   Leu Gln Asp Leu Phe Glu Lys Leu Asp Lys Val Cys Lys Leu Thr
   1730 1735 1740
   Ser Val His Thr Val Arg Glu Ser Thr Val Glu Gln Phe Ser Gln
   1745 1750 1755
   Arg Val Asn Ser Ile Lys Arg Ser Arg Lys Ala Val Thr Leu His
   1760 1765 1770
   Trp Asn Arg Tyr Asn Arg Arg Asp Gln Phe Glu Ala Leu Val Ile
   1775 1780 1785
   Val Lys Gly Glu Glu Ala Lys Ser Asp Tyr Arg Ile Ser Thr Ile
   1790 1795 1800
   Thr Ser Ala Lys Ala Phe Arg Lys Ile Asp Glu Gln Leu Glu Ile
   1805 1810 1815
   Asp Gly Arg Leu Ser Ser Thr Gln Trp Ser Leu Pro Ala Leu Pro
   1820 1825 1830
   Ser Arg Glu Lys Asn Ile Leu Phe Gly Tyr Val Ser Ser Val Phe
   1835 1840 1845
   Leu Lys Met Asn Leu Ala Leu Ser Ala Asn Asp Met Asp Arg Glu
   1850 1855 1860
   Arg Leu Ile Glu Thr Ile Glu Gly Thr Ala Pro Gly Leu Ile Ser
   1865 1870 1875
   Trp Lys Glu Lys Leu Glu His Arg Asp His Ala His Arg Ser Asp
   1880 1885 1890
   Ile Glu Glu Lys Gly Ile Thr Gln Asp Lys Ile Phe Asn Leu Ile
   1895 1900 1905
   Cys Leu  Ser Trp Val Leu Lys  Gly Leu Arg Tyr Gly  Val Trp Asp
       1910                 1915                 1920
   Thr Asp  Ala Gln Ser Ile Val  Ala Gln Thr Val Tyr  Ile Thr Arg
       1925                 1930                 1935
   Gly Pro  Lys Leu Cys Pro Leu  Gly Glu Lys Pro Lys  Arg Ile Phe
       1940                 1945                 1950
   Ala Ser  Phe Lys Leu Gln Ser  Gly Lys Arg Val Glu  Asp Ala Lys
       1955                 1960                 1965
   Gly Phe  Leu Ser Ala Leu Leu  His Leu Glu Gly Phe  Phe Pro Leu
       1970                 1975                 1980
   Gly Glu  Gln
       1985
<210> 27
   <211> 11065
   <212> DNA
   <213> Viral hemorrhagic septicemia virus
<400> 27
   aataacggca cataagtggc aaaaagtttt caagtttgaa attccaagaa tttggaaagt      60
   gaaagttgaa cacagagtca tatctcgtaa tctttgaaca aaagaactca gttgaagaat     120
   ggacggagga cttcgtgcag cgttctcagg tttgaatgac gtcaggatcg accccaccgg     180
   tggagaggga cgggtacttg tacctggtga ggtggagctc attgtgtatg tgggtgggtt     240
   tagtgcagat gatgggaagg tgatcgtaga tgcactttct gcacttgggg gaccccagac     300
   tgtgcaggca ttgtccgttc ttctctccta tgtacttcaa gggaacaaac aggaggacct     360
   ggaggcaaag tgcaaggtgc tcacagacat gggcttcagg gtgacacagt caaccagggc     420
   gacgggcatc gatgcaggaa tcctgatgcc tatgagagag ctggccctgg ccgtcaataa     480
   cgacagcctc atggacatcg ttaaggggac cctgatgaca tgttcccttc tgaccaagta     540
   ctcggtggac aagatgatca agtacatcac caagaagctt ggggacctgg cagacactca     600
   gggaattggg gaactgcagc acttcaccgc cgacaaggca gccatcagga aactcgcagg     660
   gtgtgtgcgt cccgggcaga agatcaccaa ggccctctat gcattcatcc tgaccgagat     720
   cgcagatccc accacccaat caagggcccg agccatgggg gcattgaggc tcaacgggac     780
   aggaatgacc atgatcgggt tgttcaccca ggccgccaac aacctgggca ttgccccggc     840
   gaagctgctg gaggacctct gcatggagtc tctggttgag tcagccagac ggatcatcca     900
   gctgatgaga caggtgtcag aggcaaagtc catccaagag cgctacgcca tcatgatgag     960
   tcggatgctg ggggaatcct actacaagtc gtacggacta aatgacaact ccaagatctc     1020
   ctacattctg tcgcagatca gtgggaagta cgcagtggac tccctggaag gcctggaggg 1080
   gatcaaggtg acagagaagt tccgcgagtt cgctgaactt gttgccgagg tcctggtgga 1140
   caagtacgag aagattgggg aggacagtac tgaggtttca gatgtcatca gggaggcggc 1200
   tagacagcac gcgcgcagga catccgccaa gccagagccc aaggcccgca acttcaggag 1260
   ccccaccgga agagggaagg agcaggatac agaggagtct gacgatgagg actaccccga 1320
   agactccgat taaaaagcac tctcgtctca tgaccaacat agatagaaaa aaatggcacg 1380
   attataagaa tttctctttg atcaaagcaa ggcaaacacc gaaatcacac aatggctgat 1440
   attgagatga gtgaatcctt ggtcctgtcc catgggtccc tagctgacct ggataggaaa 1500
   ctagacaacg cccccaagga cactaggtca gctctttttt catctacctc agggttcacc 1560
   aaacaaaaat caagtcccaa gaagaagccc aatcctacaa cgctggaaga actcattgga 1620
   cacttcgtct ctgaggatct ccagctagac gccacaaagg ccttcggaca gctcctaaga 1680
   cgtatcaaga tgtcccatca ggaagaactt actcaacatc tggagaaggt caacggagag 1740
   aaccgggcca agatgggagc cctcctggag tctcagaaag aaaatgggaa gaagaccgac 1800
   aacatactct caatcctcat ctccatgcga ggagaagggg cggaaaatgc atccaaaaag 1860
   cccaaggtcc tagatgggga tcaggtccgg aacgagaggg cacttggatt caacagggga 1920
   ctgactacgg ctgccatcgc catgaagaag ttcaagttag aagatcctct cgcactctgc 1980
   aaaggctcag tcaaacgagc agctctctcc gccatggaaa aggaggaata cgacggggag 2040
   cgagagacct actccacagt ctcaaaagcg attaaggcgg agctggacaa gctagagtag 2100
   ggacaacaca atgcatcgta cagatagaaa aaaacggcac gactgttcga gcaaacttac 2160
   tcccaaaaag agacactcca gcagccagca aaccccagac aatccacaac tcaagacatg 2220
   actctgttca aaagaaagcg caccatcctg gttccccctc ctcacatcac gtccaacgac 2280
   gaggaccgtg tctcaaccat cctgacggaa ggcactctaa ccatcacggg tcctcctccg 2340
   ggaaaccagg tcgacaagat ctgcatggct atgaagctgg ctcgagccat tctctgcgag 2400
   gatcagcacc cagcattcaa tcccctggtt tatctattcc agagtgctat gatctttgga 2460
   gagaccagtg agaagattga cttcgggact cgtagcaaga ccctgatcac aagcttcaag 2520
   atagctgagg caaaggccat ctacctggaa tcaagtccgg tgagatctcg catagaagcc 2580
   aagaagtata ccactcccat cagacatggg agtgtgactt actatggccc gttcgtgttt 2640
   gcggatgacc acgttggagg gaaaggccac cgcgagaaac tgggggcact atgtgggttt 2700
   ctccagtcgg gaccttacgg gcaggcgaag gactactaca atcgtgctgt cgaggaagaa 2760
   atgggcattc tcccgaggga cccaaagcgc agatctggag cctcttctgc tcagccccgg 2820
   taggtccaaa tgacaaactc aggggtgata ccatactccc attagataga aaaaaatggc 2880
   acatttgtgt acacaacaag ctatacctac aatggaatgg aatacttttt tcttggtgat 2940
   cttgatcatt ctcatcaaga gcaccacatc acagatcacc caaagacctc cgatcgagaa 3000
   catctcgacg taccatgcag attgggacac tccgttgtac actcacccct ccaactgcag 3060
   agaagactcc tttgtcccga ttcgaccagc tcaactcagg tgtcctcatg aatttgaaga 3120
   catcaacaag ggactggtct ccgttccaac tcagatcatt catctcccgc tatcagtcac 3180
   cagcgtctcc gcagttgcaa gtggccacta cctgcacaga gtgacctatc gagtcacctg 3240
   ctcaaccagc ttctttgggg gacaaaccat cgaaaagacc atcctggaag caaaattgtc 3300
   tcgtcaggag gccgcaaatg aggcaagcaa agaccatgag tacccgttct ttcctgaacc 3360
   ctcctgcatc tggatgaaaa ataatgtcca taaggatata actcactatt ataagacccc 3420
   aaaaacagtc tctgtggatc tctacagcag gaaatttctt aaccctgatt tcatagaggg 3480
   ggtttgtacg acctcgccct gtcagactca ttggcaagga gtctattggg tcggtgccac 3540
   acccacagcc cattgtccca cttcagaaac actagagggg cacctgttca ccagaaccca 3600
   tgatcacaga gtggtcaagg caattgtggc gggccatcac ccatggggac tcacgatggc 3660
   atgcacagtg acattctgtg ggacagattg gatcaagacc gacctgggag acctgatcaa 3720
   ggtggtagga caggggggcg aaacaaaact gactccaaaa aaatgtgtca atgctgacat 3780
   ccagatgagg ggggcaacag acgacttctc atatctcaac catctcatca caaacatggc 3840
   tcaaaggacc gagtgtctag acgcccatag tgacatcact gcctccggta aaatatcctc 3900
   gtttctcctc tcaaagtttc gtcccagcca ccccggaccc ggcaaggcac attacctcct 3960
   tgatggtcta atcatgcgag gtgactgtga ctatgaggcg gtagtcagta tcaactacaa 4020
   tagcgctcag tacaagacgg tgaacaacac atggaagtca tggaaacgga tagacaacaa 4080
   tacagatggg tacgatggga tgatatttgg ggacaaactg atcatcccag acatcgagaa 4140
   atatcagagt atctatgata gtgggatgct cgttcaaagg aacctggtgg aagttcctca 4200
   tctgagcatt gtgtttgtct ccaacacatc tgatctctcc accaatcaca tccacaccaa 4260
   cttaatcccc tcggattggt cattcaactg gagcctctgg ccgtcattat cagggatggg 4320
   agttgtggga ggggccttcc ttctattggt actttgctgt tgttgcaaag cgtctcctcc 4380
   tattccaaat tacgggattc ctatgcagca attctccaga aatcagatgg tctgaacaca 4440
   cctgcccgaa tgaacatcat tatcctcata ggtagataga aaaaaatggc acttctttga 4500
   aaaagaaaca tgacaaccca actcgcgctc gacacagtca gcttttctcc actcgtcctc 4560
   cgcgagatga tcacacacag gctcacattc gacccaagta actacctcaa ctgcgacatc 4620
   gatcggtcgg acgtatccac catgagtttc ttcgaaacga ctcttcccag gatcttggat 4680
   gacttgaggg cttgcccacg gcttccctac ctccatgtac tcgacatgag gataagtctc 4740
   ctggagagaa cccattacat gttcaaaaac gtcccctcca gtcccgccac aaccggcaga 4800
   ccgactgatc ctggactcat cattatttca tgtgcagatg ttggggtcct aacaaatggc 4860
   tccgggttca cttcatgagc ctcccccccg cccctcagat agaaaaaaat ggcacttttg 4920
   ttgtttgtaa tcctacgctc aacaaagaag tgaccctaca gggggtaata cctgacacaa 4980
   tcaaattaga tcaccttata gcaaccatgg aaatgtttga actagaccgg gaagtacatc 5040
   aggagcgact tccctccgaa tgttcgctga actctcccct aaacctttcc ctatctctcc 5100
   aactcttcgg gcgcctaact ccaaagacag aacacatcag gtaccaagct gggaggataa 5160
   agagatggct cgtcaaacag taccaactgg tgcacctcag agagcttgag attgattcaa 5220
   caagaatcca aggatatctg atcccccatc ttttgaagac acagagtaat gagttaggat 5280
   cgtcggtaat aaaaaactgg gggatggtct ccaagtacta cctgtctcta gggtacaccc 5340
   ttcccccaaa ggacaagttt gactttcgag aagttgctcc ttactggaac ttggcctcac 5400
   agttgagaga ggtcactttg gagagccaga aggtggatac cagagggaaa gaaaaaagaa 5460
   agttgtacca ggtggaagat gtagagttcg agtttaaaga gggggtggtg gtaattcgtg 5520
   cgggcctgga tggactcctg aatgagttcc ttggggggga aaaacttggg gcagtaacat 5580
   atgtggaata cttggccttc ttcaaaatca tcaatcaaag agcacaagca ctcctgctca 5640
   cggccatctg ccaggccctt gaaccagacc tagttcctcc ttgtagtgga atactttccg 5700
   tctatgcaga ggtcgactcc gtactccgtc gggcgggaca atccgccatt gacctgctca 5760
   aattgtggga acctcttgtc ctgacaaaac tgggggacgt gctcggtgac agattcggcc 5820
   tcgaggacga cttcaaggac acaattaggg gagaggcgaa caaattagct aagaaactcc 5880
   acgtcaccag atcttataaa cggatgatga agaccctaga tcaggagact agggcacagg 5940
   ccctctttca atattttgga ctgttcaaac acttcgccta cccccgcgtc tattcgagag 6000
   aaaccattga ggctattcaa gaggtcagtg acaaaccgag tgactcgtcc ccactcaatt 6060
   atctaagtga ccaatgcaaa atcagggagg agttctacat ccggtacacg aaggcttatc 6120
   acagggcacc ggccatgaac ctgggtcaac taggacaggg ttcctaccta cgccaggttt 6180
   tggaggcggg aaagatcccc aacgccaaga atgccctcta ttcgctcctc gagtggttct 6240
   ttgttcgttt tgagaagagc atcgaatggc ccctcagtga caccttatcc accttcctct 6300
   cagacaaggc gatcacccaa aatagggaca tctggtatga cgggggatca tcaggaaggg 6360
   acaccacaga gaagagacta ctcctgaagt ttatcaagga gaatgaagac agtgtggaaa 6420
   aggtgatcct caaggccgat gaaatctacg ataaggaggc ggatcagatc attgcactga 6480
   aagtcaagga gatggaactg aaaatcaaag ggagaggatt tggcttgatg gctttcaagc 6540
   caagactact tcaggtcctc cgagagagta tcgccaagaa gaccagcaag ttattcccag 6600
   aaataaccat gaccttttca gacctcgagt tgaaaaagaa aaaatttcaa ctttctagga 6660
   agagcgatga caggagggga tacatccata tcagcaaaag tcttgacata aacaagttct 6720
   gcaccagtca acgccagttc aactccctgg cagtcttcca gagcctagat gaacttctgg 6780
   ggacagatca gctcttcacg agggtacacg agatcttcga aaaaacatgg atcgtggatg 6840
   gatccgccag tgacccccca gacctggtga cgttcaaggc gaggtatgaa gaggcactgg 6900
   ccctagggat tgaggccccg cacgtgtggg cagacggagc cttctctgga ctaatgggtg 6960
   gaattgaggg tctctgtcag tacgtctgga caatctgtct cttactcaga gtggagcgcg 7020
   tcctcgctgt gacccaactg actcactttg tcatggcaca aggggacaat gtgatcatca 7080
   atctcatcat ccctgttgag gtggaccggg tgggtggggt tgtcgaaggg gaacgtgcca 7140
   gaattcaacg catcagcaaa gacatcgact ctgctctcga acgagagctg ctgaggagtg 7200
   gcttgactct taagatagag gagaccctga ccagcgaaaa cctctcaatc tacggaaaag 7260
   acctccattg cccacaacac ctgacccttg ccgtgaagaa ggcaggatct gcctctatca 7320
   tatcaagcga acaatatcaa gacgtgccca cctttctgtc aggcctcggg acgggaatgg 7380
   aaacaatctc agagtgcgtc aacaacaaag tgagtgctca tctgttcggg gtcattctgg 7440
   gagcggctgg atggaagagc ctcgcacaga gacaaacctg gaaagggtgg gaatacccct 7500
   ttcagaatga gacctcccgg agacaggtcc ggtcacaggg aatccttctt cagaaggggg 7560
   agtccacaat ggtacacaaa gagcccgagg ccaacccgga aaaacggacc atagaactgc 7620
   tcctagtgag tagtcttttc ggatcagctc tggggatgct cccctttccc actcccattg 7680
   accttgagaa gaggggcgtg ggagactacg tcactcaccg gctctcaatc gttaagatgg 7740
   ccctggtctc aaaaaaactc cccaatagga tgatcgagat gattgtctcc accatgaacc 7800
   ttcccctttc tcgagaacag gatctcacta agttattcga ctctccattc tccctcaatc 7860
   ttgccacaga ggaagacgct gcatcagtga tcaagcgact cgcaaggggc acactaaggg 7920
   ggcttgacat caaaaacaaa aaactggcag atcacatagc caccatggat cagggaataa 7980
   cccaaattga cgaagcactg gcaagtgcgg acacaatcaa ccccaggatc gcctaccaat 8040
   ttagaaacat cacagaccaa aaagagtcag aaatgttcgt caccaagttc gccaccgcga 8100
   aaaccatgcg gatggttgcc ctctcctcat ctcaggatgt ctcagttgta ggacttttga 8160
   acaaacgcag ccaggcaaaa gagatctaca ccatttggcg gacacagagg aagggggaga 8220
   cactctggac atgctcgacc cagcaggcaa agaaactcag ggaccgatcc tggggaaaaa 8280
   acataattgg ggtcacctct ccttcccctc tggaagccat tctcttcaag ctgattgatc 8340
   caatttcctg ggaggaagaa aaggaggcac accacttcac catccattac tacctctcaa 8400
   agcccagtct ttcttcaaag acagcccaca cgaccagggg tccacttgtt ccctacttcg 8460
   gaacccagac caagccactg atcgcaaaag cctacatgga gctcaaggga aatcccagga 8520
   ccaacaaggc tctgcagttg cttagcatga gggagaccat gatcaaggca gggtccaatc 8580
   tagacaaact cctcttgtcc ctgtgttcta acgccctaga catcgacgtc aactccctcc 8640
   cttcacttca ggcacaagaa gaggccagtg ctggagaggg ggtccgagga ggtatcaaag 8700
   agtccatgtc cccagttgga ccggacaacc tctacacaca catcacccac aaggtgttcg 8760
   agcgtcagtg gttgtctgag tttcatgtga acatcgctga cttcatcatc tggggaatca 8820
   cgaagactcg gcagcatctc caggtcaaca ctgacctggg ggggagcctc ccgatctgtg 8880
   ttccagcgtg tcaagagtgc tacagagaga aagaacgtgt cttcctagac atcccaaggg 8940
   agactgagtg ggtcaacacc tccaccactt cagacaaggc ccaaacctac ttctccacct 9000
   ggtgtgatct cccaagagtg tccaccctcc cctcactaga ccagaaggac gcaacctgtc 9060
   tgatggggag gtccatggca actcaaaaat ctactccggg ggaatccatc accaagttct 9120
   acagcacagc cccagacacc catcgacttc tccatcctgt gaccttggtc ctgggctatg 9180
   cagaaggaac aattttttct tacatgaaaa gtcagcacaa cattcatggg tccctttacc 9240
   atccgaacat cgaagagatc gaaccagctc ttgagaagta tgtgatcgac acgaagacat 9300
   cccataccaa gcacctggga tatctgtttc aagatgccga ctccctccag gaactgctag 9360
   agacaggaat gacaccatac attccccgat ccattcctct cacgatcact gaactgacaa 9420
   gtgcatgctg catgacactt gccaaagcaa tctccattgt cctcagaacc ggagtgacca 9480
   ttccattgat gccagaaaat ggatacggag aaaacgatat ccaagtggca agactcaccg 9540
   ccaactccct ttcccgactc atgcccaggg ggaggattca gctggtctac ctggactgtg 9600
   acctgacctc gcagatgacc gcgtgggtgc caaccagcca gccgagcgtc ctgggatctg 9660
   tcaacttcca catcgagggg gttaccatct ccctcacagc aacagagatg agagtgggac 9720
   aagaaagctg ggaagagaga aagtggacat gttcaaacaa cagacacatc atcgcaaagg 9780
   gggtcaagac caaatctctg ttcatacatc agagcgtccc cgagatcatc acacacccgc 9840
   ccgacctcat tgtggtgata ggagggggcc tagggggctg tgtagtgccc taccttcaaa 9900
   agtggagagg tcccaaagta atcttctgca ctctctttga tgaacgagag cggatctcgg 9960
   aggacggaga cctgatcatt cccccggagc tcctagttcg aggactcgct gggagaatga 10020
   ttgagaaaga actcctggag gctgagatgt gtgacatcac ggtcaagggg aatagggacc 10080
   tcctcatcaa agtagtgcag aaatgggttc agccgaacga acatgtcctt ctcattgatg 10140
   agattgaaaa taggggggat caagagtcag tactccaatc ttccatcagt gagcttctga 10200
   caagaatgga cagcgtgtgc aacctcactt ctgttcacac catccgagag accggacctc 10260
   gacaattcgc ccagagggtg aacacgatta ggagaggcag aaggacagca acactccact 10320
   ggaaccaata caaccgaaga gaccaggtgg aggccctcct acttgttgag agccacacca 10380
   ggaagaccga actgcacgtc acatcatccg ccgtacaggc ggcattcaga aagatagacg 10440
   agaagctcga gtcggagagt cgactagagc acagcaaatg gtcactccct gaactccctc 10500
   caagggagaa agacatccta cttggatatg tggcatctgt ctttttaaag ctagggctgg 10560
   ttgtgacaga cagacacatg tcggctgccg cgctcatcac tctcctcgaa gaagctgggc 10620
   caaaaatgat atcctgggat aagaagatgg aacatcgaac ctgggcctcc tcggatgcca 10680
   tcactgagaa aggaatcacc caggaccaga tcttttccct tctctgcttt gcatgggccc 10740
   ttaggggact aaaatcaggg gactgggaac acaacgcaga cgccatcatt ctccaggatg 10800
   tccacatcga cacaggtccg aggctctgtc agatgggcga gtctcccaag aggacatttg 10860
   catccttcag gctgcacaac accaaaaagg cggaggatct caaggggtac ttaggtgctc 10920
   tgctccacct ggagtccttc tttccatttg gagaacagtg acgatcgccc cgctcatcaa 10980
   ttagattgaa aaaaactatg acctgggttc aatgaccacg tgaaccctcg tgggcaccat 11040
   gtcccagacc tcccgcctcg gattt 11065
<210> 28
   <211> 404
   <212> PRT
   <213> Viral hemorrhagic septicemia virus
<400> 28
   Met Asp Gly Gly Leu Arg Ala Ala Phe Ser Gly Leu Asn Asp Val Arg
   1               5                   10                  15
   Ile Asp Pro Thr Gly Gly Glu Gly Arg Val Leu Val Pro Gly Glu Val
               20                  25                  30
   Glu Leu Ile Val Tyr Val Gly Gly Phe Ser Ala Asp Asp Gly Lys Val
           35                  40                  45
   Ile Val Asp Ala Leu Ser Ala Leu Gly Gly Pro Gln Thr Val Gln Ala
       50                  55                  60
   Leu Ser Val Leu Leu Ser Tyr Val Leu Gln Gly Asn Lys Gln Glu Asp
   65                  70                  75                  80
   Leu Glu Ala Lys Cys Lys Val Leu Thr Asp Met Gly Phe Arg Val Thr
                   85                  90                  95
   Gln Ser Thr Arg Ala Thr Gly Ile Asp Ala Gly Ile Leu Met Pro Met
               100                 105                 110
   Arg Glu Leu Ala Leu Ala Val Asn Asn Asp Ser Leu Met Asp Ile Val
           115                 120                 125
   Lys Gly Thr Leu Met Thr Cys Ser Leu Leu Thr Lys Tyr Ser Val Asp
       130                 135                 140
   Lys Met Ile Lys Tyr Ile Thr Lys Lys Leu Gly Asp Leu Ala Asp Thr
   145                 150                 155                 160
   Gln Gly Ile Gly Glu Leu Gln His Phe Thr Ala Asp Lys Ala Ala Ile
                   165                 170                 175
   Arg Lys Leu Ala Gly Cys Val Arg Pro Gly Gln Lys Ile Thr Lys Ala
               180                 185                 190
   Leu Tyr Ala Phe Ile Leu Thr Glu Ile Ala Asp Pro Thr Thr Gln Ser
           195                 200                 205
   Arg Ala Arg Ala Met Gly Ala Leu Arg Leu Asn Gly Thr Gly Met Thr
       210                 215                 220
   Met Ile Gly Leu Phe Thr Gln Ala Ala Asn Asn Leu Gly Ile Ala Pro
   225                 230                 235                 240
   Ala Lys Leu Leu Glu Asp Leu Cys Met Glu Ser Leu Val Glu Ser Ala
                   245                 250                 255
   Arg Arg Ile Ile Gln Leu Met Arg Gln Val Ser Glu Ala Lys Ser Ile
               260                 265                 270
   Gln Glu Arg Tyr Ala Ile Met Met Ser Arg Met Leu Gly Glu Ser Tyr
           275                 280                 285
   Tyr Lys Ser Tyr Gly Leu Asn Asp Asn Ser Lys Ile Ser Tyr Ile Leu
       290                 295                 300
   Ser Gln Ile Ser Gly Lys Tyr Ala Val Asp Ser Leu Glu Gly Leu Glu
   305                 310                 315                 320
   Gly Ile Lys Val Thr Glu Lys Phe Arg Glu Phe Ala Glu Leu Val Ala
                   325                 330                 335
   Glu Val Leu Val Asp Lys Tyr Glu Lys Ile Gly Glu Asp Ser Thr Glu
               340                 345                 350
   Val Ser Asp Val Ile Arg Glu Ala Ala Arg Gln His Ala Arg Arg Thr
           355                 360                 365
   Ser Ala Lys Pro Glu Pro Lys Ala Arg Asn Phe Arg Ser Pro Thr Gly
       370                 375                 380
   Arg Gly Lys Glu Gln Asp Thr Glu Glu Ser Asp Asp Glu Asp Tyr Pro
   385 390 395 400
   Glu Asp Ser Asp
<210> 29
   <211> 222
   <212> PRT
   <213> Viral hemorrhagic septicemia virus
<400> 29
   Met Ala Asp Ile Glu Met Ser Glu Ser Leu Val Leu Ser His Gly Ser
   1 5 10 15
   Leu Ala Asp Leu Asp Arg Lys Leu Asp Asn Ala Pro Lys Asp Thr Arg
   20 25 30
   Ser Ala Leu Phe Ser Ser Thr Ser Gly Phe Thr Lys Gln Lys Ser Ser
   35 40 45
   Pro Lys Lys Lys Pro Asn Pro Thr Thr Leu Glu Glu Leu Ile Gly His
   50 55 60
   Phe Val Ser Glu Asp Leu Gln Leu Asp Ala Thr Lys Ala Phe Gly Gln
   65 70 75 80
   Leu Leu Arg Arg Ile Lys Met Ser His Gln Glu Glu Leu Thr Gln His
   85 90 95
   Leu Glu Lys Val Asn Gly Glu Asn Arg Ala Lys Met Gly Ala Leu Leu
   100 105 110
   Glu Ser Gln Lys Glu Asn Gly Lys Lys Thr Asp Asn Ile Leu Ser Ile
   115 120 125
   Leu Ile Ser Met Arg Gly Glu Gly Ala Glu Asn Ala Ser Lys Lys Pro
   130 135 140
   Lys Val Leu Asp Gly Asp Gln Val Arg Asn Glu Arg Ala Leu Gly Phe
   145 150 155 160
   Asn Arg Gly Leu Thr Thr Ala Ala Ile Ala Met Lys Lys Phe Lys Leu
   165 170 175
   Glu Asp Pro Leu Ala Leu Cys Lys Gly Ser Val Lys Arg Ala Ala Leu
   180 185 190
   Ser Ala Met Glu Lys Glu Glu Tyr Asp Gly Glu Arg Glu Thr Tyr Ser
           195                 200                 205
   Thr Val Ser Lys Ala Ile Lys Ala Glu Leu Asp Lys Leu Glu
       210                 215                 220
<210> 30
   <211> 201
   <212> PRT
   <213> Viral hemorrhagic septicemia virus
<400> 30
   Met Thr Leu Phe Lys Arg Lys Arg Thr Ile Leu Val Pro Pro Pro His
   1               5                   10                  15
   Ile Thr Ser Asn Asp Glu Asp Arg Val Ser Thr Ile Leu Thr Glu Gly
               20                  25                  30
   Thr Leu Thr Ile Thr Gly Pro Pro Pro Gly Asn Gln Val Asp Lys Ile
           35                  40                  45
   Cys Met Ala Met Lys Leu Ala Arg Ala Ile Leu Cys Glu Asp Gln His
       50                  55                  60
   Pro Ala Phe Asn Pro Leu Val Tyr Leu Phe Gln Ser Ala Met Ile Phe
   65                  70                  75                  80
   Gly Glu Thr Ser Glu Lys Ile Asp Phe Gly Thr Arg Ser Lys Thr Leu
                   85                  90                  95
   Ile Thr Ser Phe Lys Ile Ala Glu Ala Lys Ala Ile Tyr Leu Glu Ser
               100                 105                 110
   Ser Pro Val Arg Ser Arg Ile Glu Ala Lys Lys Tyr Thr Thr Pro Ile
           115                 120                 125
   Arg His Gly Ser Val Thr Tyr Tyr Gly Pro Phe Val Phe Ala Asp Asp
       130                 135                 140
   His Val Gly Gly Lys Gly His Arg Glu Lys Leu Gly Ala Leu Cys Gly
   145                 150                 155                 160
   Phe Leu Gln Ser Gly Pro Tyr Gly Gln Ala Lys Asp Tyr Tyr Asn Arg
                   165                 170                 175
   Ala Val Glu Glu Glu Met Gly Ile Leu Pro Arg Asp Pro Lys Arg Arg
               180                 185                 190
   Ser Gly Ala Ser Ser Ala Gln Pro Arg
               195                 200
<210> 31
   <211> 507
   <212> PRT
   <213> Viral hemorrhagic septicemia virus
<400> 31
   Met Glu Trp Asn Thr Phe Phe Leu Val Ile Leu Ile Ile Leu Ile Lys
   1               5                   10                  15
   Ser Thr Thr Ser Gln Ile Thr Gln Arg Pro Pro Ile Glu Asn Ile Ser
               20                  25                  30
   Thr Tyr His Ala Asp Trp Asp Thr Pro Leu Tyr Thr His Pro Ser Asn
           35                  40                  45
   Cys Arg Glu Asp Ser Phe Val Pro Ile Arg Pro Ala Gln Leu Arg Cys
       50                  55                  60
   Pro His Glu Phe Glu Asp Ile Asn Lys Gly Leu Val Ser Val Pro Thr
   65                  70                  75                  80
   Gln Ile Ile His Leu Pro Leu Ser Val Thr Ser Val Ser Ala Val Ala
                   85                  90                  95
   Ser Gly His Tyr Leu His Arg Val Thr Tyr Arg Val Thr Cys Ser Thr
               100                 105                 110
   Ser Phe Phe Gly Gly Gln Thr Ile Glu Lys Thr Ile Leu Glu Ala Lys
           115                 120                 125
   Leu Ser Arg Gln Glu Ala Ala Asn Glu Ala Ser Lys Asp His Glu Tyr
       130                 135                 140
   Pro Phe Phe Pro Glu Pro Ser Cys Ile Trp Met Lys Asn Asn Val His
   145                 150                 155                 160
   Lys Asp Ile Thr His Tyr Tyr Lys Thr Pro Lys Thr Val Ser Val Asp
                   165                 170                 175
   Leu Tyr Ser Arg Lys Phe Leu Asn Pro Asp Phe Ile Glu Gly Val Cys
               180                 185                 190
   Thr Thr Ser Pro Cys Gln Thr His Trp Gln Gly Val Tyr Trp Val Gly
           195                 200                 205
   Ala Thr Pro Thr Ala His Cys Pro Thr Ser Glu Thr Leu Glu Gly His
       210                 215                 220
   Leu Phe Thr Arg Thr His Asp His Arg Val Val Lys Ala Ile Val Ala
   225                 230                 235                 240
   Gly His His Pro Trp Gly Leu Thr Met Ala Cys Thr Val Thr Phe Cys
                   245                 250                 255
   Gly Thr Asp Trp Ile Lys Thr Asp Leu Gly Asp Leu Ile Lys Val Val
               260                 265                 270
   Gly Gln Gly Gly Glu Thr Lys Leu Thr Pro Lys Lys Cys Val Asn Ala
           275                 280                 285
   Asp Ile Gln Met Arg Gly Ala Thr Asp Asp Phe Ser Tyr Leu Asn His
       290                 295                 300
   Leu Ile Thr Asn Met Ala Gln Arg Thr Glu Cys Leu Asp Ala His Ser
   305                 310                 315                 320
   Asp Ile Thr Ala Ser Gly Lys Ile Ser Ser Phe Leu Leu Ser Lys Phe
                   325                 330                 335
   Arg Pro Ser His Pro Gly Pro Gly Lys Ala His Tyr Leu Leu Asp Gly
               340                 345                 350
   Leu Ile Met Arg Gly Asp Cys Asp Tyr Glu Ala Val Val Ser Ile Asn
           355                 360                 365
   Tyr Asn Ser Ala Gln Tyr Lys Thr Val Asn Asn Thr Trp Lys Ser Trp
       370                 375                 380
   Lys Arg Ile Asp Asn Asn Thr Asp Gly Tyr Asp Gly Met Ile Phe Gly
   385                 390                 395                 400
   Asp Lys Leu Ile Ile Pro Asp Ile Glu Lys Tyr Gln Ser Ile Tyr Asp
                   405                 410                 415
   Ser Gly Met Leu Val Gln Arg Asn Leu Val Glu Val Pro His Leu Ser
               420                 425                 430
   Ile Val Phe Val Ser Asn Thr Ser Asp Leu Ser Thr Asn His Ile His
           435                 440                 445
   Thr Asn Leu Ile Pro Ser Asp Trp Ser Phe Asn Trp Ser Leu Trp Pro
       450                 455                 460
   Ser Leu Ser Gly Met Gly Val Val Gly Gly Ala Phe Leu Leu Leu Val
   465 470 475 480
   Leu Cys Cys Cys Cys Lys Ala Ser Pro Pro Ile Pro Asn Tyr Gly Ile
   485 490 495
   Pro Met Gln Gln Phe Ser Arg Asn Gln Met Val
   500 505
<210> 32
   <211> 122
   <212> PRT
   <213> Viral hemorrhagic septicemia virus
<400> 32
   Met Thr Thr Gln Leu Ala Leu Asp Thr Val Ser Phe Ser Pro Leu Val
   1 5 10 15
   Leu Arg Glu Met Ile Thr His Arg Leu Thr Phe Asp Pro Ser Asn Tyr
   20 25 30
   Leu Asn Cys Asp Ile Asp Arg Ser Asp Val Ser Thr Met Ser Phe Phe
   35 40 45
   Glu Thr Thr Leu Pro Arg Ile Leu Asp Asp Leu Arg Ala Cys Pro Arg
   50 55 60
   Leu Pro Tyr Leu His Val Leu Asp Met Arg Ile Ser Leu Leu Glu Arg
   65 70 75 80
   Thr His Tyr Met Phe Lys Asn Val Pro Ser Ser Pro Ala Thr Thr Gly
   85 90 95
   Arg Pro Thr Asp Pro Gly Leu Ile Ile Ile Ser Cys Ala Asp Val Gly
   100 105 110
   Val Leu Thr Asn Gly Ser Gly Phe Thr Ser
   115 120
<210> 33
   <211> 1984
   <212> PRT
   <213> Viral hemorrhagic septicemia virus
<400> 33
   Met Glu Met Phe Glu Leu Asp Arg Glu Val His Gln Glu Arg Leu Pro
   1 5 10 15
   Ser Glu Cys Ser Leu Asn Ser Pro Leu Asn Leu Ser Leu Ser Leu Gln
               20                  25                  30
   Leu Phe Gly Arg Leu Thr Pro Lys Thr Glu His Ile Arg Tyr Gln Ala
           35                  40                  45
   Gly Arg Ile Lys Arg Trp Leu Val Lys Gln Tyr Gln Leu Val His Leu
       50                  55                  60
   Arg Glu Leu Glu Ile Asp Ser Thr Arg Ile Gln Gly Tyr Leu Ile Pro
   65                  70                  75                  80
   His Leu Leu Lys Thr Gln Ser Asn Glu Leu Gly Ser Ser Val Ile Lys
                   85                  90                  95
   Asn Trp Gly Met Val Ser Lys Tyr Tyr Leu Ser Leu Gly Tyr Thr Leu
               100                 105                 110
   Pro Pro Lys Asp Lys Phe Asp Phe Arg Glu Val Ala Pro Tyr Trp Asn
           115                 120                 125
   Leu Ala Ser Gln Leu Arg Glu Val Thr Leu Glu Ser Gln Lys Val Asp
       130                 135                 140
   Thr Arg Gly Lys Glu Lys Arg Lys Leu Tyr Gln Val Glu Asp Val Glu
   145                 150                 155                 160
   Phe Glu Phe Lys Glu Gly Val Val Val Ile Arg Ala Gly Leu Asp Gly
                   165                 170                 175
   Leu Leu Asn Glu Phe Leu Gly Gly Glu Lys Leu Gly Ala Val Thr Tyr
               180                 185                 190
   Val Glu Tyr Leu Ala Phe Phe Lys Ile Ile Asn Gln Arg Ala Gln Ala
           195                 200                 205
   Leu Leu Leu Thr Ala Ile Cys Gln Ala Leu Glu Pro Asp Leu Val Pro
       210                 215                 220
   Pro Cys Ser Gly Ile Leu Ser Val Tyr Ala Glu Val Asp Ser Val Leu
   225                 230                 235                 240
   Arg Arg Ala Gly Gln Ser Ala Ile Asp Leu Leu Lys Leu Trp Glu Pro
                   245                 250                 255
   Leu Val Leu Thr Lys Leu Gly Asp Val Leu Gly Asp Arg Phe Gly Leu
               260                 265                 270
   Glu Asp Asp Phe Lys Asp Thr Ile Arg Gly Glu Ala Asn Lys Leu Ala
           275                 280                 285
   Lys Lys Leu His Val Thr Arg Ser Tyr Lys Arg Met Met Lys Thr Leu
       290                 295                 300
   Asp Gln Glu Thr Arg Ala Gln Ala Leu Phe Gln Tyr Phe Gly Leu Phe
   305                 310                 315                 320
   Lys His Phe Ala Tyr Pro Arg Val Tyr Ser Arg Glu Thr Ile Glu Ala
                   325                 330                 335
   Ile Gln Glu Val Ser Asp Lys Pro Ser Asp Ser Ser Pro Leu Asn Tyr
               340                 345                 350
   Leu Ser Asp Gln Cys Lys Ile Arg Glu Glu Phe Tyr Ile Arg Tyr Thr
           355                 360                 365
   Lys Ala Tyr His Arg Ala Pro Ala Met Asn Leu Gly Gln Leu Gly Gln
       370                 375                 380
   Gly Ser Tyr Leu Arg Gln Val Leu Glu Ala Gly Lys Ile Pro Asn Ala
   385                 390                 395                 400
   Lys Asn Ala Leu Tyr Ser Leu Leu Glu Trp Phe Phe Val Arg Phe Glu
                   405                 410                 415
   Lys Ser Ile Glu Trp Pro Leu Ser Asp Thr Leu Ser Thr Phe Leu Ser
               420                 425                 430
   Asp Lys Ala Ile Thr Gln Asn Arg Asp Ile Trp Tyr Asp Gly Gly Ser
           435                 440                 445
   Ser Gly Arg Asp Thr Thr Glu Lys Arg Leu Leu Leu Lys Phe Ile Lys
       450                 455                 460
   Glu Asn Glu Asp Ser Val Glu Lys Val Ile Leu Lys Ala Asp Glu Ile
   465                 470                 475                 480
   Tyr Asp Lys Glu Ala Asp Gln Ile Ile Ala Leu Lys Val Lys Glu Met
                   485                 490                 495
   Glu Leu Lys Ile Lys Gly Arg Gly Phe Gly Leu Met Ala Phe Lys Pro
               500                 505                 510
   Arg Leu Leu Gln Val Leu Arg Glu Ser Ile Ala Lys Lys Thr Ser Lys
           515                 520                 525
   Leu Phe Pro Glu Ile Thr Met Thr Phe Ser Asp Leu Glu Leu Lys Lys
       530                 535                 540
   Lys Lys Phe Gln Leu Ser Arg Lys Ser Asp Asp Arg Arg Gly Tyr Ile
   545                 550                 555                 560
   His Ile Ser Lys Ser Leu Asp Ile Asn Lys Phe Cys Thr Ser Gln Arg
                   565                 570                 575
   Gln Phe Asn Ser Leu Ala Val Phe Gln Ser Leu Asp Glu Leu Leu Gly
               580                 585                 590
   Thr Asp Gln Leu Phe Thr Arg Val His Glu Ile Phe Glu Lys Thr Trp
           595                 600                 605
   Ile Val Asp Gly Ser Ala Ser Asp Pro Pro Asp Leu Val Thr Phe Lys
       610                 615                 620
   Ala Arg Tyr Glu Glu Ala Leu Ala Leu Gly Ile Glu Ala Pro His Val
   625                 630                 635                 640
   Trp Ala Asp Gly Ala Phe Ser Gly Leu Met Gly Gly Ile Glu Gly Leu
                   645                 650                 655
   Cys Gln Tyr Val Trp Thr Ile Cys Leu Leu Leu Arg Val Glu Arg Val
               660                 665                 670
   Leu Ala Val Thr Gln Leu Thr His Phe Val Met Ala Gln Gly Asp Asn
           675                 680                 685
   Val Ile Ile Asn Leu Ile Ile Pro Val Glu Val Asp Arg Val Gly Gly
       690                 695                 700
   Val Val Glu Gly Glu Arg Ala Arg Ile Gln Arg Ile Ser Lys Asp Ile
   705                 710                 715                 720
   Asp Ser Ala Leu Glu Arg Glu Leu Leu Arg Ser Gly Leu Thr Leu Lys
                   725                 730                 735
   Ile Glu Glu Thr Leu Thr Ser Glu Asn Leu Ser Ile Tyr Gly Lys Asp
               740                 745                 750
   Leu His Cys Pro Gln His Leu Thr Leu Ala Val Lys Lys Ala Gly Ser
           755                 760                 765
   Ala Ser Ile Ile Ser Ser Glu Gln Tyr Gln Asp Val Pro Thr Phe Leu
   770 775 780
   Ser Gly Leu Gly Thr Gly Met Glu Thr Ile Ser Glu Cys Val Asn Asn
   785 790 795 800
   Lys Val Ser Ala His Leu Phe Gly Val Ile Leu Gly Ala Ala Gly Trp
   805 810 815
   Lys Ser Leu Ala Gln Arg Gln Thr Trp Lys Gly Trp Glu Tyr Pro Phe
   820 825 830
   Gln Asn Glu Thr Ser Arg Arg Gln Val Arg Ser Gln Gly Ile Leu Leu
   835 840 845
   Gln Lys Gly Glu Ser Thr Met Val His Lys Glu Pro Glu Ala Asn Pro
   850 855 860
   Glu Lys Arg Thr Ile Glu Leu Leu Leu Val Ser Ser Leu Phe Gly Ser
   865 870 875 880
   Ala Leu Gly Met Leu Pro Phe Pro Thr Pro Ile Asp Leu Glu Lys Arg
   885 890 895
   Gly Val Gly Asp Tyr Val Thr His Arg Leu Ser Ile Val Lys Met Ala
   900 905 910
   Leu Val Ser Lys Lys Leu Pro Asn Arg Met Ile Glu Met Ile Val Ser
   915 920 925
   Thr Met Asn Leu Pro Leu Ser Arg Glu Gln Asp Leu Thr Lys Leu Phe
   930 935 940
   Asp Ser Pro Phe Ser Leu Asn Leu Ala Thr Glu Glu Asp Ala Ala Ser
   945 950 955 960
   Val Ile Lys Arg Leu Ala Arg Gly Thr Leu Arg Gly Leu Asp Ile Lys
   965 970 975
   Asn Lys Lys Leu Ala Asp His Ile Ala Thr Met Asp Gln Gly Ile Thr
   980 985 990
   Gln Ile Asp Glu Ala Leu Ala Ser Ala Asp Thr Ile Asn Pro Arg Ile
   995 1000 1005
   Ala Tyr Gln Phe Arg Asn Ile Thr Asp Gln Lys Glu Ser Glu Met
   1010 1015 1020
   Phe Val  Thr Lys Phe Ala Thr  Ala Lys Thr Met Arg  Met Val Ala
       1025                 1030                 1035
   Leu Ser  Ser Ser Gln Asp Val  Ser Val Val Gly Leu  Leu Asn Lys
       1040                 1045                 1050
   Arg Ser  Gln Ala Lys Glu Ile  Tyr Thr Ile Trp Arg  Thr Gln Arg
       1055                 1060                 1065
   Lys Gly  Glu Thr Leu Trp Thr  Cys Ser Thr Gln Gln  Ala Lys Lys
       1070                 1075                 1080
   Leu Arg  Asp Arg Ser Trp Gly  Lys Asn Ile Ile Gly  Val Thr Ser
       1085                 1090                 1095
   Pro Ser  Pro Leu Glu Ala Ile  Leu Phe Lys Leu Ile  Asp Pro Ile
       1100                 1105                 1110
   Ser Trp  Glu Glu Glu Lys Glu  Ala His His Phe Thr  Ile His Tyr
       1115                 1120                 1125
   Tyr Leu  Ser Lys Pro Ser Leu  Ser Ser Lys Thr Ala  His Thr Thr
       1130                 1135                 1140
   Arg Gly  Pro Leu Val Pro Tyr  Phe Gly Thr Gln Thr  Lys Pro Leu
       1145                 1150                 1155
   Ile Ala  Lys Ala Tyr Met Glu  Leu Lys Gly Asn Pro  Arg Thr Asn
       1160                 1165                 1170
   Lys Ala  Leu Gln Leu Leu Ser  Met Arg Glu Thr Met  Ile Lys Ala
       1175                 1180                 1185
   Gly Ser  Asn Leu Asp Lys Leu  Leu Leu Ser Leu Cys  Ser Asn Ala
       1190                 1195                 1200
   Leu Asp  Ile Asp Val Asn Ser  Leu Pro Ser Leu Gln  Ala Gln Glu
       1205                 1210                 1215
   Glu Ala  Ser Ala Gly Glu Gly  Val Arg Gly Gly Ile  Lys Glu Ser
       1220                 1225                 1230
   Met Ser  Pro Val Gly Pro Asp  Asn Leu Tyr Thr His  Ile Thr His
       1235                 1240                 1245
   Lys Val  Phe Glu Arg Gln Trp  Leu Ser Glu Phe His  Val Asn Ile
       1250                 1255                 1260
   Ala Asp Phe Ile Ile Trp Gly Ile Thr Lys Thr Arg Gln His Leu
       1265                1270                1275
   Gln Val Asn Thr Asp Leu Gly Gly Ser Leu Pro Ile Cys Val Pro
       1280                1285                1290
   Ala Cys Gln Glu Cys Tyr Arg Glu Lys Glu Arg Val Phe Leu Asp
       1295                1300                1305
   Ile Pro Arg Glu Thr Glu Trp Val Asn Thr Ser Thr Thr Ser Asp
       1310                1315                1320
   Lys Ala Gln Thr Tyr Phe Ser Thr Trp Cys Asp Leu Pro Arg Val
       1325                1330                1335
   Ser Thr Leu Pro Ser Leu Asp Gln Lys Asp Ala Thr Cys Leu Met
       1340                1345                1350
   Gly Arg Ser Met Ala Thr Gln Lys Ser Thr Pro Gly Glu Ser Ile
       1355                1360                1365
   Thr Lys Phe Tyr Ser Thr Ala Pro Asp Thr His Arg Leu Leu His
       1370                1375                1380
   Pro Val Thr Leu Val Leu Gly Tyr Ala Glu Gly Thr Ile Phe Ser
       1385                1390                1395
   Tyr Met Lys Ser Gln His Asn Ile His Gly Ser Leu Tyr His Pro
       1400                1405                1410
   Asn Ile Glu Glu Ile Glu Pro Ala Leu Glu Lys Tyr Val Ile Asp
       1415                1420                1425
   Thr Lys Thr Ser His Thr Lys His Leu Gly Tyr Leu Phe Gln Asp
       1430                1435                1440
   Ala Asp Ser Leu Gln Glu Leu Leu Glu Thr Gly Met Thr Pro Tyr
       1445                1450                1455
   Ile Pro Arg Ser Ile Pro Leu Thr Ile Thr Glu Leu Thr Ser Ala
       1460                1465                1470
   Cys Cys Met Thr Leu Ala Lys Ala Ile Ser Ile Val Leu Arg Thr
       1475                1480                1485
   Gly Val Thr Ile Pro Leu Met Pro Glu Asn Gly Tyr Gly Glu Asn
       1490                1495                1500
   Asp Ile Gln Val Ala Arg Leu Thr Ala Asn Ser Leu Ser Arg Leu
   1505 1510 1515
   Met Pro Arg Gly Arg Ile Gln Leu Val Tyr Leu Asp Cys Asp Leu
   1520 1525 1530
   Thr Ser Gln Met Thr Ala Trp Val Pro Thr Ser Gln Pro Ser Val
   1535 1540 1545
   Leu Gly Ser Val Asn Phe His Ile Glu Gly Val Thr Ile Ser Leu
   1550 1555 1560
   Thr Ala Thr Glu Met Arg Val Gly Gln Glu Ser Trp Glu Glu Arg
   1565 1570 1575
   Lys Trp Thr Cys Ser Asn Asn Arg His Ile Ile Ala Lys Gly Val
   1580 1585 1590
   Lys Thr Lys Ser Leu Phe Ile His Gln Ser Val Pro Glu Ile Ile
   1595 1600 1605
   Thr His Pro Pro Asp Leu Ile Val Val Ile Gly Gly Gly Leu Gly
   1610 1615 1620
   Gly Cys Val Val Pro Tyr Leu Gln Lys Trp Arg Gly Pro Lys Val
   1625 1630 1635
   Ile Phe Cys Thr Leu Phe Asp Glu Arg Glu Arg Ile Ser Glu Asp
   1640 1645 1650
   Gly Asp Leu Ile Ile Pro Pro Glu Leu Leu Val Arg Gly Leu Ala
   1655 1660 1665
   Gly Arg Met Ile Glu Lys Glu Leu Leu Glu Ala Glu Met Cys Asp
   1670 1675 1680
   Ile Thr Val Lys Gly Asn Arg Asp Leu Leu Ile Lys Val Val Gln
   1685 1690 1695
   Lys Trp Val Gln Pro Asn Glu His Val Leu Leu Ile Asp Glu Ile
   1700 1705 1710
   Glu Asn Arg Gly Asp Gln Glu Ser Val Leu Gln Ser Ser Ile Ser
   1715 1720 1725
   Glu Leu Leu Thr Arg Met Asp Ser Val Cys Asn Leu Thr Ser Val
   1730 1735 1740
   His Thr Ile Arg Glu Thr Gly Pro Arg Gln Phe Ala Gln Arg Val
   1745 1750 1755
   Asn Thr Ile Arg Arg Gly Arg Arg Thr Ala Thr Leu His Trp Asn
   1760 1765 1770
   Gln Tyr Asn Arg Arg Asp Gln Val Glu Ala Leu Leu Leu Val Glu
   1775 1780 1785
   Ser His Thr Arg Lys Thr Glu Leu His Val Thr Ser Ser Ala Val
   1790 1795 1800
   Gln Ala Ala Phe Arg Lys Ile Asp Glu Lys Leu Glu Ser Glu Ser
   1805 1810 1815
   Arg Leu Glu His Ser Lys Trp Ser Leu Pro Glu Leu Pro Pro Arg
   1820 1825 1830
   Glu Lys Asp Ile Leu Leu Gly Tyr Val Ala Ser Val Phe Leu Lys
   1835 1840 1845
   Leu Gly Leu Val Val Thr Asp Arg His Met Ser Ala Ala Ala Leu
   1850 1855 1860
   Ile Thr Leu Leu Glu Glu Ala Gly Pro Lys Met Ile Ser Trp Asp
   1865 1870 1875
   Lys Lys Met Glu His Arg Thr Trp Ala Ser Ser Asp Ala Ile Thr
   1880 1885 1890
   Glu Lys Gly Ile Thr Gln Asp Gln Ile Phe Ser Leu Leu Cys Phe
   1895 1900 1905
   Ala Trp Ala Leu Arg Gly Leu Lys Ser Gly Asp Trp Glu His Asn
   1910 1915 1920
   Ala Asp Ala Ile Ile Leu Gln Asp Val His Ile Asp Thr Gly Pro
   1925 1930 1935
   Arg Leu Cys Gln Met Gly Glu Ser Pro Lys Arg Thr Phe Ala Ser
   1940 1945 1950
   Phe Arg Leu His Asn Thr Lys Lys Ala Glu Asp Leu Lys Gly Tyr
   1955 1960 1965
   Leu Gly  Ala Leu Leu His Leu  Glu Ser Phe Phe Pro  Phe Gly Glu
       1970                 1975                 1980
   Gln